# EUROPEAN PATENT SPECIFICATION

(11) **EP 2 518 148 B1**
(45) Date of publication and mention of the grant of the patent: **26.07.2017**
(21) Application number: 10839538.5
(22) Date of filing: 24.12.2010
(51) Int. Cl.: A01H 5/00, C07K 14/415, C12N 5/00, C12N 15/82, C12Q 1/68

(54) **GENE DRO1 CONTROLLING DEEP-ROOTED CHARACTERISTICS OF PLANT AND UTILIZATION OF SAME**
GEN DRO1 ZUR STEUERUNG DER VERERBUNGSEIGENSCHAFTEN EINER PFLANZE UND VERWENDUNG DAVON
GÈNE DRO1 CONTRÔLANT LES CARACTÉRISTIQUES D'ENRACINEMENT PROFOND DE PLANTES ET UTILISATION DE CELLES-CI

(30) Priority: 24.12.2009 JP 2009292524
(43) Date of publication of application: 31.10.2012
(73) Proprietor: National Agriculture and Food Research Organization, Tsukuba-shi, Ibaraki (JP)
(72) Inventor: UGA, Yusaku, Tsukuba-shi Ibaraki 305-8602 (JP)
(74) Representative: Bösl, Raphael Konrad
(86) International application number: PCT/JP2010/073288
(87) International publication number: WO 2011/078308

(56) References cited:
- EP-A1- 1 886 552
- JP-A- 2005 185 101
- US-A1- 2006 253 938
- DATABASE Geneseq [Online] 12 June 2008 (2008-06-12), "Rice genomic DNA sequence SEQ ID NO 54812.", XP002712746, retrieved from EBI accession no. GSN:AQE22906 Database accession no. AQE22906 -& DATABASE Geneseq [Online] 12 June 2008 (2008-06-12), "Rice genomic DNA sequence SEQ ID NO 54813.", XP002712747, retrieved from EBI accession no. GSN:AQE22907 Database accession no. AQE22907 -& DATABASE Geneseq [Online] 12 June 2008 (2008-06-12), "Rice genomic DNA sequence SEQ ID NO 47199.", XP002712748, retrieved from EBI accession no. GSN:AQE15293 Database accession no. AQE15293 -& US 2007/039076 A1 (BOUKHAROV ANDREY A [US] ET AL) 15 February 2007 (2007-02-15)
- DATABASE Geneseq [Online] 28 December 2007 (2007-12-28), "Oryza sativa nucleotide sequence SEQ ID NO 32322.", XP002712749, retrieved from EBI accession no. GSN:ANL18320 Database accession no. ANL18320 -& DATABASE Geneseq [Online] 28 December 2007 (2007-12-28), "Oryza sativa amino acid sequence SEQ ID NO 134805.", XP002712750, retrieved from EBI accession no. GSP:ANM20804 Database accession no. ANM20804 -& DATABASE Geneseq [Online] 28 December 2007 (2007-12-28), "Oryza sativa nucleotide sequence SEQ ID NO 48501.", XP002712751, retrieved from EBI accession no. GSN:ANL34499 Database accession no. ANL34499 -& US 2004/123343 A1 (LA ROSA THOMAS J [US] ET AL LA ROSA THOMAS [US] ET AL) 24 June 2004 (2004-06-24)
- DATABASE Geneseq [Online] 12 June 2008 (2008-06-12), "Rice cDNA clone SEQ ID No 18476.", XP002712752, retrieved from EBI accession no. GSN:AQD24617 Database accession no. AQD24617 & US 2006/123505 A1 (KIKUCHI SHOSHI [JP] ET AL) 8 June 2006 (2006-06-08)
- DATABASE UniProt [Online] 13 September 2004 (2004-09-13), "SubName: Full=Os09g0439800 protein; SubName: Full=Putative uncharacterized protein OJ1344_B01.21;", XP002712753, retrieved from EBI accession no. UNIPROT:Q69P88 Database accession no. Q69P88
- DATABASE UniProt [Online] 1 September 2009 (2009-09-01), "SubName: Full=Putative uncharacterized protein Sb02g025775; Flags: Fragment;", XP002712754, retrieved from EBI accession no. UNIPROT:C5XDP7 Database accession no. C5XDP7
- DATABASE EMBL [Online] 31 October 2008 (2008-10-31), "Zea mays clone 335330 mRNA sequence.", XP002712755, retrieved from EBI accession no. EM_STD:EU969762 Database accession no. EU969762
- CHAITRA J ET AL: "Validation of markers linked to maximum root length in rice (Oryza sativa L.)", CURRENT SCIENCE (BANGALORE), vol. 90, no. 6, March 2006 (2006-03), pages 835-838, XP002712756, ISSN: 0011-3891
- UGA YUSAKU ET AL: "QTLs underlying natural variation in stele and xylem structures of rice root", BREEDING SCIENCE, vol. 58, no. 1, March 2008 (2008-03), pages 7-14, XP002712757, ISSN: 1344-7610
- UGA YUSAKU ET AL: "Variation in root morphology and anatomy among accessions of cultivated rice (Oryza sativa L.) with different genetic backgrounds", BREEDING SCIENCE, vol. 59, no. 1, March 2009 (2009-03), pages 87-93, XP002712758, ISSN: 1344-7610
- UGA YUSAKU ET AL: "Dro1, a major QTL involved in deep rooting of rice under upland field conditions", JOURNAL OF EXPERIMENTAL BOTANY, vol. 62, no. 8, May 2011 (2011-05), pages 2485-2494, XP002712759,
- UGA YUSAKU ET AL: "Control of root system architecture by DEEPER ROOTING 1 increases rice yield under drought conditions.", NATURE GENETICS 28 AUG 2013, vol. 45, no. 9, 28 August 2013 (2013-08-28), pages 1097-1102, XP002712760, ISSN: 1546-1718
- 'The Rice Annotation Project, Curated genome annotation of Oryza sativa ssp. japonica and comparative genome analysis with Arabidopsis thaliana' GENOME RES. vol. 17, no. 2, February 2007, pages 175 - 183
- YUSAKU UGA: 'Seiri Keitai Keishitsu ni Kan'yo suru Idenshi no Kaiseki' KENKYU SEIKA DAI 473 SHU 'GENOME IKUSHU NI YORU KORITSUTEKI HINSHU IKUSEI GIJUTSU NO KAIHATSU -QTL IDENSHI KAISEKI NO SUISHIN-'
- OMORI F. ET AL: 'QTL mapping of root angle in F2 populations from maize 'B73 x teosinte 'Zea luxurians'' PLANT ROOT. vol. 1, 2007, pages 57 - 65, XP008161283
- KATO Y. ET AL: 'Genotypic variation in root growth angle in rice(Oryza sativa L.) and its association with deep root development in upland fields with different water regimes' PLANT SOIL vol. 287, 2006, pages 117 - 129, XP019437172
- SHEN L. ET AL: 'Evaluation of near-isogenic lines of rice introgressed with QTLs for root depth through marker-aided selection' THEOR.APPL.GENET. vol. 103, 2001, pages 75 - 83

### SEQUENCE LISTING

<110> NATIONAL INSTITUTE OF AGROBIOLOGICAL SCIENCES
<120> Gene, Dro1, controlling deep root in plant and utilization thereof
<130> MOA-A0904P
<150> JP 2009-292524
   <151> 2009-12-24
<160> 18
<170> PatentIn version 3.4
<210> 1
   <211> 3058
   <212> DNA
   <213> Oryza sativa
<400> 1
   agatgaagta tagcaagcta tcctacagcc agccacgctg caccgaaccc tccaatcctt      60
   ggaacttgaa ccccttcaaa tcacacccag taggctacta gtagtacttc tctgcaccaa     120
   ttccatcgcc aatagcagcc actacaagtc ttacatctct cctttcctcc tctctctgcc     180
   attgctagga gcttgcattt cttggtagct tcatcagcta gctgctttct ccctccccaa     240
   tctctcattc ttcaggccag gatatgaagg taaaggctca aaccatcggt gtgattaatc     300
   atttcagaga ggttaataga tttgaagtgt tggtagtgtt gatccatttc ttgatggcat     360
   catgaggctt gggttttctc tgcagatttt cagctgggta gccaacaaga tcagtgggaa     420
   gcaagaagca aatcggtttc ccgcaaattc ttctgcgcct taccgtgagt gtacctgaac     480
   tttcagtttc cttgtaaatt ctttcagaac taaggtgatt gttgttcacc aggcttcatg     540
   ctgttcttat gaaatgtttc cctgttcctt gaggggagag ggagagtacg gacagtaatt     600
   tggaagccgt ttcctaattt gatattttct ttctgtcatt ctgctgcagt tataatgtct     660
   ttacctctta gcagtttctg cttaggcatc atttgcctta ttcatttctg gatacgatga     720
   tactttattg ctttgtccta atttaatctt ttcctgctgt cattctgctc catttatggt     780
   gtctttacct cttagcagtt tccgcttagg catcatttgc catgttcatt tctgaatatg     840
   ctgatatact ttgttgtctt ggtagccata tacaagagtg aacgatttaa aggtttctcc     900
   taattgctag taacagaacg cttttcgctc tgcgattttc tggattacca tcccctgatt     960
   cctgagtaac ctatcacaac gccaatgtga tggagcaaac attgatgtat tatgacaatg    1020
   ttgtttctga tcctttgaga aaattttctt atatgcactt gggtgttgtt tggaacgcag    1080
   gaatttcact ggaaacagtt caattatcgc acttccggaa aaaaaattcc tgcattttaa    1140
   acggcgcttt aagttcagta ggagttttca attttatttt cctcttccct agcacgaaaa    1200
   gtgagagatt tttgtgcata tgttataccc ccaaaggact atttcatggc ctgatgcccg    1260
   ccacgccaat atctctgtaa agcactgctt ccaactagtc agtatgggtc aaagtcttga    1320
   ctaagtgcaa ccctgcaagc aagtgtttcc aactagtcag tatggcttca attatttagt   1380
   tgatttataa ccccaaccct ccacttgtga ccaagaagaa tttgatttga gcagacaata   1440
   tcctgacctc ggatggtttc gtaaatcgta ctaaggaatc agatctgagc cactagtcca   1500
   gctggactac agtacattat ttatctctca agattacata aaaagactac tattgtacag   1560
   agaatgcatt gtttaagtgg taacaactgc tgtgataagt tattcctggt atacagaaaa   1620
   atggcaggtg aaaacatcag ggagtggagt aagcatgggc agacattgta cttaaagagt   1680
   gataggccaa tgagaatcac tgggtgttta aggattaaaa atttgtggtc catgctctag   1740
   acatccaatt tatgctaata tgaacttagg accatatatt tatgtttgtt tactgatctt   1800
   gcggcttaat cgagttctaa tgcgcaggtg ctaacgtgtc agattgtcga aaggatgaat   1860
   tcagtgattg gccccaatca ttgcttgcta ttgggacatt tggaaataag cagattgagg   1920
   aggtagctca ggtggagaat tcatccgaca atgtgcagtc agtgcaagat accgtcaaat   1980
   ttacagagga agaagtagac aagatacgga aagaattcga aacgctacta gcaatcaaag   2040
   atcaagcaga agctcaacgc tcgcatgatg atgaccaagt aggtttacaa aagcgtgccg   2100
   atggggaaga caatgagaag catattagac agttgatcaa caaaaggatc attgtaagta   2160
   aatcaaagaa ttcgttagga aagaaaggaa atacactcaa gccgagatca gtcgcttcgt   2220
   tgctcaagtt attcatgtgt aagggtggct ttacctccgt agttccagaa ccaaggaaca   2280
   catttcctca atcaagaatg gagaaggtac acagtgaaat tctttttttc ttatgtaaca   2340
   tgataagttt tacatacttt ctcgtcctct tattatctta tatgatgttc attgcagttg   2400
   ctcaaggcaa tattgcagaa gaaaatacac ccgcaaaatt cttccacgct agttgctaag   2460
   aggcatttgg actggaagcc agatgagaca gaaatcaatg agtgcctgga ggatgcactg   2520
   cgtgatctag acgatgatgg tgcaaaatgg gtcaaaactg attcagagtg taagtagcat   2580
   acttgcctat catgaattga accttttctt ttgccacttt tgtcaatgga gcaactgact   2640
   gaatatgaac ttttcttgtt tccagatatt gtgcttgaaa tgtaaagttg ggtgaatatg   2700
   ttagttcgtt catgccaggt atataccttt ctttgatccc caaattttca actcaactca   2760
   aatgtgaatt atccttgatc tagcatgtct ctctgttttg atctgattag tgtgattcca   2820
   atctgcaggt tccaaaggtc ctggcaagct tggggtttat ggtgttacta cgtaatgata   2880
   taaatatggg atatagttag caatgaagct ctatgatcat gtaatgctcc tccattattt   2940
   ctgacatgaa ccatctgtaa tttgaatcat gataaggagg ttctgggaca aaggcctatt   3000
   ccatgtgtct actctcttct gccctgaaac tgtaaagaac gcattcaatt ttttcaac     3058
<210> 2
   <211> 1282
   <212> DNA
   <213> Oryza sativa
<400> 2
   agatgaagta tagcaagcta tcctacagcc agccacgctg caccgaaccc tccaatcctt     60
   ggaacttgaa ccccttcaaa tcacacccag taggctacta gtagtacttc tctgcaccaa    120
   ttccatcgcc aatagcagcc actacaagtc ttacatctct cctttcctcc tctctctgcc    180
   attgctagga gcttgcattt cttggtagct tcatcagcta gctgctttct ccctccccaa    240
   tctctcattc ttcaggccag gatatgaaga ttttcagctg ggtagccaac aagatcagtg    300
   ggaagcaaga agcaaatcgg tttcccgcaa attcttctgc gccttaccgt gctaacgtgt    360
   cagattgtcg aaaggatgaa ttcagtgatt ggccccaatc attgcttgct attgggacat    420
   ttggaaataa gcagattgag gaggtagctc aggtggagaa ttcatccgac aatgtgcagt    480
   cagtgcaaga taccgtcaaa tttacagagg aagaagtaga caagatacgg aaagaattcg    540
   aaacgctact agcaatcaaa gatcaagcag aagctcaacg ctcgcatgat gatgaccaag    600
   taggtttaca aaagcgtgcc gatggggaag acaatgagaa gcatattaga cagttgatca    660
   acaaaaggat cattgtaagt aaatcaaaga attcgttagg aaagaaagga aatacactca    720
   agccgagatc agtcgcttcg ttgctcaagt tattcatgtg taagggtggc tttacctccg    780
   tagttccaga accaaggaac acatttcctc aatcaagaat ggagaagttg ctcaaggcaa    840
   tattgcagaa gaaaatacac ccgcaaaatt cttccacgct agttgctaag aggcatttgg    900
   actggaagcc agatgagaca gaaatcaatg agtgcctgga ggatgcactg cgtgatctag    960
   acgatgatgg tgcaaaatgg gtcaaaactg attcagagta tattgtgctt gaaatgtaaa   1020
   gttgggtgaa tatgttagtt cgttcatgcc aggttccaaa ggtcctggca agcttggggt   1080
   ttatggtgtt actacgtaat gatataaata tgggatatag ttagcaatga agctctatga   1140
   tcatgtaatg ctcctccatt atttctgaca tgaaccatct gtaatttgaa tcatgataag   1200
   gaggttctgg gacaaaggcc tattccatgt gtctactctc ttctgccctg aaactgtaaa   1260
   gaacgcattc aattttttca ac                                             1282
<210> 3
   <211> 251
   <212> PRT
   <213> Oryza sativa
<400> 3
   Met Lys Ile Phe Ser Trp Val Ala Asn Lys Ile Ser Gly Lys Gln Glu
   1               5                   10                  15
   Ala Asn Arg Phe Pro Ala Asn Ser Ser Ala Pro Tyr Arg Ala Asn Val
               20                  25                  30
   Ser Asp Cys Arg Lys Asp Glu Phe Ser Asp Trp Pro Gln Ser Leu Leu
           35                  40                  45
   Ala Ile Gly Thr Phe Gly Asn Lys Gln Ile Glu Glu Val Ala Gln Val
       50                  55                  60
   Glu Asn Ser Ser Asp Asn Val Gln Ser Val Gln Asp Thr Val Lys Phe
   65                  70                  75                  80
   Thr Glu Glu Glu Val Asp Lys Ile Arg Lys Glu Phe Glu Thr Leu Leu
                   85                  90                  95
   Ala Ile Lys Asp Gln Ala Glu Ala Gln Arg Ser His Asp Asp Asp Gln
               100                 105                 110
   Val Gly Leu Gln Lys Arg Ala Asp Gly Glu Asp Asn Glu Lys His Ile
           115                 120                 125
   Arg Gln Leu Ile Asn Lys Arg Ile Ile Val Ser Lys Ser Lys Asn Ser
       130                 135                 140
   Leu Gly Lys Lys Gly Asn Thr Leu Lys Pro Arg Ser Val Ala Ser Leu
   145                 150                 155                 160
   Leu Lys Leu Phe Met Cys Lys Gly Gly Phe Thr Ser Val Val Pro Glu
                   165                 170                 175
   Pro Arg Asn Thr Phe Pro Gln Ser Arg Met Glu Lys Leu Leu Lys Ala
               180                 185                 190
   Ile Leu Gln Lys Lys Ile His Pro Gln Asn Ser Ser Thr Leu Val Ala
           195                 200                 205
   Lys Arg His Leu Asp Trp Lys Pro Asp Glu Thr Glu Ile Asn Glu Cys
       210                 215                 220
   Leu Glu Asp Ala Leu Arg Asp Leu Asp Asp Asp Gly Ala Lys Trp Val
   225                 230                 235                 240
   Lys Thr Asp Ser Glu Tyr Ile Val Leu Glu Met
                   245                 250
<210> 4
   <211> 20
   <212> DNA
   <213> Artificial
<220>
   <223> An artificially synthesized primer sequence
<400> 4
   gcagacgctc gtaacacgta 20
<210> 5
   <211> 20
   <212> DNA
   <213> Artificial
<220>
   <223> An artificially synthesized primer sequence
<400> 5
   gtggcagctc catcaactct 20
<210> 6
   <211> 20
   <212> DNA
   <213> Artificial
<220>
   <223> An artificially synthesized primer sequence
<400> 6
   gcacaagatg ggaggagagt 20
<210> 7
   <211> 20
   <212> DNA
   <213> Artificial
<220>
   <223> An artificially synthesized primer sequence
<400> 7
   catgggtgag aatcgtgttg 20
<210> 8
   <211> 20
   <212> DNA
   <213> Artificial
<220>
   <223> An artificially synthesized primer sequence
<400> 8
   gtctagatca cgcagtgaat 20
<210> 9
   <211> 20
   <212> DNA
   <213> Artificial
<220>
   <223> An artificially synthesized primer sequence
<400> 9
   tcgcatgatg atgaccaagt 20
<210> 10
   <211> 23
   <212> DNA
   <213> Artificial
<220>
   <223> An artificially synthesized primer sequence
<400> 10
   atcgtctaga tcacgcagtg aac 23
<210> 11
   <211> 20
   <212> DNA
   <213> Artificial
<220>
   <223> An artificially synthesized primer sequence
<400> 11
   agggtggctt tacctccgta 20
<210> 12
   <211> 789
   <212> DNA
   <213> Sorghum bicolor
<220>
   <221> CDS
   <222> (1)..(789)
<400> 12
   ctg cag att ttc agt tgg gta gcc aac aag atc ggc ggg aag caa gaa        48
   Leu Gln Ile Phe Ser Trp Val Ala Asn Lys Ile Gly Gly Lys Gln Glu
   1               5                   10                  15
   cca aag cga tct cct gca cct tat cgt aag cct act tgt ggc aat gta        96
   Pro Lys Arg Ser Pro Ala Pro Tyr Arg Lys Pro Thr Cys Gly Asn Val
               20                  25                  30
   tca gaa tgt cgc aac gac gag ttc agt gat tgg ccc caa tca ttg ctt       144
   Ser Glu Cys Arg Asn Asp Glu Phe Ser Asp Trp Pro Gln Ser Leu Leu
           35                  40                  45
   gca att ggg aca ttt gga aac aag cag ctc gag gaa gaa gta gca tca       192
   Ala Ile Gly Thr Phe Gly Asn Lys Gln Leu Glu Glu Glu Val Ala Ser
       50                  55                  60
   gag agt tct tct gcg aat gct cag acc acg caa gat ccc gcc aag ttt       240
   Glu Ser Ser Ser Ala Asn Ala Gln Thr Thr Gln Asp Pro Ala Lys Phe
   65                  70                  75                  80
   aca gag gag gaa gta gac aac ata cga aga gaa ttc gag gtg ctg ctg       288
   Thr Glu Glu Glu Val Asp Asn Ile Arg Arg Glu Phe Glu Val Leu Leu
                   85                  90                  95
   caa ggc aat gct caa gca gaa gct cag ggc tcc tgt gaa gat gaa cag       336
   Gln Gly Asn Ala Gln Ala Glu Ala Gln Gly Ser Cys Glu Asp Glu Gln
               100                 105                 110
   gta gca gct tcg aaa gaa cat gac ggt gaa gac aac aac gag aag cag       384
   Val Ala Ala Ser Lys Glu His Asp Gly Glu Asp Asn Asn Glu Lys Gln
           115                 120                 125
   cgc agg gag caa ttg atg aac agg gag atg atc ata agc aaa gcg aga       432
   Arg Arg Glu Gln Leu Met Asn Arg Glu Met Ile Ile Ser Lys Ala Arg
       130                 135                 140
   gaa cta gta ggg aag aaa aag agt gct aag ctc aag cca aga tcg atg      480
   Glu Leu Val Gly Lys Lys Lys Ser Ala Lys Leu Lys Pro Arg Ser Met
   145                 150                 155                 160
   gtt tcg ctc ctt aga tta ctc gcg tgc aag ggc ggt ttt acc acc ccg      528
   Val Ser Leu Leu Arg Leu Leu Ala Cys Lys Gly Gly Phe Thr Thr Pro
                   165                 170                 175
   gtt ctg gaa cca agg aac gct ttc ccc caa tca aga atg gag aag ctg      576
   Val Leu Glu Pro Arg Asn Ala Phe Pro Gln Ser Arg Met Glu Lys Leu
               180                 185                 190
   ctc aag gca ata cta cag aag aaa ata cac cca cag aat tcc aac acg      624
   Leu Lys Ala Ile Leu Gln Lys Lys Ile His Pro Gln Asn Ser Asn Thr
           195                 200                 205
   gta gca acc agg agg cac ttg gac tgg aag cta gac gag aaa gag atc      672
   Val Ala Thr Arg Arg His Leu Asp Trp Lys Leu Asp Glu Lys Glu Ile
       210                 215                 220
   aac gaa tgt ctt gag gat gcg ctg cgt gat ctt gat gac gat gat gat      720
   Asn Glu Cys Leu Glu Asp Ala Leu Arg Asp Leu Asp Asp Asp Asp Asp
   225                 230                 235                 240
   ggc gca aag tgg gtc aaa act gat tca gac tgt tcc gaa ggt ctt ggt      768
   Gly Ala Lys Trp Val Lys Thr Asp Ser Asp Cys Ser Glu Gly Leu Gly
                   245                 250                 255
   ggg ctt ggt aca ttc acg gtg                                          789
   Gly Leu Gly Thr Phe Thr Val
               260
<210> 13
   <211> 263
   <212> PRT
   <213> Sorghum bicolor
<400> 13
   Leu Gln Ile Phe Ser Trp Val Ala Asn Lys Ile Gly Gly Lys Gln Glu
   1               5                   10                  15
   Pro Lys Arg Ser Pro Ala Pro Tyr Arg Lys Pro Thr Cys Gly Asn Val
               20                  25                  30
   Ser Glu Cys Arg Asn Asp Glu Phe Ser Asp Trp Pro Gln Ser Leu Leu
           35                  40                  45
   Ala Ile Gly Thr Phe Gly Asn Lys Gln Leu Glu Glu Glu Val Ala Ser
       50                  55                  60
   Glu Ser Ser Ser Ala Asn Ala Gln Thr Thr Gln Asp Pro Ala Lys Phe
   65                  70                  75                  80
   Thr Glu Glu Glu Val Asp Asn Ile Arg Arg Glu Phe Glu Val Leu Leu
                   85                  90                  95
   Gln Gly Asn Ala Gln Ala Glu Ala Gln Gly Ser Cys Glu Asp Glu Gln
                   100                 105                 110
   Val Ala Ala Ser Lys Glu His Asp Gly Glu Asp Asn Asn Glu Lys Gln
           115                 120                 125
   Arg Arg Glu Gln Leu Met Asn Arg Glu Met Ile Ile Ser Lys Ala Arg
       130                 135                 140
   Glu Leu Val Gly Lys Lys Lys Ser Ala Lys Leu Lys Pro Arg Ser Met
   145                 150                 155                 160
   Val Ser Leu Leu Arg Leu Leu Ala Cys Lys Gly Gly Phe Thr Thr Pro
                   165                 170                 175
   Val Leu Glu Pro Arg Asn Ala Phe Pro Gln Ser Arg Met Glu Lys Leu
               180                 185                 190
   Leu Lys Ala Ile Leu Gln Lys Lys Ile His Pro Gln Asn Ser Asn Thr
           195                 200                 205
   Val Ala Thr Arg Arg His Leu Asp Trp Lys Leu Asp Glu Lys Glu Ile
       210                 215                 220
   Asn Glu Cys Leu Glu Asp Ala Leu Arg Asp Leu Asp Asp Asp Asp Asp
   225                 230                 235                 240
   Gly Ala Lys Trp Val Lys Thr Asp Ser Asp Cys Ser Glu Gly Leu Gly
                   245                 250                 255
   Gly Leu Gly Thr Phe Thr Val
               260
<210> 14
   <211> 768
   <212> DNA
   <213> Zea mays
<220>
   <221> CDS
   <222> (1)..(768)
<400> 14
   atg aag att ttc agt tgg gta gcc aac aag atc ggc ggg aag caa gaa       48
   Met Lys Ile Phe Ser Trp Val Ala Asn Lys Ile Gly Gly Lys Gln Glu
   1               5                   10                  15
   ccg aag cga tct gcc gcg cat tat cgt ggc aac gta tca gaa tgt cgc       96
   Pro Lys Arg Ser Ala Ala His Tyr Arg Gly Asn Val Ser Glu Cys Arg
               20                  25                  30
   aac gac gag ttc agt gat tgg ccc caa tca ttg ctg gca atc ggg acg      144
   Asn Asp Glu Phe Ser Asp Trp Pro Gln Ser Leu Leu Ala Ile Gly Thr
           35                  40                  45
   ttt ggg aac agg cag ctg gag gaa ggg gtg gtg gag acg tct tct ggg      192
   Phe Gly Asn Arg Gln Leu Glu Glu Gly Val Val Glu Thr Ser Ser Gly
       50                  55                  60
   aac gtc cag gcc gcg caa gac ccc gcc aag ttc aca gag gaa gaa gag      240
   Asn Val Gln Ala Ala Gln Asp Pro Ala Lys Phe Thr Glu Glu Glu Glu
   65                  70                  75                  80
   gcg gac agc ata cgg aga gaa ctc gag gtg ctg ctg ctg caa ggc aat      288
   Ala Asp Ser Ile Arg Arg Glu Leu Glu Val Leu Leu Leu Gln Gly Asn
                   85                  90                  95
   aat aat aat ggc ggc caa gca gag gcg cag ggc tct cgt gga gac gaa      336
   Asn Asn Asn Gly Gly Gln Ala Glu Ala Gln Gly Ser Arg Gly Asp Glu
               100                 105                 110
   cga cga cag gta gct tgg aaa gag cat gac ggt gaa tgt agc aag gag      384
   Arg Arg Gln Val Ala Trp Lys Glu His Asp Gly Glu Cys Ser Lys Glu
           115                 120                 125
   aag cag ccg acg agc ggg gag atg gtc acg agc aag gcg aga gcg aga      432
   Lys Gln Pro Thr Ser Gly Glu Met Val Thr Ser Lys Ala Arg Ala Arg
       130                 135                 140
   gaa atg gta gca ggg aag aaa agg agc acg ctg aag cca agg tcg gtg      480
   Glu Met Val Ala Gly Lys Lys Arg Ser Thr Leu Lys Pro Arg Ser Val
   145                 150                 155                 160
   gcc tcg ctc ctt aga ctg ctc gcg tgc aag ggc ggc ttt gcc acc ccg      528
   Ala Ser Leu Leu Arg Leu Leu Ala Cys Lys Gly Gly Phe Ala Thr Pro
                   165                 170                 175
   gtt ctg gaa ccg agg agc cct ttc ccc cag tcg aga atg gag aag ctg      576
   Val Leu Glu Pro Arg Ser Pro Phe Pro Gln Ser Arg Met Glu Lys Leu
               180                 185                 190
   ctc aag gcg ata ctg gag aag aag ata cac ccg cag aac ccc agc acg      624
   Leu Lys Ala Ile Leu Glu Lys Lys Ile His Pro Gln Asn Pro Ser Thr
           195                 200                 205
   gct gca gcg agg cgg cac cag ctg gac tgg aag cta gac gag aaa gag      672
   Ala Ala Ala Arg Arg His Gln Leu Asp Trp Lys Leu Asp Glu Lys Glu
       210                 215                 220
   atc gac gag tgc ctt gac gac gcg ctg cgt gac ctc gac gac gac ggc      720
   Ile Asp Glu Cys Leu Asp Asp Ala Leu Arg Asp Leu Asp Asp Asp Gly
   225                 230                 235                 240
   gcc aag tgg gtc aaa act gac tcg gac ttt att gtg ctc gaa atg taa      768
   Ala Lys Trp Val Lys Thr Asp Ser Asp Phe Ile Val Leu Glu Met
                   245                 250                 255
<210> 15
   <211> 255
   <212> PRT
   <213> Zea mays
<400> 15
   Met Lys Ile Phe Ser Trp Val Ala Asn Lys Ile Gly Gly Lys Gln Glu
   1               5                   10                  15
   Pro Lys Arg Ser Ala Ala His Tyr Arg Gly Asn Val Ser Glu Cys Arg
               20                  25                  30
   Asn Asp Glu Phe Ser Asp Trp Pro Gln Ser Leu Leu Ala Ile Gly Thr
           35                  40                  45
   Phe Gly Asn Arg Gln Leu Glu Glu Gly Val Val Glu Thr Ser Ser Gly
       50                  55                  60
   Asn Val Gln Ala Ala Gln Asp Pro Ala Lys Phe Thr Glu Glu Glu Glu
   65                  70                  75                  80
   Ala Asp Ser Ile Arg Arg Glu Leu Glu Val Leu Leu Leu Gln Gly Asn
                   85                  90                  95
   Asn Asn Asn Gly Gly Gln Ala Glu Ala Gln Gly Ser Arg Gly Asp Glu
               100                 105                 110
   Arg Arg Gln Val Ala Trp Lys Glu His Asp Gly Glu Cys Ser Lys Glu
           115                 120                 125
   Lys Gln Pro Thr Ser Gly Glu Met Val Thr Ser Lys Ala Arg Ala Arg
       130                 135                 140
   Glu Met Val Ala Gly Lys Lys Arg Ser Thr Leu Lys Pro Arg Ser Val
   145                 150                 155                 160
   Ala Ser Leu Leu Arg Leu Leu Ala Cys Lys Gly Gly Phe Ala Thr Pro
                   165                 170                 175
   Val Leu Glu Pro Arg Ser Pro Phe Pro Gln Ser Arg Met Glu Lys Leu
               180                 185                 190
   Leu Lys Ala Ile Leu Glu Lys Lys Ile His Pro Gln Asn Pro Ser Thr
           195                 200                 205
   Ala Ala Ala Arg Arg His Gln Leu Asp Trp Lys Leu Asp Glu Lys Glu
       210                 215                 220
   Ile Asp Glu Cys Leu Asp Asp Ala Leu Arg Asp Leu Asp Asp Asp Gly
   225                 230                 235                 240
   Ala Lys Trp Val Lys Thr Asp Ser Asp Phe Ile Val Leu Glu Met
                   245                 250                 255
<210> 16
   <211> 1284
   <212> DNA
   <213> Oryza sativa
<400> 16
   gagatgaagt atagcaagct atcctacagc cagccacgct gcaccgaacc ctccaatcct      60
   tggaacttga accccttcaa atcacaccca gtaggctact agtagtactt ctctgcacca     120
   attccatcgc caatagcagc cactacaagt cttacatctc tcctttcctc ctctctctgc     180
   cattgctagg agcttgcatt tcttggtagc ttcatcagct agctgctttc tccctcccca     240
   atctctcatt cttcaggcca ggatatgaag attttcagct gggtagccaa caagatcagt     300
   gggaagcaag aagcaaatcg gtttcccgca aattcttctg cgccttaccg tgctaacgtg     360
   tcagattgtc gagaggatga attcagtgat tggccccaat cattgcttgc tattgggaca     420
   tttggaaata agcagattga ggaggtagct caggtggaga attcatccga caatgtgcag     480
   tcagtgcaag ataccgtcaa atttacagag gaagaagtag acaagatacg gaaagaattc     540
   gaaacgctac tagcaatcaa agatcaagca gaagctcaac gctcgcatga tgatgaccaa     600
   gtaggtttac aaaagcgtgc cgatggggaa gacaatgaga agcatattag acagttgatc     660
   aacaaaagga tcattgtaag taaatcaaag aattcgttag gaaagaaagg aaatacactc     720
   aagccgagat cagtcgcttc gttgctcaag ttattcatgt gtaagggtgg ctttacctcc     780
   gtagttccag aaccaaggaa cacatttcct caatcaagaa tggagaagtt gctcaaggca     840
   atattgcaga agaaaataca cccgcaaaat tcttccacgc tagttgctaa gaggcatttg     900
   gactggaagc cagatgagac agaaatcaat gagtgcctgg aggatgcact gcgtgatcta     960
   gacgatgatg gtgcaaaatg ggtcaaaact gattcagagt atattgtgct tgaaatgtaa    1020
   agttgggtga atatgttagt tcgttcatgc caggttccaa aggtcctggc aagcttgggg    1080
   tttatggtgt tactacgtaa tgatataaat atgggatata gttagcaatg aagctctatg    1140
   atcatgtaat gctcctccat tatttctgac atgaaccatc tgtaatttga atcatgataa    1200
   ggaggttctg ggacaaaggc ctattccatg tgtctactct cttctgccct gaaactgtaa    1260
   agaacgcatt caattttttc aacg                                            1284
<210> 17
   <211> 4283
   <212> DNA
   <213> Oryza sativa
<400> 17
   gcagagatat atatatatat atatatatat atatatagca ctatcatgtc atgttagctt     60
   gttggtccag gtgcatccac ggtctagtac cattattgcg tagttaatgg acggaatagc    120
   tgctagtgga gatgggggtg tacttcagca gttaaccccc agaaacgtga ttagcatcgg    180
   tgcaggcgcc cctcgtgctg gttaaccaag gcgggcgccc cgtacttacc cgcgctaatg    240
   ccgggtttaa ggaagttaac acgtgattct ggcacacgcg tcgggcccga ccaagtgcga    300
   tgggatccgt tgcggcgcgg tcgtgacact tggtgcccca acttgccgtc gatctcgccg    360
   cgctgtactt tatcttagct aataactctt cttctccccc tggaaaaaaa aattaacaac    420
   cgggtttctt cctttgaggc attgattttt ggatactctc tcgctggctg cagtgcaagc    480
   gaaccagtgc acggtattca aacagtctgc aatacctggg taaatctgag tcctttgatt    540
   ttgatctggc gaacataata tgactgttta aaactacagt caccctactt attgcagctg    600
   aaccgtcaaa ttatagcttc ttctagggat ggattgggaa ttttcttaca tgttgagttt    660
   ttgaacacct gactagacga tctcacgata tactaccttc gtcctaaaat atagtaattt    720
   ttagctatga atttggacac acaaaaattg ctatattttg ggacagagag agtagtcaga    780
   tagttttatc attcttaaga aggtatatcg agatgatggt tttttatcgt aaaacttaat    840
   atctctagta ggtgtctcga aatatcgtac aagtcctgca tgtactttct taagtaccat    900
   aaacttctct acaatttaag tagggtagaa gcacccccgt tgaggtgtag ctaccacaat    960
   tttgggttcc tcaaaagtgt gaggtcgatc acaagtttac actatctata tctagaaaca   1020
   tcaattcaat atttacaatc cgtatggcgc atatgggcgt acggtagctg gagttgcaaa   1080
   taccgaattt tctcttccga agaaagtgca aattccattg tttatatata tagctgtgtc   1140
   ctgttatcat tccatggctc acctgttgag ctggtaaccc ggtttggagt catctataaa   1200
   aggaaggtaa aggaagtgac atgccagatg aagtatagca agctatccta cagccagcca   1260
   cgctgcaccg aaccctccaa tccttggaac ttgaacccct tcaaatcaca cccagtaggc   1320
   tactagtagt acttctctgc accaattcca tcgccaatag cagccactac aagtcttaca   1380
   tctctccttt cctcctctct ctgccattgc taggagcttg catttcttgg tagcttcatc   1440
   agctagctgc tttctccctc cccaatctct cattcttcag gccaggatat gaaggtaaag   1500
   gctcaaacca tcggtgtgat taatcatttc agagaggtta atagatttga agtgttggta   1560
   gtgttgatcc atttcttgat ggcatcatga ggcttgggtt ttctctgcag attttcagct   1620
   gggtagccaa caagatcagt gggaagcaag aagcaaatcg gtttcccgca aattcttctg   1680
   cgccttaccg tgagtgtacc tgaactttca gtttccttgt aaattctttc agaactaagg   1740
   tgattgttgt tcaccaggct tcatgctgtt cttatgaaat gtttccctgt tccttgaggg   1800
   gagagggaga gtacggacag taatttggaa gccgtttcct aatttgatat tttctttctg   1860
   tcattctgct gcagttataa tgtctttacc tcttagcagt ttctgcttag gcatcatttg   1920
   ccttattcat ttctggatac gatgatactt tattgctttg tcctaattta atcttttcct   1980
   gctgtcattc tgctccattt atggtgtctt tacctcttag cagtttccgc ttaggcatca   2040
   tttgccatgt tcatttctga atatgctgat atactttgtt gtcttggtag ccatatacaa   2100
   gagtgaacga tttaaaggtt tctcctaatt gctagtaaca gaacgctttt cgctctgcga   2160
   ttttctggat taccatcccc tgattcctga gtaacctatc acaacgccaa tgtgatggag   2220
   caaacattga tgtattatga caatgttgtt tctgatcctt tgagaaaatt ttcttatatg   2280
   cacttgggtg ttgtttggaa cgcaggaatt tcactggaaa cagttcaatt atcgcacttc   2340
   cggaaaaaaa attcctgcat tttaaacggc gctttaagtt cagtaggagt tttcaatttt   2400
   attttcctct tccctagcac gaaaagtgag agatttttgt gcatatgtta tacccccaaa   2460
   ggactatttc atggcctgat gcccgccacg ccaatatctc tgtaaagcac tgcttccaac   2520
   tagtcagtat gggtcaaagt cttgactaag tgcaaccctg caagcaagtg tttccaacta   2580
   gtcagtatgg cttcaattat ttagttgatt tataacccca accctccact tgtgaccaag   2640
   aagaatttga tttgagcaga caatatcctg acctcggatg gtttcgtaaa tcgtactaag   2700
   gaatcagatc tgagccacta gtccagctgg actacagtac attatttatc tctcaagatt   2760
   acataaaaag actactattg tacagagaat gcattgttta agtggtaaca actgctgtga   2820
   taagttattc ctggtataca gaaaaatggc aggtgaaaac atcagggagt ggagtaagca   2880
   tgggcagaca ttgtacttaa agagtgatag gccaatgaga atcactgggt gtttaaggat   2940
   taaaaatttg tggtccatgc tctagacatc caatttatgc taatatgaac ttaggaccat   3000
   atatttatgt ttgtttactg atcttgcggc ttaatcgagt tctaatgcgc aggtgctaac   3060
   gtgtcagatt gtcgaaagga tgaattcagt gattggcccc aatcattgct tgctattggg   3120
   acatttggaa ataagcagat tgaggaggta gctcaggtgg agaattcatc cgacaatgtg   3180
   cagtcagtgc aagataccgt caaatttaca gaggaagaag tagacaagat acggaaagaa   3240
   ttcgaaacgc tactagcaat caaagatcaa gcagaagctc aacgctcgca tgatgatgac   3300
   caagtaggtt tacaaaagcg tgccgatggg gaagacaatg agaagcatat tagacagttg   3360
   atcaacaaaa ggatcattgt aagtaaatca aagaattcgt taggaaagaa aggaaataca   3420
   ctcaagccga gatcagtcgc ttcgttgctc aagttattca tgtgtaaggg tggctttacc   3480
   tccgtagttc cagaaccaag gaacacattt cctcaatcaa gaatggagaa ggtacacagt   3540
   gaaattcttt ttttcttatg taacatgata agttttacat actttctcgt cctcttatta   3600
   tcttatatga tgttcattgc agttgctcaa ggcaatattg cagaagaaaa tacacccgca   3660
   aaattcttcc acgctagttg ctaagaggca tttggactgg aagccagatg agacagaaat   3720
   caatgagtgc ctggaggatg cactgcgtga tctagacgat gatggtgcaa aatgggtcaa   3780
   aactgattca gagtgtaagt agcatacttg cctatcatga attgaacctt ttcttttgcc   3840
   acttttgtca atggagcaac tgactgaata tgaacttttc ttgtttccag atattgtgct   3900
   tgaaatgtaa agttgggtga atatgttagt tcgttcatgc caggtatata cctttctttg   3960
   atccccaaat tttcaactca actcaaatgt gaattatcct tgatctagca tgtctctctg   4020
   ttttgatctg attagtgtga ttccaatctg caggttccaa aggtcctggc aagcttgggg   4080
   tttatggtgt tactacgtaa tgatataaat atgggatata gttagcaatg aagctctatg   4140
   atcatgtaat gctcctccat tatttctgac atgaaccatc tgtaatttga atcatgataa   4200
   ggaggttctg ggacaaaggc ctattccatg tgtctactct cttctgccct gaaactgtaa   4260
   agaacgcatt caattttttc aac                                            4283
<210> 18
   <211> 4313
   <212> DNA
   <213> Oryza sativa
<400> 18
   gcagatatat atatatatat atatatatat atatatatat atatatatat atatatatat 60
   atatatagca ctatcatgtc atgttagctt gttggtccag gtgcatccac ggtctagtac 120
   cattattgcg tagttaatgg acggaatagc tgctagtgga gatgggggtg tacttcagca 180
   gttaaccccc agaaacgtga ttagcatcgg tgcaggcgcc cctcgtgctg gttaaccaag 240
   gcgggcgccc cgtacttacc cgcgctaatg ccgggtttaa ggaagttaac acgtgattct 300
   ggcacacgcg tcgggcccga ccaagtgcga tgggatccgt tgcggcgcgg tcgtgacact 360
   tggtgcccca acttgccgtc gatctcgccg cgctgtactt tatcttagct aataactctt 420
   cttctccccc tggaaaaaaa aattaacaac cgggtttctt cctttgaggc attgattttt 480
   ggatactctc tcgctggctg cagtgcaagc gaaccagtgc acggtattca aacagtctgc 540
   aatacctggg taaatctgag tcctttgatt ttgatctggc gaacataata tgactgttta 600
   aaactacagt caccctactt attgcagctg aaccgtcaaa ttatagcttc ttctagggat 660
   ggattgggaa ttttcttaca tgttgagttt ttgaacacct gactagacga tctcacgata 720
   tactaccttc gtcctaaaat atagtaattt ttagctatga atttggacac acaaaaattg 780
   ctatattttg ggacagagag agtagtcaga tagttttatc attcttaaga aggtatatcg 840
   agatgatggt tttttatcgt aaaacttaat atctctagta ggtgtctcga aatatcgtac 900
   aagtcctgca tgtactttct taagtaccat aaacttctct acaatttaag tagggtagaa 960
   gcacccccgt tgaggtgtag ctaccacaat tttgggttcc tcaaaagtgt gaggtcgatc 1020
   acaagtttac actatctata tctagaaaca tcaattcaat atttacaatc cgtatggcgc 1080
   atatgggcgt acggtagctg gagttgcaaa taccgaattt tctcttccga agaaagtgca 1140
   aattccattg tttatatata tagctgtgtc ctgttatcat tccatggctc acctgttgag 1200
   ctggtaaccc ggtttggagt catctataaa aggaaggtaa aggaagtgac atgccagatg 1260
   aagtatagca agctatccta cagccagcca cgctgcaccg aaccctccaa tccttggaac 1320
   ttgaacccct tcaaatcaca cccagtaggc tactagtagt acttctctgc accaattcca 1380
   tcgccaatag cagccactac aagtcttaca tctctccttt cctcctctct ctgccattgc 1440
   taggagcttg catttcttgg tagcttcatc agctagctgc tttctccctc cccaatctct 1500
   cattcttcag gccaggatat gaaggtaaag gctcaaacca tcggtgtgat taatcatttc 1560
   agagaggtta atagatttga agtgttggta gtgttgatcc atttcttgat ggcatcatga 1620
   ggcttgggtt ttctctgcag attttcagct gggtagccaa caagatcagt gggaagcaag 1680
   aagcaaatcg gtttcccgca aattcttctg cgccttaccg tgagtgtacc tgaactttca 1740
   gtttccttgt aaattctttc agaactaagg tgattgttgt tcaccaggct tcatgctgtt 1800
   cttatgaaat gtttccctgt tccttgaggg gagagggaga gtacggacag taatttggaa 1860
   gccgtttcct aatttgatat tttctttctg tcattctgct gcagttataa tgtctttacc 1920
   tcttagcagt ttctgcttag gcatcatttg ccttattcat ttctggatac gatgatactt 1980
   tattgctttg tcctaattta atcttttcct gctgtcattc tgctccattt atggtgtctt 2040
   tacctcttag cagtttccgc ttaggcatca tttgccatgt tcatttctga atatgctgat 2100
   atactttgtt gtcttggtag ccatatacaa gagtgaacga tttaaaggtt tctcctaatt 2160
   gctagtaaca gaacgctttt cgctctgcga ttttctggat taccatcccc tgattcctga 2220
   gtaacctatc acaacgccaa tgtgatggag caaacattga tgtattatga caatgttgtt 2280
   tctgatcctt tgagaaaatt ttcttatatg cacttgggtg ttgtttggaa cgcaggaatt 2340
   tcactggaaa cagttcaatt atcgcacttc cggaaaaaaa attcctgcat tttaaacggc 2400
   gctttaagtt cagtaggagt tttcaatttt attttcctct tccctagcac gaaaagtgag 2460
   agatttttgt gcatatgtta tacccccaaa ggactatttc atggcctgat gcccgccacg 2520
   ccaatatctc tgtaaagcac tgcttccaac tagtcagtat gggtcaaagt cttgactaag 2580
   tgcaaccctg caagcaagtg tttccaacta gtcagtatgg cttcaattat ttagttgatt 2640
   tataacccca accctccact tgtgaccaag aagaatttga tttgagcaga caatatcctg 2700
   acctcggatg gtttcgtaaa tcgtactaag gaatcagatc tgagccacta gtccagctgg 2760
   actacagtac attatttatc tctcaagatt acataaaaag actactattg tacagagaat 2820
   gcattgttta agtggtaaca actgctgtga taagttattc ctggtataca gaaaaatggc 2880
   aggtgaaaac atcagggagt ggagtaagca tgggcagaca ttgtacttaa agagtgatag 2940
   gccaatgaga atcactgggt gtttaaggat taaaaatttg tggtccatgc tctagacatc 3000
   caatttatgc taatatgaac ttaggaccat atatttatgt ttgtttactg atcttgcggc 3060
   ttaatcgagt tctaatgcgc aggtgctaac gtgtcagatt gtcgaaagga tgaattcagt 3120
   gattggcccc aatcattgct tgctattggg acatttggaa ataagcagat tgaggaggta 3180
   gctcaggtgg agaattcatc cgacaatgtg cagtcagtgc aagataccgt caaatttaca 3240
   gaggaagaag tagacaagat acggaaagaa ttcgaaacgc tactagcaat caaagatcaa 3300
   gcagaagctc aacgctcgca tgatgatgac caagtaggtt tacaaaagcg tgccgatggg 3360
   gaagacaatg agaagcatat tagacagttg atcaacaaaa ggatcattgt aagtaaatca 3420
   aagaattcgt taggaaagaa aggaaataca ctcaagccga gatcagtcgc ttcgttgctc 3480
   aagttattca tgtgtaaggg tggctttacc tccgtagttc cagaaccaag gaacacattt 3540
   cctcaatcaa gaatggagaa ggtacacagt gaaattcttt ttttcttatg taacatgata 3600
   agttttacat actttctcgt cctcttatta tcttatatga tgttcattgc agttgctcaa 3660
   ggcaatattg cagaagaaaa tacacccgca aaattcttcc acgctagttg ctaagaggca   3720
   tttggactgg aagccagatg agacagaaat caatgagtgc ctggaggatg cactgcgtga   3780
   tctagacgat gatggtgcaa aatgggtcaa aactgattca gagtgtaagt agcatacttg   3840
   cctatcatga attgaacctt ttcttttgcc acttttgtca atggagcaac tgactgaata   3900
   tgaacttttc ttgtttccag atattgtgct tgaaatgtaa agttgggtga atatgttagt   3960
   tcgttcatgc caggtatata cctttctttg atccccaaat tttcaactca actcaaatgt   4020
   gaattatcct tgatctagca tgtctctctg ttttgatctg attagtgtga ttccaatctg   4080
   caggttccaa aggtcctggc aagcttgggg tttatggtgt tactacgtaa tgatataaat   4140
   atgggatata gttagcaatg aagctctatg atcatgtaat gctcctccat tatttctgac   4200
   atgaaccatc tgtaatttga atcatgataa ggaggttctg ggacaaaggc ctattccatg   4260
   tgtctactct cttctgccct gaaactgtaa agaacgcatt caattttttc aac            4313

## Description

### Technical Field

The present invention is concerned with a gene that controls plant root morphology and methods for controlling plant root morphology using the gene. More specifically, the present invention is concerned with a gene related to the deep rooting of plants and methods for controlling the deep rooting of plants using the gene.

### Background Art

Currently, the world population is continuing to increase primarily in developing countries, and it is essential to increase food production to feed the population. However, it has been difficult for agricultural lands to have stable rainfall due to climate shifts in recent years such as global warming and desertification. As a result, droughts due to rainfall reduction have caused severe damage in crop production all over the world. In particular, grains such as rice, wheat, and corn, which are mostly cultivated solely by rain water, are more severely damaged by droughts. Therefore, an important objective in grain breeding is to confer drought resistance to crop plants.

For crop plants, the root is an essential phenotype, and is involved in the yield which depends on water and nutrient absorption or such, as well as aboveground plant lodging resistance and drought avoidance. The rice plant is a monocotyledon, and forms a root system with a number of crown roots that extend from internodes after seminal root development. The distribution of rice root system is determined according to the crown root length and its growth direction (Shigenori Morita, Ne no Dezain (Root design), pp. 107, 2003 (Non-patent Document 1)). Of the two, the direction of crown root growth is particularly important in the root system distribution. Specifically, lateral growth of crown roots results in a shallow rooting, while vertical growth results in a deep rooting. When compared to shallow-rooted plants, deep-rooted plants have more roots distributed into deeper soil layers. Thus, when plants are exposed to dry conditions, the plants can avoid drought by absorbing water from the deeper soil layers. Accordingly, the deep rooting is an important trait (Yoshida and Hasegawa, Drought resistance in crops with emphasis on rice, p. 97-114. 1982 (Non-patent Document 2); Shigenori Morita, Ne no Hatsuikugaku (Study on root development) pp. 132, 2000 (Non-patent Document 3)). Thus, it is expected that drought resistance can be conferred to plants by altering shallow-rooted crop plants into a deep-rooted type to improve drought avoidance.

There are three types of drought resistance in crop plants: drought escape, drought tolerance, and drought avoidance. In general, crop plants are most vulnerable to desiccation between the stages of panicle formation and ear emergence. The drought escape type is obtained by altering crop plants into an early-maturing type so that the low-rainfall period does not overlap with the period of panicle formation stage to ear emergence stage. This is the most common method for developing drought-resistant cultivars. There are a number of isolated genes that can be used to control the timing of ear emergence (WO 01/032881 (Patent Document 1); Japanese Patent Application Kokai Publication No. (JP-A) 2002-153283 (unexamined, published Japanese patent application) (Patent Document 2); JP-A (Kokai) 2003-339382 (Patent Document 3); JP-A (Kokai) 2004-089036 (Patent Document 4); JP-A (Kokai) 2004-290190 (Patent Document 5); JP-A (Kokai) 2005-110579 (Patent Document 6)). However, the ear emergence stage is determined for each cultivar, and it would be effective if the change in rainfall during the cultivation period was consistent every year. The problem is that the plants will be affected by drought if the climate shift triggers a drought between the stages of panicle formation and ear emergence. When exposed to drought, the drought-tolerant type has the nature of tolerating drought by controlling cell osmotic pressure or such. Drought tolerance can be assessed simply by not watering plants. Thus, a number of genes related to the drought-tolerant type have been isolated by molecular biology experiments (JP-A (Kokai) 2007-222129 (Patent Document 7); for a review, Tran et al., Methods in Enzymology 428: 109-28. 2007 (Non-patent Document 4)). However, screening for the drought-tolerant type under field conditions is not simple since it is difficult to control environmental conditions such as the soil water content. Thus, breeding of drought-tolerant type cultivars is less-advanced. Furthermore, while the drought-tolerant type has the ability to tolerate drought, the plant growth is suppressed and the plant is eventually killed when the drought period is prolonged since the plant cannot absorb water and nutrients from the soil. This becomes a problem in crop production, where the final product is the grains and it is important to maximize the crop yield rather than to preserve the plants. The drought avoidance type has the nature to acquire drought resistance by avoiding drought stress. The above-described deep rooting is of such nature. In the field, drought generally progresses from ground surface to deeper soil layers. In common agricultural lands, the deeper soil layers contain water. Thus, deep-rooted plants can evade desiccation under drought conditions by using water in the deeper soil layers. In Japan, cultivars with the deep rooting have been developed in upland rice cultivation to avoid drought damage as a result of low rainfall/sunshine following the end of rainy season. "Yumenohatamochi", a deep-rooted cultivar, was developed as a highly drought-resistant upland rice variety with excellent flavor (Hideo Hirasawa et al., Breeding Science 48: 415-419. 1998 (Non-patent Document 5)). However, gene isolation that aims to improve the deep rooting has not been reported.

A variety of genes related to tropism, which is an important factor that controls the direction of root growth, have been isolated by molecular biology techniques using mutants and the like. Tropism refers to root growth with curved extension in response to extrinsic environmental stimuli. Tropism includes gravitropism, phototropism, hydrotropism, haptotropism, galvanotropism, magnetotropism, and chemotropism. The identified *Arabidopsis* genes include a number of gravitropism genes (for a review, Morita and Tasaka Current Opinion in Plant Biology 7(6): 712-718. 2004 (Non-patent Document 6)), as well as hydrotropism genes MIZ1 (Kobayashi et al., Proc. Natl. Acad. Sci. USA 104(11): 4724-4729. 2007 (Non-patent Document 8)) and MIZ2 (Miyazawa et al., Plant Physiology 149(2): 835-840. 2009 (Non-patent Document 9)). With respect to the rice plant, there are only a small number of reports published on genes related to root tropism. The phototropism gene CPT1 (Haga et al., Plant Cell 17(1): 103-115. 2005 (Non-patent Document 7)) and the crown root formation gene Crl1 (Inukai et al., Plant Cell 17(5): 1387-1396. 2005 (Non-patent Document 10)) have been reported to be related to gravitropism. Many of the genes were isolated using loss-of-function mutants or the like. There is no report describing that the deep rooting can be conferred to crop plants by altering these genes.

There have been reports of genetic studies on the deep rooting of corn and rice plant with the aim to avoid drought. With respect to corn, Tuberosa *et al.* and Trachsel *et al.* have discovered a quantitative trait locus (QTL) related to root length (Tuberosa et al., Plant Molecular Biology 48: 697-712. 2002 (Non-patent Document 11); Trachsel et al., Theoretical and Applied Genetics DOI 10.1007/s00122-009-1144-9. 2009 (Non-patent Document 12)). Among rice plant cultivars, there are a broad range of spontaneous mutations in terms of traits such as the deep rooting, root length, or root thickness (Uga Trachsel et al., Breeding Science 59: 87-93. 2009 (Non-patent Document 13)). Various quantitative trait loci (QTL) correlated with root morphological mutations observed among the cultivars have been identified by genetic analysis using molecular markers (for a review, Price et al., Journal of Experimental Botany 53: 989-1004. 2002 (Non-patent Document 14)). The deep rooting is determined by two traits: root length and direction (angle) of root growth. Most reports on QTL involved in the deep rooting are related to root length (for a review, Price et al., Journal of Experimental Botany 53: 989-1004. 2002 (Non-patent Document 14); Courtois et al., Euphytica 134: 335-345. 2003 (Non-patent Document 15); Zheng et al., Theoretical and Applied Genetics 107: 1505-1515. 2003 (Non-patent Document 16); Li et al., Theoretical and Applied Genetics 110: 1244-1252. 2005 (Non-patent Document 17)). Only a single QTL in corn has been reported to be involved in the root growth angle; however, it was simply predicted by a statistical procedure, and the gene has not yet been identified/isolated (Omori F. and Mano Y. Plant Root 1: 57-65. 2007 (Non-patent Document 18)). As described above, there is no report on successful gene identification/isolation for the root-related QTL based on spontaneous mutations. A plausible explanation is that unlike with traits of the aerial part, it is difficult to accurately and reproducibly assess the phenotype of root system morphology. Furthermore, another problem is that experimental studies under field conditions are effort intensive (Yadav et al., Theoretical and Applied Genetics 95: 619-632. 1997 (Non-patent Document 19)).

Known methods for assessing the deep rooting of plants include the trench method, monolith method, and core sampling method. These methods are suitable for assessing the deep rooting in the field (Nemoto et al., Breeding Science 48: 321-324. 1998 (Non-patent Document 20); Masakata Hirayama et al., Nihon Sakumotsu Gakkai Kiji (Japanese Journal of Crop Science) 76: 245-252. 2007 (Non-patent Document 21)). The trench method is a method that determines the thickness and number of roots at each depth, after plant cultivation and then removal of soil from the field. However, the trench method requires considerable efforts to remove soil, and therefore it is not suitable for assessing a large number of plants. In the monolith method, two iron frames are driven into soil at the foot of the cultivated plant. The resulting square monolith (for example, with width of 30 cm x thickness of 5 cm x depth of 30 cm) is excavated, and a chunk of soil is excised for assessing the length and number of roots at each depth. In the core sampling method, metal cylindrical tubes with a diameter of 5 to 8 cm and a length of 30 to 50 cm are driven into soil at the plant foot or between plants; and roots are exposed by washing the resulting soil samples to assess the length and number of roots. Sampling is simpler in both methods than in the trench method; however, these methods cannot accurately assess the condition of roots in soil because there are sampling errors depending on the site. As a method for assessing a large number of plants under an artificial environment such as in a greenhouse, a method for assessing the deep rooting under a drought stress condition in which plants are planted and cultivated in a cylindrical cultivation container (with a diameter of 5 to 10 cm) filled with a cultivation medium has been developed (WO 2006/123392 (Patent Document 8)). This method has revealed that the degree of leaf senescence was smaller in cultivars having the deep rooting than in shallow-rooted cultivars when plants were exposed to drought stress by lowering the water level in a stepwise manner during the cultivation period. As seen from the result described above, this method assesses the deep rooting not directly but based on the senescence of the aerial part. An advantage of the method is simple assessment of the deep rooting without the step of washing soil off the roots. However, in the case of cultivars whose roots are long but extend horizontally, the cultivars may wrongly be assessed to have the deep rooting because their roots extend downward along with the cylinder after reach. Thus, although this method can be used to assess the root length which is one of the properties that constitute the deep rooting, it is difficult to use this method to assess the direction of root growth.

The basket method was developed to assess wheat (Oyanagi et al., Nihon Sakumotsu Gakkai Kiji (Japanese Journal of Crop Science) 62: 565-570. 1993 (Non-patent Document 22)), as a simple quantitative method for assessing the direction of root growth alone. Furthermore, a previous report describes that the basket method was used to assess rice plants for the deep rooting (Kato et al., Plant Soil 287: 117-129. 2006 (Non-patent Document 23)). In this method, a mesh basket is filled with soil, and placed in a field or pot. After a certain period of cultivation, the deep rooting is assessed by measuring the growth angle of root extending through the basket relative to ground surface. This method enables simple assessment of the growth angle; however, it requires more space and extensive water control when assessing a large number of samples at one time for the purpose of gene isolation.

Under the circumstances described above, it is essential to isolate genes that are related to the deep rooting using natural variants and to elucidate the drought avoidance effect of the genes for efficient development of cultivars with improved drought avoidance ability.

Dro1 (Deeper Rooting 1) is a QTL related to the deep rooting, which was statistically predicted to be located on the long arm of chromosome 9 by QTL analysis with progeny line (BC₂F₂) resulting from backcross of the upland rice cultivar of tropical *japonica,* Kinandang Patong, using an *indica* paddy rice cultivar IR64 as a recurrent parent (Uga et al., The 2nd International Conference on Plant Molecular Breeding. 2007 (Non-patent Document 24); Uga et al., Nihon Ikusyu Gakkai Dai 112 Kai Kouenkai Youshisyu (112nd Meeting of The Japanese Society of Breeding, Program and Abstracts) PP. 188, 2007 (Non-patent Document 25); Uga et al., Dai 27 Kai Ne Kenkyu Syukai (27th Research Meeting of The Japanese Society for Root Research), 2007 (Non-patent Document 26); Uga et al. The 5th International Crop Science Congress Abstracts 243 p. 2008 (Non-patent Document 27)). IR64 is a difficult cultivar for gene introduction by the Agrobacterium transformation method. Currently, the Agrobacterium transformation method, in which dedifferentiated culture tissues (for example, calluses) are used as a plant sample, is commonly used for rice plants (Japanese Patent No. 2649287 (Patent Document 9)). With respect to IR64, however, the method using calluses only gives a very low transformation efficiency Therefore, the callus-based Agrobacterium transformation method has not yet been established for IR64 (Hiei and Komari, Nature Protocols 3: 824-834. 2008 (Non-patent Document 28)). Hiei and Komari have reported a method using immature embryos instead of calluses as a plant sample to achieve IR64 transformation. However, since the immature embryo method requires immature embryos after anthesis, it is necessary to prepare the plant always on a paddy field or in a greenhouse so that immature embryos can be made available immediately when needed. For example, seeds can be planted every two weeks so that immature embryos are always available; therefore, a vast cultivation area and significant workforce are essential for keeping the plants. Thus, the establishment of gene transfer into IR64 based on the callus-based Agrobacterium transformation method is very important for achieving the practical use of molecular breeding.

### [Prior Art Documents]

### [Patent Documents]

[Patent Document 1] WO 01/032881 (Plant photosensitive gene Hd1 and use thereof)
[Patent Document 2] JP-A (Kokai) 2002-153283 (Plant anthesis-inducing gene Hd3a and use thereof)
[Patent Document 3] JP-A (Kokai) 2003-339382 (Plant anthesis-enhancing gene Ehdl and use thereof)
[Patent Document 4] JP-A (Kokai) 2004-089036 (Plant anthesis-enhancing gene RFT1 and methods for predicting the time of bloom)
[Patent Document 5] JP-A (Kokai) 2004-290190 (Plant anthesis-controlling gene Lhd4 and use thereof)
[Patent Document 6] JP-A (Kokai) 2005-110579 (Plant photosensitive gene Hd5 and use thereof)
[Patent Document 7] JP-A (Kokai) 2007-222129 (Methods for producing plants resistant to environmental stress)
[Patent Document 8] WO 2006/123392 (Methods for assessing deep rooting of plants)
[Patent Document 9] Japanese Patent No. 2649287
[Patent Document 10] US 2007/039076
[Patent Document 11] US 2004/123343
[Patent Document 12] US 2006/123505
[Patent Document 13] US 2006/253938
[Patent Document 14] EP 1 886 552

### [Non-patent Documents]

[Non-patent Document 1] Morita S., (2003) Ne no Dezain (Root design), pp. 107, Yokendo.
[Non-patent Document 2] Yoshida S. and Hasegawa S. (1982) The rice root system: its development and function. In "Drought resistance in crops with emphasis on rice" International Rice Research Institute, Los Banos, Laguna, Philippines. p. 97-114.
[Non-patent Document 3] Morita S., (2000) Ne no Hatsuikugaku (Study on root development) pp. 132, University of Tokyo Press.
[Non-patent Document 4] Tran L. S., Nakashima K., Shinozaki K., and Yamaguchi-Shinozaki K. (2007) Plant gene networks in osmotic stress response: from genes to regulatory networks. Methods in Enzymology 428: 109-28.
[Non-patent Document 5] Hideo Hirasawa, Hiroshi Nemoto, Tatsuo Suga, Masatoshi Ishihara, Masakata Hirayama, Kazuyuki Okamoto, and Masaru Miyamoto, (1998) Development of "Yumenohatamochi", a highly drought resistant upland rice variety with excellent eating quality, Breeding Science 48: 415-419.
[Non-patent Document 6] Morita M. T., Tasaka M. (2004) Gravity sensing and signaling. Current Opinion in Plant Biology 7(6): 712-718.
[Non-patent Document 7] Haga K., Takano M., Neumann R., Iino M. (2005) The Rice COLEOPTILE PHOTOTROPISM1 gene encoding an ortholog of Arabidopsis NPH3 is required for phototropism of coleoptiles and lateral translocation of auxin. Plant Cell 17(1): 103-115.
[Non-patent Document 8] Kobayashi A., Takahashi A., Kakimoto Y, Miyazawa Y., Fujii N., Higashitani A., Takahashi H. (2007) A gene essential for hydrotropism in roots. Proc. Natl. Acad. Sci. USA 104(11): 4724-4729.
[Non-patent Document 9] Miyazawa Y., Takahashi A., Kobayashi A., Kaneyasu T., Fujii N., Takahashi H. (2009) GNOM-mediated vesicular trafficking plays an essential role in hydrotropism of Arabidopsis roots. Plant Physiology 149(2): 835-840.
[Non-patent Document 10] Inukai Y., Sakamoto T., Ueguchi-Tanaka M., Shibata Y., Gomi K., Umemura I., Hasegawa Y., Ashikari M., Kitano H., Matsuoka M. (2005) Crown rootless1, which is essential for crown root formation in rice, is a target of an AUXIN RESPONSE FACTOR in auxin signaling. Plant Cell 17(5): 1387-1396.
[Non-patent Document 11] Tuberosa R., Sanguineti M. C., Landi P., Giuliani M. M., Salvi S., Conti S. (2002) Identification of QTLs for root characteristics in maize grown in hydroponics and analysis of their overlap with QTLs for grain yield in the field at two water regimes. Plant Molecular Biology 48: 697-712.
[Non-patent Document 12] Trachsel S., Messmer R., Stamp P., Hund A. (2009) Mapping of QTLs for lateral and axile root growth of tropical maize. Theoretical and Applied Genetics DOI 10.1007/s00122-009-1144-9.
[Non-patent Document 13] Uga Y., Ebana K., Abe J., Morita S., Okuno K., Yano M. (2009) Variation in root morphology and anatomy among accessions of cultivated rice (Oryza sativa L.) with different genetic backgrounds. Breeding Science 59: 87-93.
[Non-patent Document 14] Price A. H., Cairns J. E., Horton P., Jones H. G., Griffiths H. (2002) Linking drought-resistance mechanisms to drought avoidance in upland rice using a QTL approach: progress and new opportunities to integrate stomatal and mesophyll responses. Journal of Experimental Botany 53: 989-1004.
[Non-patent Document 15] Courtois B., Shen L., Petalcorin W., Carandang S., Mauleon R. Li Z. (2003) Locating QTLs controlling constitutive root traits in the rice population IAC 165 x Co39. Euphytica 134: 335-345.
[Non-patent Document 16] Zheng B. S., Yang L., Zhang W. P., Mao C. Z., Wu Y. R., Yi K. K., Liu F. Y., Wu P. (2003) Mapping QTLs and candidate genes for rice root traits under different water-supply conditions and comparative analysis across three populations. Theoretical and Applied Genetics 107: 1505-1515.
[Non-patent Document 17] Li Z., MuP. , Li C., Zhang H., Li Z., Gao Y., Wang X. (2005) QTL mapping of root traits in a doubled haploid population from a cross between upland and lowland japonica rice in three environments. Theoretical and Applied Genetics 110: 1244-1252.
[Non-patent Document 18] Omori F., Mano Y. (2007) QTL mapping of root angle in F2 populations from maize 'B73' × teosinte 'Zea luxurians' Plant Root 1: 57-65.
[Non-patent Document 19] Yadav R., Courtois B., Huang N., McLaren G. (1997) Mapping genes controlling root morphology and root distribution in a doubled-haploid population of rice. Theoretical and Applied Genetics 95: 619-632.
[Non-patent Document 20] Nemoto, H., Suga, R., Ishihara M., Okutsu Y. (1998) Deep rooted rice varieties detected through the observation of root characteristics using the trench method. Breeding Science 48: 321-324.
[Non-patent Document 21] Masakata Hirayama, Hiroshi Nemoto, and Hideo Hirasawa (2007) Hojosaibaisita Nakate Okute Jukuki Nippon Rikutou Hinshu no Konkei Hattatsu Teido to Taikansei tono Kankei (Relation between the degree of root system development and drought resistance in field-cultivated medium maturing and late maturing Japanese upland rice varieties), Nihon Sakumotsu Gakkai Kiji (Japanese Journal of Crop Science) 76: 245-252.
[Non-patent Document 22] Oyanagi A., Nakamoto T., Wada M. (1993) Relationship between root growth angle of seedlings and vertical distribution of roots in the field in wheat cultivars. Nihon Sakumotsu Gakkai Kiji (Japanese Journal of Crop Science) 62: 565-570.
[Non-patent Document 23] Kato Y., Abe J., Kamoshita A., Yamagishi J. (2006) Genotypic variation in root growth angle in rice (Oryza sativa L.) and its association with deep root development in upland fields with different water regimes. Plant Soil 287: 117-129.
[Non-patent Document 24] Uga Y., K. Okuno and M. Yano (2007) Relationship between QTLs for vascular system and vertical distribution of roots on chromosome 9 of rice. The 2nd International Conference on Plant Molecular Breeding.
[Non-patent Document 25] Yusaku Uga, Kazutoshi Okuno, and Masahiro Yano, (2007) Ine Dai 9 Sensyokutai jyouni Miidasareta Shinkonsei nikansuru QTL (QTL involved in the deep rooting, found on rice chromosome 9), Nihon Ikusyu Gakkai Dai 112 Kai Kouenkai Youshisyu (112nd Meeting of The Japanese Society of Breeding, Program and Abstracts) 188 p.
[Non-patent Document 26] Yusaku Uga, Kazutoshi Okuno, and Masahiro Yano, (2007) Ine Shinkonsei kanren Idenshiza Dro2 no Fain Mappingu (Fine mapping of rice deep rooting-related locus Dro1), Dai 27 Kai Ne Kenkyu Syukai (27th Research Meeting of The Japanese Society for Root Research).
[Non-patent Document 27] Uga Y., K. Okuno and M. Yano (2008) Fine mapping of a deeper-rooting QTL, Dro1, on chromosome 9 in rice. The 5th International Crop Science Congress Abstracts 243 p.
[Non-patent Document 28] Hiei Y., Komari T (2008) Agrobacterium-mediated transformation of rice using immature embryos or calli induced from mature seed. Nature Protocols 3: 824-834.
[Non-patent Document 29] Yano M., 2nd International Symposium on Genomics of Plant Genetic Resources (Italy), Apr. 24, 2010
[Non-patent Document 30] Yano M., Gatersleben Lecture (Institute of Plant Genetics and Crop Plant Research (Germany), Apr. 29, 2010
[Non-patent Document 31] Yano M., Third International Conference of Plant Molecular Breeding, Sep. 7, 2010
[Non-patent Document 32] Yusaku Uga, Nihon Ikusyu Gakkai Dai 118 Kai Kouenkai (118th Meeting of The Japanese Society of Breeding), Sept. 24, 2010; Ikusyugaku Kenkyu Dai 12 Gou Bessatsu 2 Gou (Breeding Research No. 12, supplement No. 2), pp. 21
[Non-patent Document 33] Yusaku Uga, CIAT (International Center of Tropical Agriculture, Colombia) niokeru Teiki Semina (Regular Meeting of CIAT), Oct. 29, 2010
[Non-patent Document 34] DATABASE Geneseq, GSN: AQD24617
[Non-patent Document 35] DATABASE UNIPROT: Q69P88
[Non-patent Document 36] DATABASE UNIPROT: C5XDP7
[Non-patent Document 37] DATABASE EMBL, EM_STD: EU969762

### Summary of the Invention

### [Problems to be Solved by the Invention]

The present invention was achieved in view of the above circumstances. An objective of the present invention is to provide methods for altering the plant root morphology to confer drought resistance to plants by improving the drought avoidance ability. More specifically, an objective of the present invention is to provide methods for conferring a deep rooting trait to plants, transformed plants having a deep rooting trait, methods for producing such plants, and methods for assessing whether or not a plant has a deep rooting trait.

### [Means for Solving the Problems]

A candidate region for Dro1 is located within a chromosomal region of 1,443 kbp between insertion-deletion (InDel) markers ID07_14 and ID07_17 (based on the nucleotide sequence in Nipponbare). The region has been predicted to contain 166 genes from RAP-DB. These genes have not been proven to include any gene previously reported to be related to the deep rooting of rice plant or other plants, or homologs thereof. Thus, it would be impossible to predict gene candidates based on functions of the putative genes. Furthermore, since the nucleotide sequences of the two cultivars, IR64 and Kinandang Patong, had not been determined, it would be difficult to predict genes based on differences in the nucleotide sequences of these genes. Under the circumstances described above, the present inventors aimed to identify and isolate the Dro1 gene. First, using a large-scale segregating population of about 4,500 plants, the present inventors performed a high-resolution linkage analysis for the locus (Dro1 locus) responsible for the determination of the deep rooting detected in plants from crossing IR64, a shallow-rooted rice cultivar, with Kinandang Patong, a deep-rooted rice cultivar. A conventional basket method was improved to test several hundred plants at one time for their deep rooting ratio (percentage of roots that penetrated the basket's bottom area relative to the total number of roots that penetrated the basket). This allowed examination of 500 plants at one time. However, it was impossible to examine at one time a greater number of plants than the above. Several lines were selected from a group of hybrid lines selected from the large-scale population, and the candidate region was narrowed step by step using the phenotype/genotype data for 40 plants per line. After narrowing the candidate region from the 1,443 kbp region to about 100 kbp, the inventors constructed and screened bacterial artificial chromosome (BAC) libraries of IR64 and Kinandang Patong covering the 100-kbp candidate region. Both BACs for the cultivars were analyzed to identify their nucleotide sequences. To consider whether it is possible to narrow down the candidate genes by nucleotide sequence comparison, the nucleotide sequences of RAP-DB-predicted genes for the two cultivars were compared. However, many genes have mutations in their exon nucleotide sequences, and therefore it was impossible to narrow down the candidate genes. Then, the present inventors performed linkage analysis for a total of five times, while producing DNA markers based on mutations in the nucleotide sequence of the candidate region. As a result, the Dro1 gene region was narrowed down to a region of 6.0 kbp between insertion-deletion (InDel) marker Dro1-INDEL09 (primer 5'-GCAGACGCTCGTAACACGTA -3' ((SEQ ID NO: 4) and primer 5'-GTGGCAGCTCCATCAACTCT -3' (SEQ ID NO: 5)) and Cleaved Amplified Polymorphic Sequences (CAPS) marker Dro1-CAPS05 (primer 5'- GCACAAGATGGGAGGAGAGT -3' (SEQ ID NO: 6) and primer 5'- CATGGGTGAGAATCGTGTTG -3' (SEQ ID NO: 7); the amplified DNA was treated with restriction enzyme *Hinf*I)*.* This region only contains a single putative gene predicted in RAP-DB. The putative gene was deduced to encode a protein with unknown function. Since it is very difficult to transform IR64, before performing a complementation test using the transformation method, whether the predicted gene is actually related to the deep rooting was assessed. First, to assess whether the gene has a function related to the deep rooting, its function was predicted based on the amino acid sequence of the putative gene on the following several websites for functional search:
"SALAD database": Database for genome-wide comparison of plant protein sequences (salad.dna.affrc.go.jp/salad/)
"Pfam": Protein domain database (pfam.janelia.org/)
"PSORT": Site for predicting protein hydrophobicity and localization (www.psort.org/) "InterPro": Database for protein families, domains, functional sties (www.ebi.ac.uk/interpro/) However, not one conserved domain can be found using any of these sites. Then, the gene was examined to reveal whether its exons have nucleotide sequence variations between IR64 and Kinandang Patong. The result showed that IR64 had a 1 b deletion in exon 4 while Kinandang Patong had an adenine at the same position. This 1 b deletion causes a frame shift, resulting in a premature stop codon. The present inventors suspected that the deletion reduced the expression level of the candidate gene in IR64. Thus, the expression level was assessed using leaf blades and an upper portion of leaf sheath, a basal portion of leaf sheath, and crown roots of IR64 and Kinandang Patong on days 6 and 12 after germination. Total RNA was extracted from the three types of tissues, and the gene expression level was determined by real-time PCR. The result showed that in these two cultivars, the gene was expressed in the basal portion of leaf sheath but hardly expressed in both the crown root and leaf blade/upper portion of leaf sheath. The expression levels were compared between IR64 and Kinandang Patong. The samples showed that the difference between the two cultivars was only less than twice both on days 6 and 12. Whether the predicted gene was the true Dro1 gene could not be judged from the result on expression level. Nonetheless, the gene remained to be a candidate, because the basal portion of leaf sheath contained the crown root primordium, which is the rudiment of crown root, or alternatively because the gene's function was potentially different between the two cultivars at the amino acid level.

Next, whether the gene is related to the deep rooting was assessed based on the correlation between the deep rooting ratio and the 1 b deletion in exon 4. Sixty four cultivars including IR64 and Kinandang Patong, 22 IR cultivar lines developed by the International Rice Research Institute (Los Banos, Philippines; IR64 is also a variety developed by this Institute), and 20 wild type lines (seven *Oryza nivara* lines, twelve *O. rufipogon* lines, and one *O*. *meridionalis* line) were analyzed for their nucleotide sequences around the 1 b-deletion site. The nucleotide sequence comparison revealed that IR64 alone contained the 1 b deletion of exon 4. Thus, whether the predicted gene is the true Dro1 gene could not be judged based on the correlation between the 1 b deletion and deep rooting ratio.

The nucleotide sequence of Dro1 complementary DNA (cDNA) was fully analyzed for 14 varieties of *japonica* including Kinandang Patong. The nucleotide sequence was identical among all of the varieties. Meanwhile, the deep rooting ratio determined by the basket method varied greatly from 15.9% (at the lowest by Tupa729) to 83.1% (at the greatest by Kinandang Patong). This could be explained by the presence of other QTL responsible for the deep rooting. Alternatively, some differences in the nucleotide sequence of the promoter region could affect the regulation of gene expression. Thus, the nucleotide sequence up to about 2.2 kb upstream (up to the 5' upstream end of the candidate region) was compared between Kinandang Patong and Nipponbare. Mutations were found at two sites. One is located 1,250 bp upstream of the 5'-untranslated region; the nucleotide at this position is thymine in the nucleotide sequence of Nipponbare, and substituted with guanine in Kinandang Patong. The sequence adjacent to the mutation was analyzed on PLACE (A Database of Plant Cis-acting Regulatory DNA Elements; www.dna.affrc.go.jp/PLACE/), a website for searching promoter regions for *cis* elements. This analysis revealed that thymine was replaced with guanine in a motif called "GATA box". The other mutation was a deletion of 30 bp from 1,189 bp to 1,218 bp upstream of the 5' untranslated region in Kinandang Patong. These mutations in the nucleotide sequence were a potential cause of the deep rooting mutation among the cultivars.

A full-length cDNA for the predicted Nipponbare gene in the 6.0-kbp region has been deposited under AK068870. Thus, the FOX hunting system (Full-length cDNA Over-eXpressing gene hunting system) was searched for the Nipponbare gene. As a result, the search revealed two lines of T1 generation and two lines of T2 generation. Then, the deep rooting ratio was determined for the four lines using five plants per line. Several plants were demonstrated to have a deep rooting ratio significantly higher than that of Nipponbare (wild type) as a control. This result increases the likelihood that the predicted gene is the true Dro1 gene. To verify that the predicted gene is absolutely the true Dro1 gene, the present inventors produced transformants by introducing into IR64 calluses a vector carrying the predicted gene (Kinandang Patong-type Dro1 gene). The transformation efficiency was 1/10 or less than that of a conveniently transformed cultivar such as Nipponbare. Thus, the present inventors carried out gene transfer after preparing more than ten times as many calluses for the transformation. The resulting plant transformants were assessed for their deep rooting ratio. The plants transformed with the predicted gene were demonstrated to have a higher deep rooting ratio.

These two experiments described above showed that the predicted gene in the candidate region of 6.0 kbp was the true Dro1. Thus, the present inventors for the first time successfully identified and isolated the deep rooting gene Dro1.

Furthermore, using IR64 and a near-isogenic line (Dro1-NIL) which has the same genetic background as IR64 except the region adjacent to Dro1 which was replaced with that of Kinandang Patong, the present inventors assessed whether the deep rooting due to Dro 1 is related to the improvement of drought resistance. The result demonstrated that Dro 1-NIL became resistant to drought as a result of deep rooting, leading to a significant increase in the yield relative to IR64.

The present invention provides subject-matter as defined in the claims.

Based on the findings described above, the present invention or description, respectively, provides:
[1] a DNA of any one of (a) to (e) below:
   (a) a DNA comprising the nucleotide sequence of SEQ ID NO: 1;
   (b) a DNA comprising a coding region of the nucleotide sequence of any one of SEQ ID NOs: 1, 2, 12, 14, 16, and 17;
   (c) a DNA that encodes a protein comprising the amino acid sequence of any one of SEQ ID NOs: 3, 13, and 15;
   (d) a DNA that hybridizes under a stringent condition to a DNA comprising the nucleotide sequence of any one of SEQ ID NOs: 1, 2, 12, 14, 16, and 17, and has an activity of conferring a deep rooting phenotype to a plant; or
   (e) a DNA that encodes a protein comprising an amino acid sequence with one or more amino acid substitutions, deletions, additions, and/or insertions in the amino acid sequence of any one of SEQ ID NOs: 3, 13, and 15, and has an activity of conferring a deep rooting phenotype to a plant;
[2] the DNA of [1], wherein the plant is a monocotyledon;
[3] the DNA of [2], wherein the monocotyledon is a gramineous plant;
[4] the DNA of [3], wherein the gramineous plant is selected from the group consisting of rice, wheat variety (wheat, barley, rye, oat, and Job's tears (hatomugi)), corn, millet, foxtail millet, Japanese millet, sorghum, finger millet, pearl millet, teff, sugarcane, timothy, Kentucky bluegrass, orchardgrass, Italian rye grass, perennial ryegrass, tall fescue, and Bahia grass;
[5] The DNA of [3], wherein the gramineous plant is selected from the group consisting of rice, sorghum, and corn;
[6] a vector comprising the DNA of any one of [1] to [5];
[7] a transformed cell harboring the DNA of any one of [1] to [5] in an expressible manner;
[8] a plant transformed with the DNA of any one of [1] to [5], which has a deep rooting phenotype;
[9] a transformed plant produced by introducing into a plant cell the DNA of any one of [1] to [5] or the vector of [6], which has a deep rooting phenotype;
[10] a transformed plant which is obtained by the steps of (a) to (d) below:
   (a) introducing into a plant cell the DNA of any one of [1] to [5] or the vector of [6;
   (b) determining the copy number of the DNA of any one of [1] to [5] in the plant cell of step (a);
   (c) selecting a transformed plant cell containing the introduced DNA or vector in a single copy;and
   (d) regenerating a plant from the transformed plant cell selected in step (c), and which has a deep rooting phenotype;
[11] the plant of any one of [8] to [10], wherein the plant is a monocotyledon;
[12] the plant of [11], wherein the monocotyledon is a gramineous plant;
[13] the plant of [12], wherein the gramineous plant is selected from the group consisting of rice, wheat variety (wheat, barley, rye, oat, Job's tears (hatomugi)), corn, millet, foxtail millet, Japanese millet, sorghum, finger millet, pearl millet, teff, sugarcane, timothy, Kentucky bluegrass, orchardgrass, Italian ryegrass, perennial ryegrass, tall fescue, and Bahia grass;
[14] the plant of [12], wherein the gramineous plant is selected from the group consisting of rice, sorghum, and corn;
[15] a transformed plant which is a progeny or clone of the transformed plant of any one of [8] to [14];
[16] a cell isolated from the transformed plant of any one of [8] to [15];
[17] a propagation material of the transformed plant of any one of [8] to [15];
[18] an organ isolated from the transformed plant of any one of [8] to [15];
[19] a processed food prepared from at least one of the cell of [16], the propagation material of [17], and the organ of [18];
[20] a method for producing the transformed plant of any one of [8], and [11] to [13], which comprises the steps of introducing into a plant cell the DNA of any one of [1] to [5] or the vector of [6], and regenerating a plant from the plant cell;
[21] the method of [20], which further comprises the step of selecting a transformed plant cell or transformed plant, which has the DNA of any one of [1] to [5] in a single copy;
[22] a method for assessing whether a plant has a deep rooting phenotype, wherein a test plant is judged to have a deep rooting phenotype when a molecular weight or nucleotide sequence is identical, and which comprises the steps of (a) to (c) below:
   (a) preparing a DNA sample from a test plant;
   (b) amplifying from the DNA sample a region comprising the DNA of any one of [1] to [5]; and
   (c) comparing the molecular weight or nucleotide sequence of the amplified DNA fragment with that of the DNA of any one of [1] to [5];
[23] a method for assessing whether a plant has a deep rooting phenotype, wherein a test plant is judged to have a deep rooting phenotype when an amplified product is obtained, which comprises the step of carrying out PCR with a primer comprising the nucleotide sequence of SEQ ID NO: 8 and a primer comprising the nucleotide sequence of SEQ ID NO: 9 using a genomic DNA prepared from the test plant as a template;
[24] a method for assessing whether a plant has a deep rooting phenotype, wherein a test plant is judged not to have a deep rooting phenotype when an amplified product is obtained, which comprises the step of carrying out PCR with a primer comprising the nucleotide sequence of SEQ ID NO: 10 and a primer comprising the nucleotide sequence of SEQ ID NO: 11 using a genomic DNA prepared from the test plant as a template;
[25] a method for selecting a plant having a deep rooting phenotype, which comprises the steps of (a) and (b) below:
   (a) producing a cultivar by crossing an arbitrary plant with a plant having a deep rooting phenotype; and
   (b) assessing by the method of any one of [22] to [24] whether a plant obtained in step (a) has a deep rooting phenotype;
[26] a protein encoded by the DNA of any one of [1] to [5];
[27] an antibody that binds to the protein of [26];
[28] a DNA comprising at least 15 consecutive nucleotides complementary to the DNA of any one of [1] to [5] or a complementary sequence thereof; and
[29] a DNA comprising the nucleotide sequence of any one of SEQ ID NOs: 4 to 11.

### [Effects of the Invention]

The Dro1 gene, which controls the deep rooting of plants such as rice plants, and plants transformed with the gene were used in the present invention. The gene in connection with the present invention can be used to manipulate the plant root system morphology from a shallow rooting to deep rooting or from a deep rooting to shallow rooting. Specifically, drought resistance can be conferred by improving the drought avoidance ability, for example, by manipulating the Dro1 gene to convert a shallow-rooted plant into a deep rooting plant. Droughts have caused serious reductions in the world crop yield. Major overseas enterprises have focused on the development of drought-tolerant crop plants. On the other hand, wet resistance can be conferred through conversion of a deep rooted plant into shallow rooting by manipulating the Dro1 gene. In Japan, the shift in agriculture policy recommends upland cultivation in fallow rice fields. However, since paddy fields have poor drainage efficiency, wet damage has been problematic for soy and corn without wet resistance. Therefore, the conversion of crop plants into a shallow rooted type has been studied with the aim to improve wet resistance. Under these international and domestic circumstances, it is very important to develop crop plant varieties that are resistant to drought or wet damage by using genes that control the plant root morphology.

### Brief Description of the Drawings

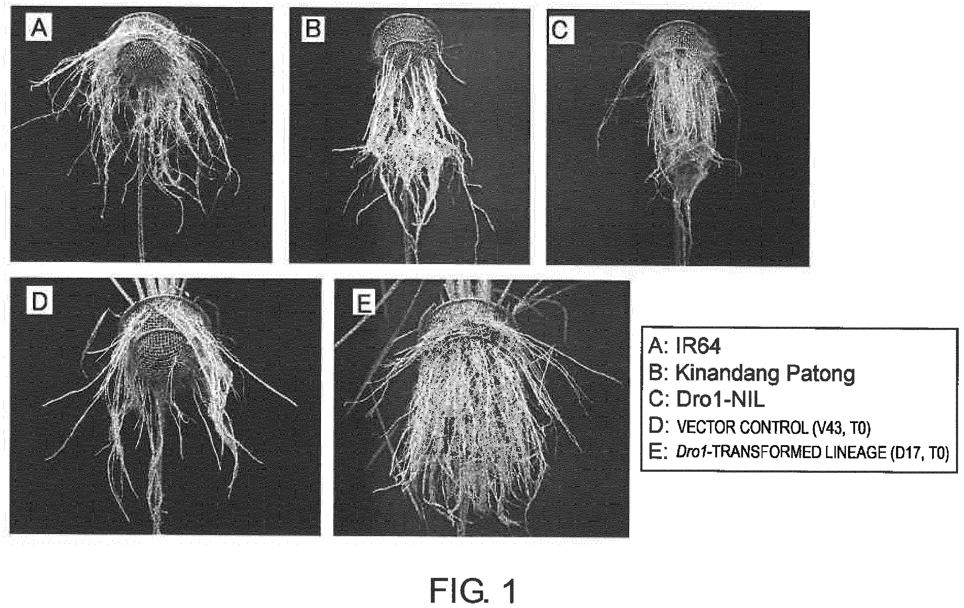

Fig. 1 presents a photograph showing assessment of each lineage for the deep rooting using an improved basket method. A ring is attached to a stainless-steel basket. The ring is arranged at a position so that the roots are judged to be deep roots when elongating at an angle of 50 degrees or deeper with respect to ground surface. In each legend, the first and second abbreviations refer to the lineage name and generation, respectively.

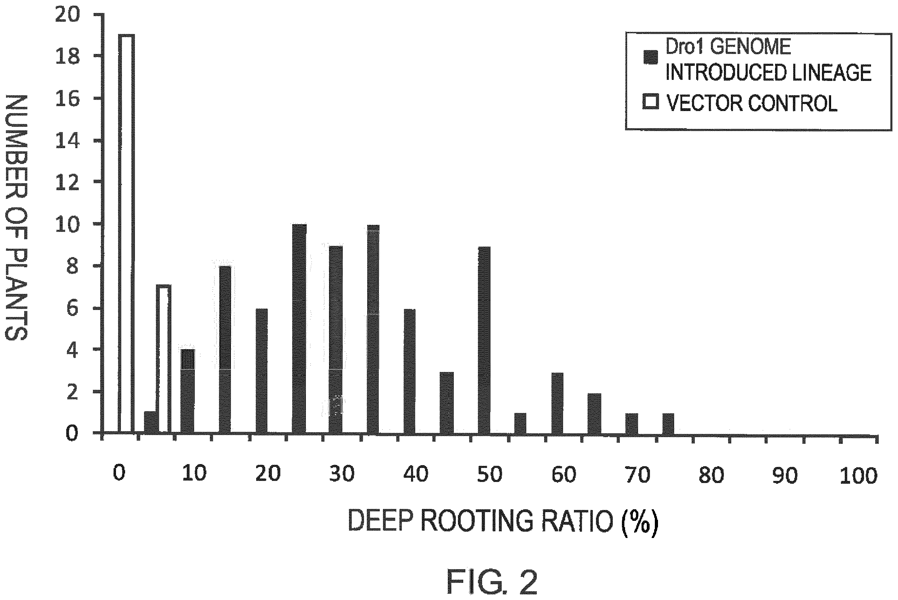

Fig. 2 presents a graph showing distribution of the deep rooting ratio in the T0 generation of the IR64 transformants introduced with a vector carrying Dro1 or the vector alone.

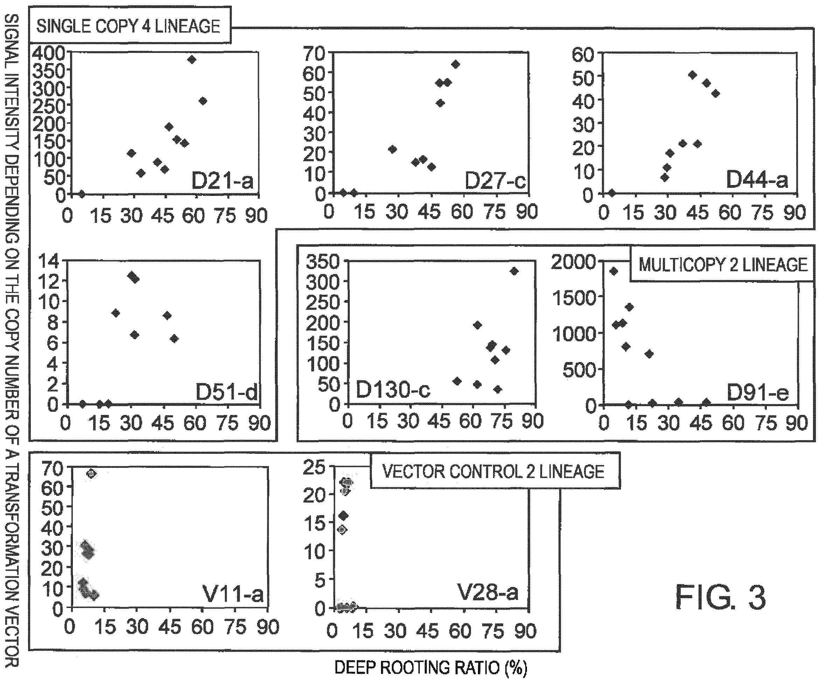

Fig. 3 presents graphs showing the relationship between the deep rooting ratio and the signal intensity (real-time PCR) according to the copy number of the transformation vector in the T1 generation of the IR64 transformants introduced with a vector carrying Dro1 or the vector alone. Single copy and multicopy refer to a lineage introduced with only a single copy of Dro 1 and a lineage introduced with multiple copies of Dro1 in the T0 generation, respectively. In the T1 generation, the single-copy lineage is separated into null type (0 copy), heterozygote (one copy), and homozygote (two copies), and the signal intensities are grouped into three types according to the number of copies. However, unlike Southern analysis, real-time PCR, in principle, does not always give the same signal intensity even if the copy number is the same among plants. The abbreviation in each graph shows the lineage number for the respective clone.

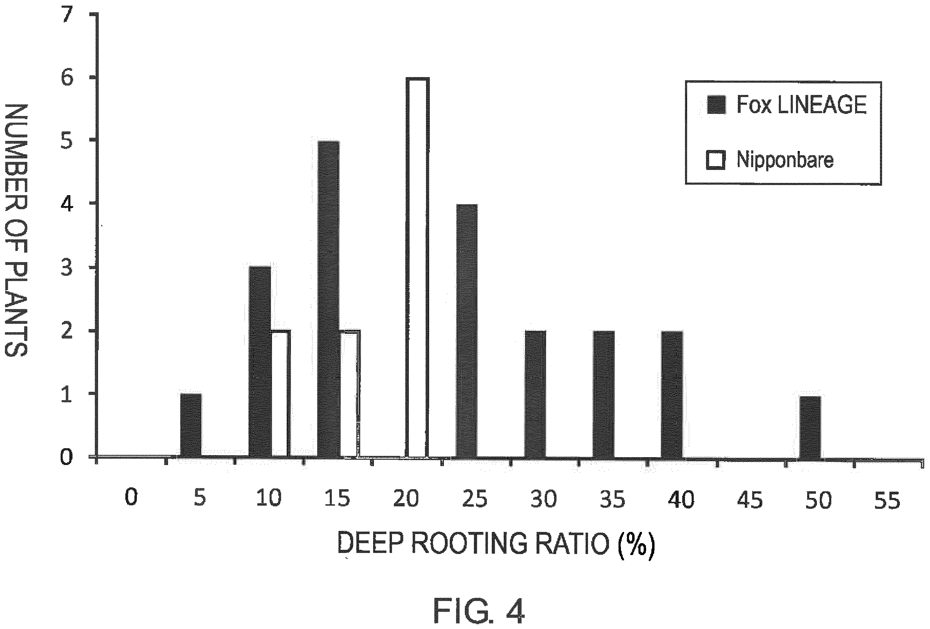

Fig. 4 presents a graph showing the distributions of the deep rooting ratio in Fox lineage and Nipponbare.

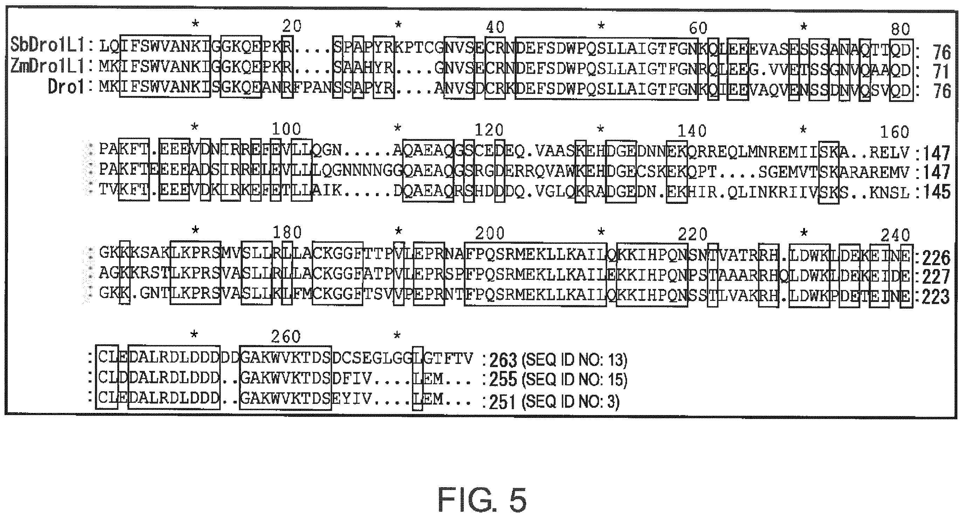

Fig. 5 presents a diagram showing the result of analyzing the sorghum and corn orthologs. Dro1, rice (Kinandang Patong); SbDro1L1, sorghum ortholog of Dro1; ZmDro1L1, corn ortholog of Dro1.

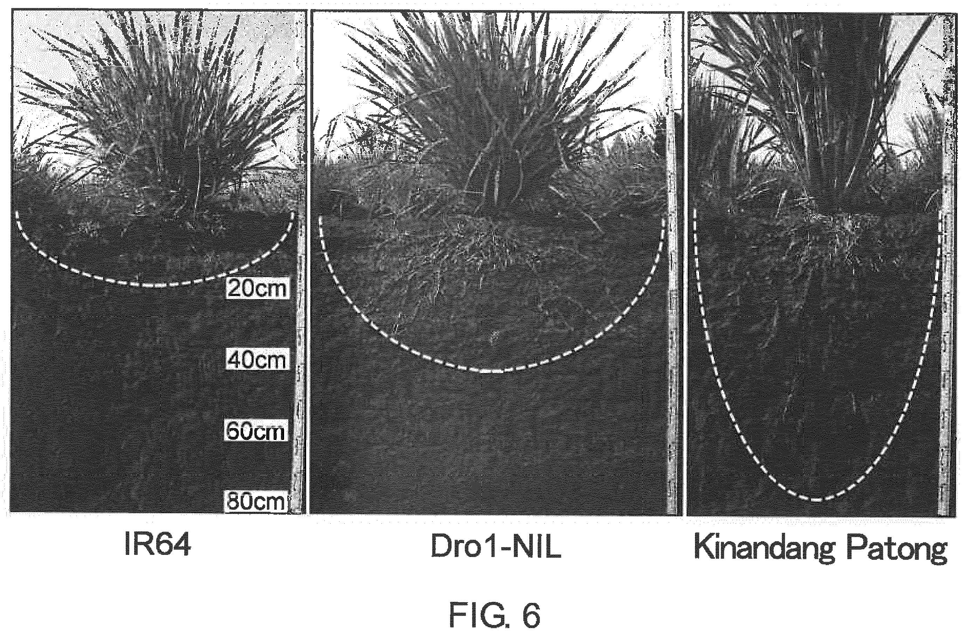

Fig. 6 presents photographs showing comparison of the deep rooting of Dro1-NIL with those of IR64 and Kinandang Patong in a field. The white numeral shows the soil depth below ground surface. The white dashed line indicates an outline of the distribution area of the root system.

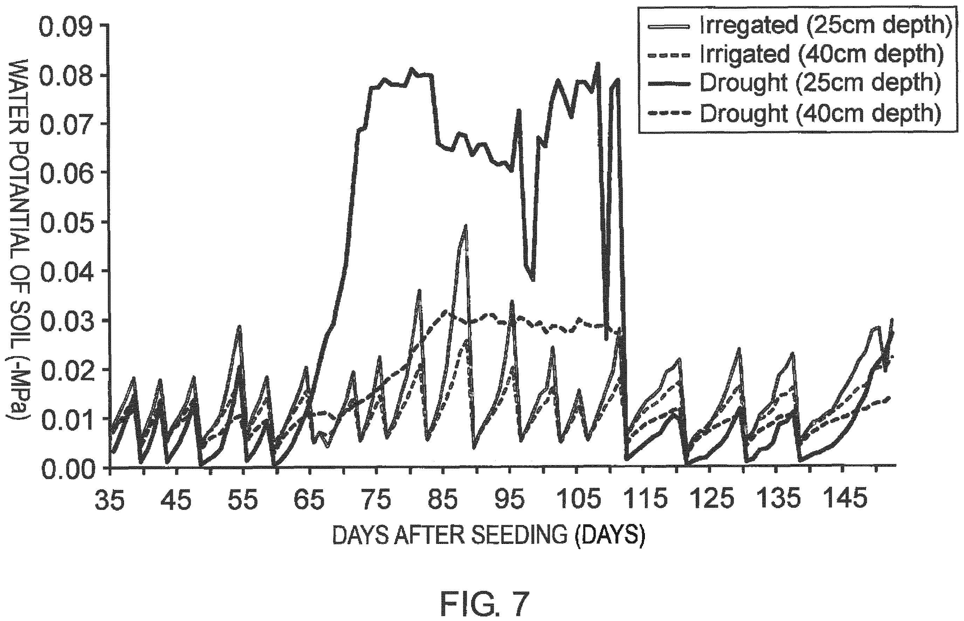

Fig. 7 presents a graph showing the time course of soil water potential in the experimental field for a drought resistance test. "Irregated" and "Drought" indicate the irrigation area and drought stress area, respectively. The soil depths where the water potential was monitored are shown in parentheses. At a soil depth of 25 cm in the drought stress area, a rapid progression of soil desiccation is observed about 70 days after seeding.

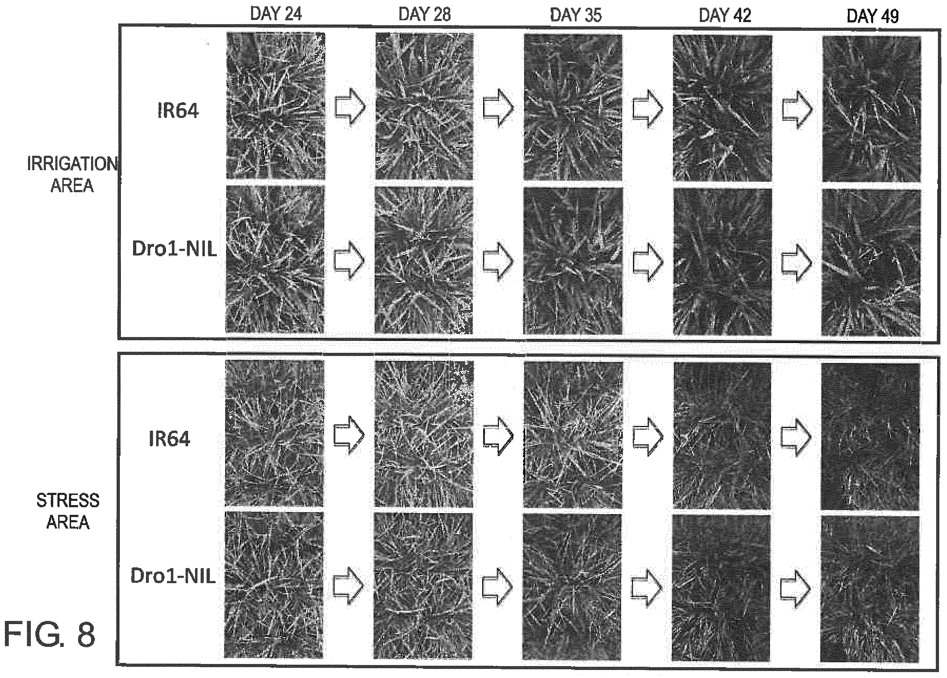

Fig. 8 presents photographs showing time-dependent difference in resistance to leaf rolling between IR64 and Dro1-NIL under a drought stress condition. The plant photographs are views taken from straight above. The numbers of days shown at the top of the figure are the days of stress treatment after irrigation was terminated in the drought stress area.

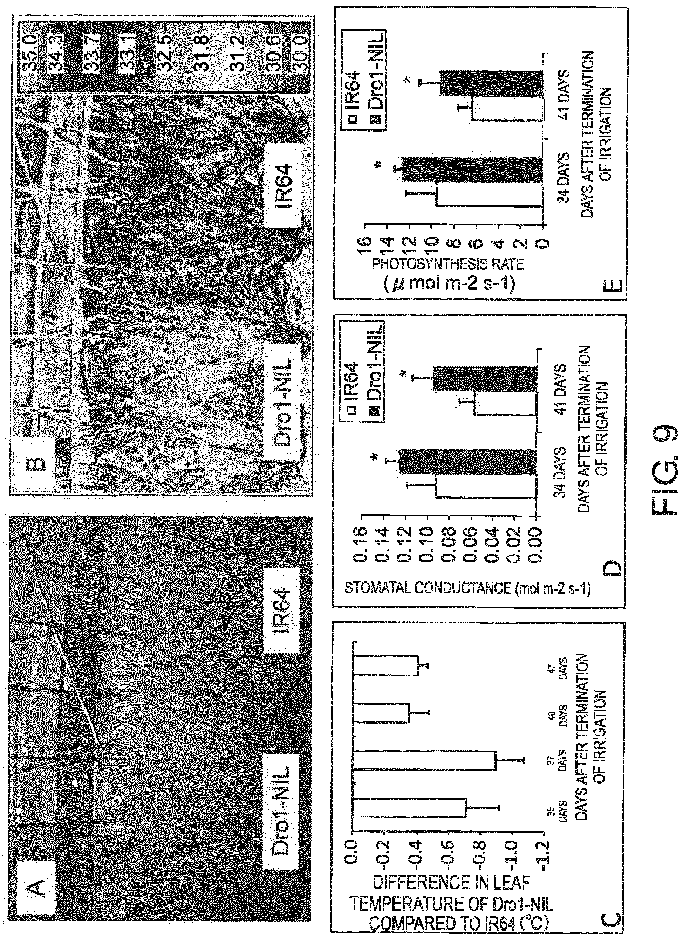

Fig. 9 presents graphs and photographs showing differences in the leaf temperature, stomatal conductance, and photosynthesis rate between IR64 and Dro1-NIL under a drought stress condition. A shows a visible image of rice plants 35 days after termination of irrigation. The first and second lines from right are IR64; the third and fourth are Dro1-NIL. B shows an image showing the temperature distribution of the rice plants 35 days after termination of irrigation (an infrared thermography image taken for the same plants as shown in A). The first and second lines from right are IR64; the third and fourth are Dro1-NIL. The color difference implies the following: as the color is warmer, the temperature is higher; as the color is cooler, the temperature is lower. This photograph suggests that the leaf temperature is different between IR64 and Dro1-NIL (the leaf temperature of Dro1-NIL is on average 0.7°C lower than that of IR64). The cool colors are distributed more widely in Dro1-NIL, suggesting that the leaf temperature of Dro1-NIL is lower than that of IR64. C shows changes in the leaf temperature of Dro1-NIL after termination of irrigation in the drought stress area. The values are relative to the leaf temperature of IRG4 and were determined by subtracting the mean leaf temperature of IR64 from that of Dro1-NIL. The leaf temperature of Dro1-NIL was found to be lower than that of IR64 at every measurement date. D shows difference in the stomatal conductance between IR64 and Dro1-NIL after termination of irrigation in the drought stress area. * indicates that the stomatal conductance of Dro1-NIL is significantly greater than that of IR64 at a level of 5%. E shows difference in the photosynthesis rate between IR64 and Dro1-NIL after termination of irrigation in the drought stress area. * indicates that the photosynthesis rate of Dro1-NIL is significantly greater than that of IR64 at a level of 5%.

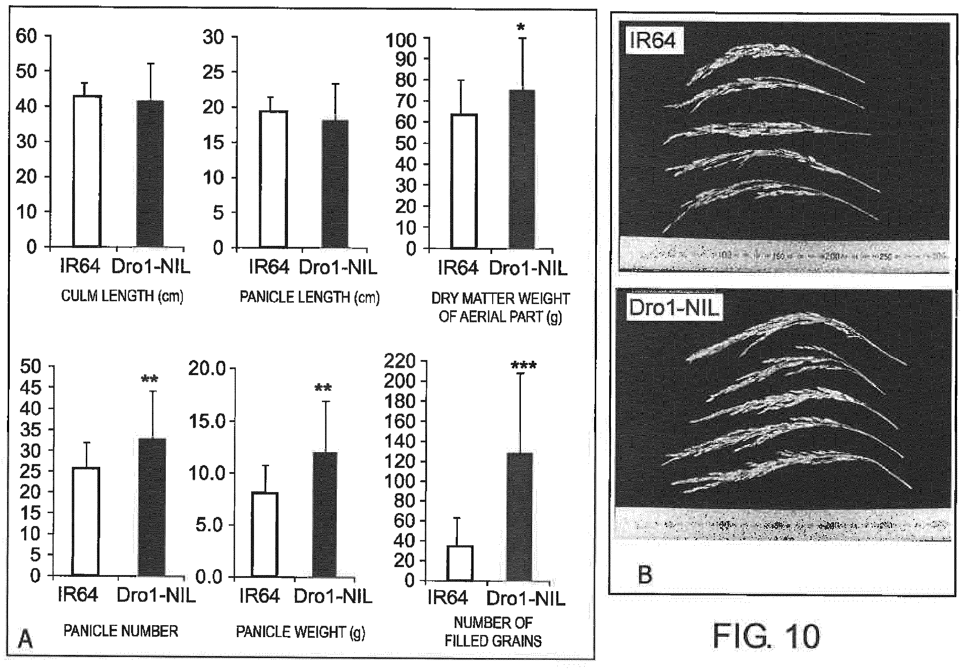

Fig. 10 presents diagrams and photographs showing results of yield determination for IR64 and Dro1-NIL cultivated under a drought stress condition. A shows various phenotypic differences between harvested IR64 and Dro1-NIL, which were grown in the drought stress area. Vertical bar indicates standard deviation. *, **, or *** indicates that Dro1-NIL is significantly larger than IR64 at a level of 5%, 1%, or 0.1%, respectively. B presents photographs showing the average panicles of IR64 and Dro1-NIL harvested in the drought stress area.

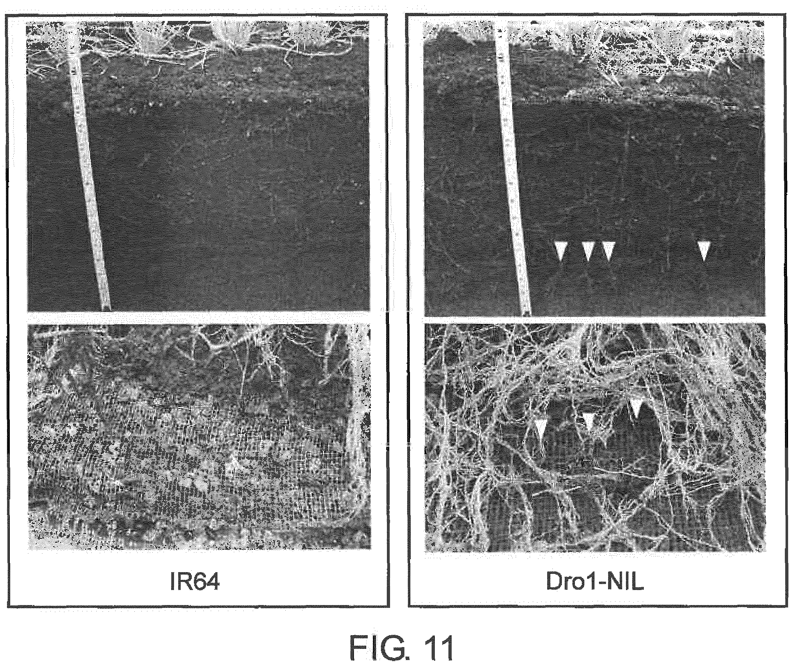

Fig. 11 presents photographs showing a lateral view of the dissected experimental field for testing drought resistance, which shows differences in the root system distribution between IR64 and Dro1-NIL in a drought stress area. The upper panel for each lineage shows a sectional view of the experimental field, and the lower panel shows an enlarged image obtained by washing off the additional topsoil to observe whether the roots penetrated the gravel stratum. The arrow heads indicate roots of Dro1-NIL that have penetrated the gravel stratum. It is shown that in IR64, there was no root that penetrated the gravel stratum and reached the deeper layer, but in Dro1-NIL, many roots penetrated the gravel stratum and reached the deeper layer.

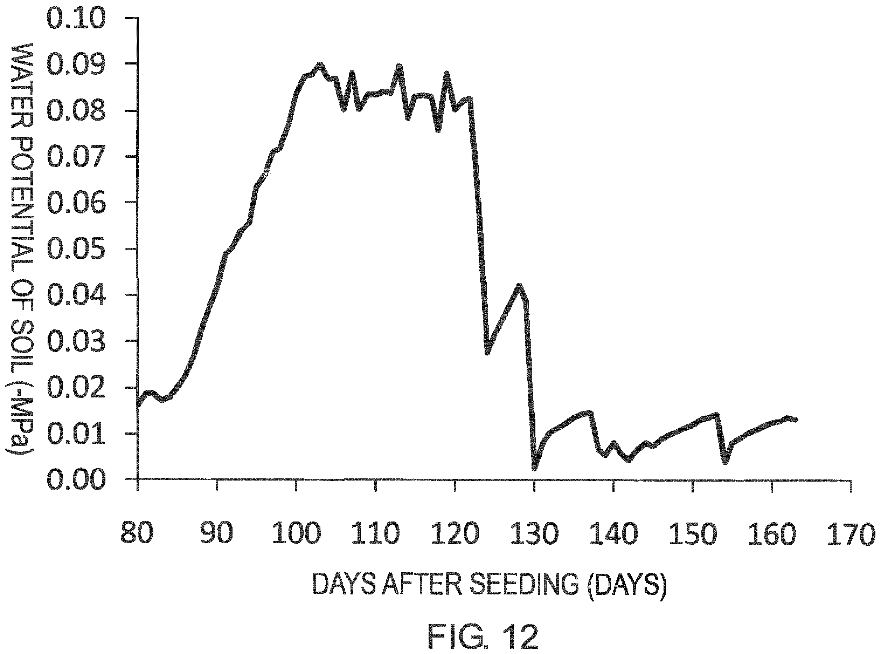

Fig. 12 presents a graph showing the time course of water potential in the field soil.

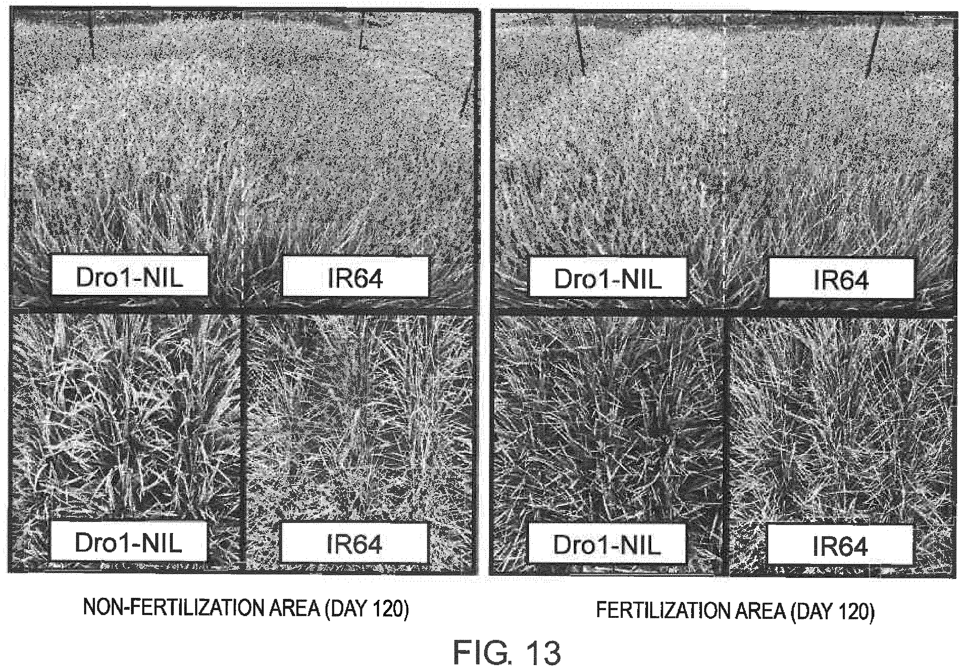

Fig. 13 presents photographs showing the appearance of plants 120 days after seeding in field areas with or without fertilization. The upper panels show the overall view including the respective areas. The lower panels are photographs taken from above as enlarged images of the respective areas. In IR64, leaf rolling is observed both in the areas with or without fertilization. Meanwhile, Dro1-NIL shows no leaf rolling.

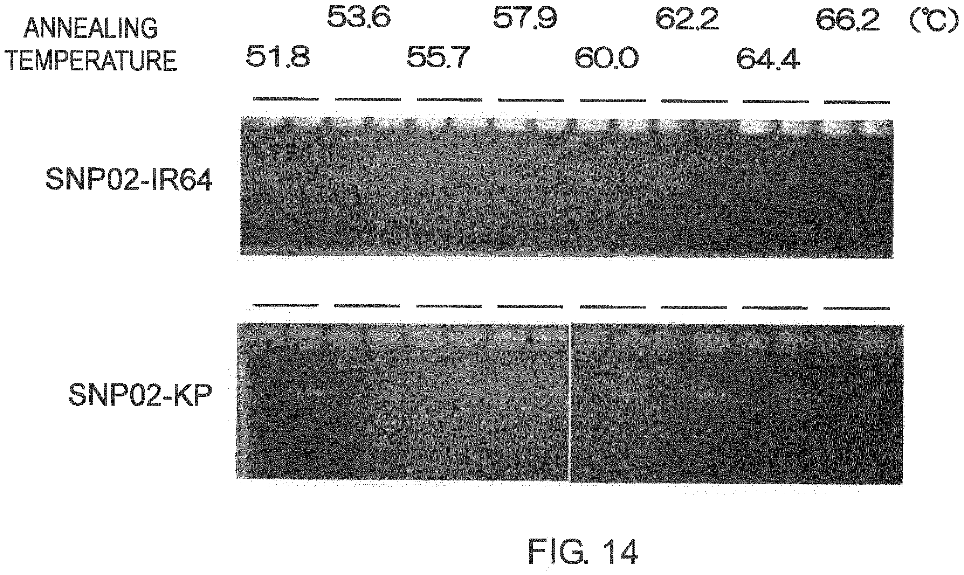

Fig. 14 presents a photograph showing PCR marker results for assessing the Dro1 gene deletion. At each annealing temperature, the left and right lanes show the results for IR64 and Kinandang Patong, respectively.

### [Mode for Carrying Out the Invention]

Provided is a DNA of any one of (a) to (e) below:
(a) a DNA comprising the nucleotide sequence of SEQ ID NO: 1;
(b) a DNA comprising the coding region of the nucleotide sequence of any one of SEQ ID NOs: 1, 2, 12, 14, 16, and 17;
(c) a DNA that encodes a protein comprising the amino acid sequence of any one of SEQ ID NOs: 3, 13, and 15;
(d) a DNA that hybridizes under a stringent condition to a DNA comprising the nucleotide sequence of any one of SEQ ID NOs: 1, 2, 12, 14, 16, and 17, and has an activity of conferring a deep rooting phenotype to a plant; or
(e) a DNA that encodes a protein comprising an amino acid sequence with one or more amino acid substitutions, deletions, additions, and/or insertions in the amino acid sequence of any one of SEQ ID NOs: 3, 13, and 15, and has an activity of conferring a deep rooting phenotype to a plant.

Hereinafter, occasionally, the above-described DNAs are also referred to as "DNA in the context of the present invention" or "Dro1 gene". Meanwhile, a protein encoded by a DNA in the context of the present invention is sometimes referred to as "protein in the context of the present invention" or "Dro1 protein".

Herein, "comprise" also refers to both "comprise" and "consist of".

Meanwhile, the DNA of (e) above can also be referred to as:
(e) a DNA encoding a protein having at least a mutation selected from one or more amino acid substitutions, deletions, additions, and insertions in the amino acid of any one of SEQ ID NOs: 3, 13, and 15, and having the activity of conferring a deep rooting trait to a plant.

Furthermore, a DNA in the context of the present invention can be referred to as "isolated DNA".

The genomic DNA nucleotide sequence for the Dro1 gene of rice cultivar Kinandang Patong is shown in SEQ ID NO: 1; the nucleotide sequence of the cDNA is shown in SEQ ID NO: 2; and the amino acid sequence of the protein encoded by the coding regions of the nucleotide sequences (Dro1 protein) is shown in SEQ ID NO: 3.

The protein-encoding region in the nucleotide sequence of SEQ ID NO: 1 consists of the nucleotides at positions 264 to 2,685.

Meanwhile, the protein-encoding region in the nucleotide sequence of SEQ ID NO: 2 consists of the nucleotides at positions 264 to 1019.

Furthermore, the nucleotide sequence of the Dro1 gene and its upstream including the promoter region of Kinandang Patong is shown in SEQ ID NO: 17. When compared to the sequence of the equivalent region in Nipponbare (SEQ ID NO: 18), the sequence in Kinandang Patong (SEQ ID NO: 17) contains a single-nucleotide substitution and a deletion of 30 nucleotides about 1.2 kb upstream of the Dro1 gene. Specifically, "T" of the "GATA" motif (the nucleotide at position 6 in the nucleotide sequence of SEQ ID NO: 18) in Nipponbare is substituted with "G" in Kinandang Patong (the nucleotide at position 6 in the nucleotide sequence of SEQ ID NO: 17). In addition, the 30 nucleotides at positions 7 to 36 in SEQ ID NO: 18 are deleted in the corresponding region of Kinandang Patong.

The nucleotide sequence of the coding sequence (CDS) in the sorghum-derived Dro1 gene is shown in SEQ ID NO: 12, while the amino acid sequence of the protein encoded by the coding region of the nucleotide sequence is shown in SEQ ID NO: 13.

The protein coding region of the nucleotide sequence of SEQ ID NO: 12 (CDS sequence) consists of the nucleotides at positions 1 to 789.

The nucleotide sequence of CDS in the corn-derived Dro1 gene is shown in SEQ ID NO: 14, while the amino acid sequence of the protein encoded by the coding region of the nucleotide sequence is shown in SEQ ID NO: 15.

The protein coding region of the nucleotide sequence of SEQ ID NO: 14 (CDS sequence) consists of the nucleotides at positions 1 to 768.

The Dro1 gene in connection with the present invention has the activity of conferring a deep rooting trait to a plant. Herein, the "deep rooting trait" refers to the nature that the root, an underground organ of plant, extends in soil with a deep angle relative to ground surface. Herein, "a deep angle" means that the angle of a root with respect to ground surface is at least 50°, preferably 60°, more preferably 70°, and yet more preferably 80° or more.

Whether a DNA has the activity of conferring a deep rooting trait to a plant can be confirmed by preparing a plant transformed with the Dro1 gene and determining the angle of the plant relative to ground surface. Plants can be assessed for the angle relative to ground surface, for example, by the basket method and an improved basket method described in the Examples herein as well as by the trench method, monolith method, and core sampling method; however, such methods are not limited thereto.

DNAs encoding a Dro1 protein in the context of the present invention include genomic DNAs, cDNAs, and chemically-synthesized DNAs. Such genomic DNAs and cDNAs can be prepared by conventional methods known to those skilled in the art. The genomic DNAs can be prepared, for example, as follows. Genomic DNA is extracted from plants of a rice cultivar (for example, Kinandang Patong), sorghum, or corn having a deep rooting to prepare a genomic library (vectors such as plasmids, phages, cosmids, BACs, and P1 artificial chromosomes (PACs) can be used, but are not limited thereto); and the library is amplified, and screened by colony or plaque hybridization using a probe prepared from a DNA encoding Dro1 protein (for example, DNA having the nucleotide sequence of any one of SEQ ID NOs: 1, 2, 12, 14, 16, and 17). Alternatively, such genomic DNAs can be prepared by PCR using primers prepared to be specific to a DNA encoding Dro1 protein (for example, DNA comprising the nucleotide sequence of any one of SEQ ID NOs: 1, 2, 12, 14, 16, and 17). Meanwhile, the cDNAs can be prepared, for example, as follows. cDNA is synthesized using mRNA extracted from plants of a rice cultivar (for example, Kinandang Patong) having a deep rooting, and inserted into a vector such as λZAP to construct a cDNA library; and the library is amplified and screened by colony or plaque hybridization. Alternatively, the cDNAs can be prepared by PCR in the same manner described above.

On the other hand, the chemically-synthesized DNAs can be prepared, for example, by using oligonucleotide synthesizers available on the market.

Alternatively, DNAs encoding a Dro1 protein in the context of the present invention can be prepared by extracting genomic DNA or mRNA from sorghum (for example, leaf blade and sheath) or corn (for example, leaf blade and sheath) having a deep rooting.

The present invention involves DNAs that encode a protein functionally equivalent to the Dro1 protein of any one of SEQ ID NOs: 3, 13, and 15. Herein, "functionally equivalent to Dro1 protein" means that the protein of interest has a function of conferring a deep rooting trait to a plant. The preferred DNAs are those derived from monocotyledons, more preferably those derived from the *Gramineae* family, *Liliaceae* family, *Bromeliaceae* family, *Palmae* family, *Araceae* family, *Zingiberaceae* family, and *Orchidaceae* family, still more preferably those derived from rice, wheat varieties (wheat, barley, rye, oat, and Job's tears (hatomugi)), corn, millet, foxtail millet, Japanese millet, sorghum, finger millet, pearl millet, teff, sugarcane, timothy, Kentucky bluegrass, orchardgrass, Italian ryegrass, perennial ryegrass, tall fescue, and Bahia grass, and particularly preferably those derived from rice, sorghum, and corn.

Such DNAs include, for example, mutants, derivatives, alleles, variants, and homo logs that encode a protein having the function to confer a deep rooting trait to a plant and comprising an amino acid sequence with one or more (for example, 2, 3, 4, 5, 10, 20, 30, 40, 50, or 100 residues) amino acid substitutions, deletions, additions, and/or insertions in the amino acid sequence of any one of SEQ ID NOs: 3, 13, and 15.

Examples of methods well-known in the art for preparing DNAs encoding a protein with altered amino acid sequence include site-directed mutagenesis methods (Kramer, W. and Fritz, H.-J. (1987) Oligonucleotide-directed construction of mutagenesis via gapped duplex DNA. Methods in Enzymology, 154: 350-367). In nature, mutations in nucleotide sequences may also lead to mutations in the amino acid sequences of proteins encoded thereby. As described above, DNAs encoding a protein having an amino acid sequence with one or more amino acid substitutions, deletions, or additions in the amino acid sequence encoding the naturally-occurring Dro1 protein are included in the DNAs in the context of the present invention, as long as they encode a protein having the function equivalent to the naturally-occurring Dro1 protein (amino acid sequence of any one of SEQ ID NOs: 3, 13, and 15). Such DNAs include, for example, DNAs comprising the nucleotide sequence of SEQ ID NO: 16. The DNAs comprising the nucleotide sequence of SEQ ID NO: 16 have a nucleotide sequence with a 1-bp addition at each of the 5' and 3' ends in a DNA comprising the nucleotide sequence of SEQ ID NO: 2. The DNAs also have a substitution of G for A at bp position 373 from the 5' end (at bp position 372 from the 5' end in SEQ ID NO: 2). Because of this nucleotide substitution, the amino acid sequence encoded by such a DNA comprising the nucleotide sequence of SEQ ID NO: 16 has a non-synonymous amino acid substitution of glutamic acid for lysine at position 37 in the amino acid sequence of SEQ ID NO: 3. Also provided are such proteins comprising an amino acid sequence with a substitution of glutamic acid for lysine at position 37 in the amino acid sequence of SEQ ID NO: 3. Also provided are DNAs encoding such a protein (for example, DNAs comprising the nucleotide sequence of SEQ ID NO: 16).

Even if the nucleotide sequence has a mutation, in some cases, the mutation may not result in any mutations in the amino acid sequence of the protein (mutation degeneracy). Such mutants (degenerate mutants) are also included in the DNAs in the context of the present invention.

Other methods well known to those skilled in the art for preparing a DNA encoding a protein functionally equivalent to the Dro1 protein of any one of SEQ ID NOs: 3, 13, and 15 include methods using hybridization techniques (Southern, E. M. (1975) Journal of Molecular Biology, 98, 503) or PCR techniques (Saiki, R. K. et al., (1985) Science, 230, 1350-1354; Saiki, R. K. et al., (1988) Science, 239, 487-491). Specifically, those skilled in the art can readily isolate DNAs with high homology to the Dro1 gene from rice or other plants using as a probe the nucleotide sequence of the Dro1 gene (the nucleotide sequence of any one of SEQ ID NOs: 1, 2, 12, 14, 16, and 17) or a portion thereof, or using as primers oligonucleotides that specifically hybridize to the Dro1 gene (the nucleotide sequence of any one of SEQ ID NOs: 1, 2, 12, 14, 16, and 17). The DNAs in the context of the present invention also comprise such DNAs encoding a protein that is functionally equivalent to the Dro1 protein, which can be isolated by using hybridization or PCR techniques.

To isolate such DNAs, a hybridization reaction is preferably carried out under stringent conditions. Those skilled in the art can appropriately select stringent hybridization conditions. For example, pre-hybridization is carried out in a hybridization solution containing 25% formamide or 50% formamide under more stringent conditions, and 4x SSC, 50 mM Hepes (pH 7.0), 10x Denhardt's solution, and 20 µg/ml denatured salmon sperm DNA at 42°C overnight; then labeled probes are added, and hybridization is carried out by incubation at 42°C overnight. Post-hybridization washing can be carried out with the following conditions for the wash solution and temperature: for example, "2x SSC, 0.1% SDS, 50°C", "2x SSC, 0.1% SDS, 42°C", "1x SSC, 0.1% SDS, 37°C", or so; "2x SSC, 0.1% SDS, 65°C", "0.5x SSC, 0.1% SDS, 42°C", or so for a more stringent condition; and "0.2x SSC, 0.1% SDS, 65°C" or so for an even more stringent condition. As the stringency of the hybridization increases, isolation of DNAs with high homology to the probe sequence is expected. However, the above-described combinations of SSC, SDS, and temperature conditions are mere examples, and those skilled in the art can achieve similar stringencies as those described above by appropriately combining the above or other elements (such as probe concentration, probe length, or hybridization reaction time) which determine the stringency of hybridization.

Thus, the protein encoded by such an isolated DNA is expected to have high homology to the amino acid sequence of Dro1 protein (SEQ ID NO: 3, 13, or 15) at the amino acid level. Furthermore, the DNA is expected to have high homology to the nucleotide sequence of a DNA encoding Dro1 protein (SEQ ID NO: 1, 2, 12, 14, 16, or 17) at the nucleotide sequence level. The high homology refers to a sequence identity of at least 50% or more, preferably 70% or more, more preferably 90% or more (for example, 91%, 92%, 93%, 94%, 95%, 96%, 97%, 98%, 99%, or more) over the entire amino acid or nucleotide sequence. Such amino acid sequence or nucleotide sequence identity can be determined using the BLAST algorithm by Karin and Altschul (Proc. Natl. Acad. Sci. USA 87: 2264-2268, 1990; Proc Natl Acad Sci USA 90: 5873, 1993). Programs called BLASTN and BLASTX have been developed based on the BLAST algorithm (Altschul SF, et al., J Mol Biol 215: 403, 1990). When nucleotide sequences are analyzed using BLASTN, parameters may be set at: score = 100 and wordlength = 12, for example. Alternatively, when amino acid sequences are analyzed using BLASTX, parameters may be set at: score = 50 and wordlength = 3, for example. When the BLAST and Gapped BLAST programs are used, it is possible to use default parameters for each program. Specific procedures are known for these analytical methods.

The DNAs in the context of the present invention can be used, for example, in preparing recombinant proteins and producing plant transformants having a deep rooting trait.

Recombinant proteins are typically prepared by inserting DNAs encoding proteins in the context of the present invention into appropriate expression vectors, introducing the vectors into appropriate cells, culturing the transformed cells, and purifying the expressed proteins. Recombinant proteins can be expressed as fusion proteins with other proteins to make purification easier, for example, as fusion proteins with maltose-binding protein using *Escherichia coli* as a host (New England Biolabs, USA, vector pMAL series), as fusion proteins with glutathione S-transferase (GST) (Amersham Pharmacia Biotech, vector pGEX series), or tagged with histidine (Novagen, pET series). The host cells are not particularly limited, so long as the cell is suitable for expressing the recombinant proteins. It is possible to use, for example, yeast, various plant or animal cells, insect cells or such in addition to the above-described *E. coli.* Vectors can be introduced into host cells by a variety of methods known to those skilled in the art. For example, introduction methods using calcium ions can be used for introduction into *E. coli* (Mandel, M. & Higa, A. (1970) Journal of Molecular Biology, 53, 158-162; Hanahan, D. (1983) Journal of Molecular Biology, 166, 557-580). Recombinant proteins expressed in the host cells can be purified and recovered from the host cells or a culture supernatant thereof by methods known to those skilled in the art. Recombinant proteins can be easily purified by affinity when expressed as fusion proteins with the above-described maltose-binding protein or such. Alternatively, plant transformants introduced with a DNA in the context of the present invention may be produced by the techniques described herein below. The proteins in the context of the present invention can be prepared from such plants. Accordingly, the plant transformants of the present invention include plants introduced with a DNA in the context of the present invention to prepare a protein in the context of the present invention as well as plants introduced with a DNA in the context of the present invention to confer a deep rooting trait to plants, which are described herein below.

The resulting recombinant protein can be used to prepare antibodies that bind to the protein. Polyclonal antibodies can be prepared, for example, as follows. Animals for immunization such as rabbits are immunized with a purified protein in the context of the present invention or a partial peptide thereof; and after a certain period, their blood is collected, and clotted blood is removed. Meanwhile, monoclonal antibodies can be prepared as follows. Myeloma cells are fused with antibody-producing cells from animals immunized with an above-described protein or peptide; single clone cells (hybridomas) producing the antibody of interest are isolated; and the antibody is obtained from the cells. The resulting antibody can be used to purify or detect proteins in the context of the present invention. The present invention envisages antibodies that bind to a protein in the context of the present invention. Such antibodies can be used to detect expression sites of the Dro1 protein in plants or to assess whether a plant species expresses the Dro1 protein. For example, the amino acid sequence from positions 227 to 251 of the Kinandang Patong-type Dro1 protein is a sequence characteristic of cultivars having a deep rooting trait. Thus, antibodies that specifically recognize the entire amino acid sequence or a portion thereof can be used to assess whether a plant species expresses the Kinandang Patong-type Dro1 protein (whether it has a deep rooting trait).

Also provided are vectors and transformed cells comprising the Dro1 gene.

With regard to the vectors in the context of the present invention, for example, when the host is *E. coli,* as long as the vector has an "ori" for amplification in *E. coli,* such that vectors are amplified and prepared in large quantities in *E. coli* (for example, JM109, DH5α, HB101, and XL1Blue) or such, and further has a selection gene for transformed *E. coli* (for example, a drug resistance gene that allows discrimination using a drug (ampicillin, tetracycline, kanamycin, chloramphenicol, or such)), the vectors are not limited. Such vectors include, for example, M13 vectors, pUC vectors, pBR322, pBluescript, and pCR-Script. In addition to the above vectors, for example, pGEM-T, pDIRECT, and pT7 can also be used for the subcloning and excision of cDNAs. When using vectors to produce the Dro1 protein, expression vectors are particularly useful. When an expression vector is expressed in *E. coli,* for example, it should have the above characteristics in order to be amplified in *E. coli.* Additionally, when *E*. *coli* such as JM109, DH5α, HB101, or XL1-Blue are used as the host, the vector must have a promoter that allows efficient expression in *E*. *coli,* for example, a lacZ promoter (Ward et al. Nature 341: 544-546, 1989; FASEB J. 6: 2422-2427, 1992), araB promoter (Better et al. Science 240:1041-1043, 1988), or T7 promoter. Other examples of the vectors include pGEX-5X-1 (Pharmacia), "QIAexpress system" (QIAGEN), pEGFP, and pET.

Furthermore, the vector may comprise a signal sequence for polypeptide secretion. When producing polypeptides into the periplasm of *E. coli,* the pelB signal sequence (Lei, S. P. et al. J. Bacteriol. 169: 4379 (1987)) may be used as a signal sequence for polypeptide secretion. For example, calcium chloride methods or electroporation methods may be used to introduce the vector into a host cell. Vectors for expressing in the plant body include pMH1, pMH2, pCAMBIA, and such.

In addition to *E. coli,* expression vectors derived from mammals (e.g., pcDNA3 (Invitrogen), pEGF-BOS (Nucleic Acids Res. 18(17): 5322 (1990)), pEF, and pCDM8), insect cells (e.g., "Bac-to-BAC baculovirus expression system" (GIBCO-BRL) and pBacPAK8), plants (e.g., pMH1 and pMH2), animal viruses (e.g., pHSV, pMV, and pAdexLcw), retroviruses (e.g., pZIPneo), yeasts (e.g., "Pichia Expression Kit" (Invitrogen), pNV11 and SP-Q01), and *Bacillus subtilis* (e.g., pPL608 and pKTH50) may also be used as vectors for producing the Dro1 protein.

For expression in animal cells such as CHO, COS, and NIH3T3 cells, the vector must have a promoter necessary for expression in such cells, for example, an SV40 promoter (Mulligan et al. Nature 277: 108 (1979)), MMLV-LTR promoter, EF1α promoter (Mizushima et al. Nucleic Acids Res. 18: 5322 (1990)), or CMV promoter. It is even more preferable that the vector comprises a gene for selecting transformants (for example, a drug-resistance gene enabling discrimination by a drug (such as neomycin and G418)). Examples of vectors with such characteristics include pMAM, pDR2, pBK-RSV, pBK-CMV, pOPRSV, and pOP13.

The transformed cells in the context of the present invention can be used, for example, as a production system for expressing or producing proteins in the context of the present invention. Such protein production systems include *in vitro* and *in vivo* systems.

When eukaryotic cells are used, for example, animal cells, plant cells, or fungal cells are used as host cells. Known animal cells include mammalian cells (for example, cells such as 3T3, myeloma cells, BHK (baby hamster kidney), HeLa, and Vero, in addition to CHO cells, COS cells, and NIH3T3 cells described above), amphibian cells (for example, *Xenopus laevis* oocytes (Valle, et al., Nature (1981) 291, 358-340)), and insect cells (for example, cells such as sf9, sf21, and Tn5). Among CHO cells, dhfr-CHO (Proc. Natl. Acad. Sci. USA (1980) 77, 4216-4220), which is DHFR gene-deficient CHO cells, and CHO K-1 (Proc. Natl. Acad. Sci. USA (1968) 60, 1275) can be preferably used in the present invention. CHO cells are particularly preferred for use in large-scale expression.

Plant cells include, for example, plant-derived cells described below as wells as *Nicotiana tabacum*-derived cells known as a protein production system. It is possible to culture calluses from cells.

Meanwhile, fungal cells include yeast cells, for example, cells of the genus *Saccharomyces,* for example, *Saccharomyces cerevisiae*; cells of filamentous fungi, for example, the genus *Aspergillus,* for example, *Aspergillus niger,* but are not limited thereto.

Also provided are the above-described cells introduced with a DNA or vector in the context of the present invention.

Furthermore, the present invention relates to plants transformed with the Dro1 gene, which have a deep rooting trait.

Whether a plant has a deep rooting trait can also be assessed by comparing it with a control. Herein, as long as the root of plant transformant extends in soil at a deeper angle with respect to ground surface when compared to a control, even if the angle difference is very small, the transformant is judged to "have a deep rooting trait". Whether a root of a plant transformant extends in soil with a deeper angle with respect to ground surface can be assessed by the methods described above.

Herein, "control" refers to a plant that is of the same type as a plant transformant of the present invention, but without artificial introduction of a DNA in the context of the present invention or which does not have any DNA in the context of the present invention. Herein, the control is not particularly limited, as long as it is a plant that is of the same type as a plant transformant of the present invention, but without artificial introduction of a DNA in the context of the present invention or which does not have any DNA in the context of the present invention. Accordingly, the "control" in the context of the present invention also comprises plants artificially introduced with DNAs other than the DNAs in the context of the present invention. Such plants include, but are not limited to, for example, plants of the same type as a plant transformant of the present invention, which are transformed with a DNA other than the DNAs in the context of the present invention, a DNA in the context of the present invention introduced with a mutation that causes loss-of-function in the DNA, a DNA in the context of the present invention converted into a type that suppresses the function, or a DNA fragment containing only a portion of a DNA in the context of the present invention which is insufficient for exerting the function of the DNA.

Plants transformed with a DNA in the context of the present invention are not particularly limited as long as they have a deep rooting trait, and may contain modifications in any other parts. Such modifications in any other parts include, for example, morphological changes of panicles, but are not limited thereto.

Plant transformant can be prepared using a DNA in the context of the present invention by the following procedure. The DNA or a vector inserted with the DNA is introduced into plant cells. Then, plants are regenerated from the resulting transformed plant cells.

In the present invention, preferred vectors are those capable of expressing inserted genes in plant cells, and include the vectors described above (for example, vectors such as pMH1, pMH2, and pCAMBIA vector); however, the vectors are not particularly limited. The vectors in the context of the present invention may comprise, for example, a promoter (for example, Cauliflower mosaic virus 35S promoter) for constitutive gene expression in plant cells. When such a promoter is used, a DNA is designed so that a DNA in the context of the present invention is operably linked downstream of the promoter. Then, a designed vector comprising the DNA is introduced into plant cells. Plant transformants expressing the DNA in the context of the present invention can be obtained by regenerating the resulting transformed plant cells. Thus, also provided are DNAs to which a DNA in the context of the present invention is operably linked downstream of a promoter. Herein, "operably linked" means that a promoter sequence is linked to a DNA in the context of the present invention so that the expression of the DNA is induced upon binding of transcriptional factors to the promoter sequence.

It is possible to use, in addition to the above, vectors with a promoter that is activated upon extrinsic stimulation in an inducible manner.

The plant species into which the aforementioned DNAs or vectors are introduced are not particularly limited and include, for example, monocotyledons. Monocotyledons include, but are not limited to, plants belonging to the *Gramineae* family, *Liliaceae* family, *Bromeliaceae* family, *Palmae* family, *Araceae* family, *Zingiberaceae* family, and *Orchidaceae* family. Plants belonging to the *Gramineae* family include, but are not limited to, rice, wheat varieties (wheat, barley, rye, oat, and Job's tears (hatomugi)), corn, millet, foxtail millet, Japanese millet, sorghum, finger millet, pearl millet, teff, sugarcane, timothy, Kentucky bluegrass, orchardgrass, Italian ryegrass, perennial ryegrass, tall fescue, and Bahia grass.

Plant cells into which the aforementioned DNAs or vectors are introduced are not particularly limited and may be in any form as long as they can be used to regenerate plants. For example, suspension-cultured cells, protoplasts, leaf sections, calli, and germinated seed can be used.

Introduction of the aforementioned DNAs or vectors into plant cells can be performed using methods known to one skilled in the art, such as polyethylene glycol methods, electroporation, Agrobacterium-mediated methods, and particle gun methods. In the Agrobacterium-mediated methods, for example, according to the method by Nagel *et al.* (Nagel, R. et al. FEMS Microbiol. Lett. 67, 1990, 325-328), a DNA can be introduced into plant cells by introducing into Agrobacteria an expression vector to which the DNA is inserted, and infecting plant cells with the Agrobacteria via direct infection or by the leaf disc method. The above-mentioned vector comprises an expression promoter so that, for example, the DNA in the context of the present invention is expressed in a plant after introduction into the plant. Generally, the DNA in the context of the present invention is located downstream of the promoter, and a terminator is located further downstream of such a DNA. The recombinant vector used for this purpose is suitably selected by one skilled in the art, depending on the type of plant or method of introduction.

The above-mentioned promoters include, for example, the CaMV35S derived from cauliflower mosaic virus and the ubiquitin promoter from corn (JP-A (Kokai) H02-79983).

Examples of the above-mentioned terminator can be a terminator derived from cauliflower mosaic virus and the terminator from the nopaline synthase gene; however, the promoter and terminator are not limited thereto, as long as they function in a plant.

Regeneration of a plant from a plant cell can be carried out according to the type of plant by methods known to those skilled in the art. Examples include the following methods, but are not limited thereto:
for rice, the method of Fujimura *et al.* (Fujimura. et al. Tissue Culture Lett. 2, 1995, 74);
for wheat, the method of Harris *et al.* (Harris, R. et al. Plant Cell Reports. 7, 1988, 337-340) and the method of Ozgen *et al.* (Ozgen, M. et al. Plant Cell Reports. 18, 1998, 331-335);
for barley, the method of Kihara and Funatsuki (Kihara, M. and Funatsuki, H. Breeding Sci. 44, 1994, 157-160) and the method of Lurs and Lorz (Lurs, R. and Lorz, H. Theor. Appl. Genet. 75, 1987, 16-25);
for corn, the method of Shillito *et al.* (Shillito, R.D., et al. Bio/Technology, 7, 1989, 581-587) and the method of Gordon-Kamm *et al.* (Gordon-Kamm, W.J. et al. Plant Cell. 2(7), 1990, 603-618);
for sorghum, the method of Wen *et al.* (Wen, F.S., et al. Euphytica. 52, 1991, 177-181) and the method of Hagio (Hagio, T. Breeding Sci. 44, 1994, 121-126);
for rye, the method of Castillo *et al.* (Castillo A. M., Vasil V., Vasil I. K. (1994) Nature Biotechnology 12: 1366-1371.);
for oat, the method of Cho *et al.* (Cho M. J., WEN J., LEMAUX P. G., (1999) Plant science 148: 9-17.);
for pearl millet, the method of O'Kennedy *et al.* (O'Kennedy M. M., Burger J. T., Botha F. C. (2004) Plant Cell Reports 22: 684-690.);
for Kentucky bluegrass, the method of Ha *et al.* (Ha C. D., Lemaux P. G., Cho M. J. (2001) In Vitro Cellular & Developmental Biology-Plant 37: 6-11.);
for orchardgrass, the method of CHO *et al.* (CHO M.J., CHOI H. W., LEMAUX P. G. (2001) Plant cell reports 20: 318-324.);
for Italian rye grass, the method of Ye *et al.* (Ye X., Wang Z. Y., Wu X., Potrykus I., Spangenberg G. (1997) Plant Cell Reports 16: 379-384.);
for perennial ryegrass, the method of Spangenberg *et al.* (Spangenberg G., Wang Z. Y., Wu X., Nagel J., Potrykus I. (1995) Plant science 108: 209-217.);
for tall fescue, the method of Wang *et al.* (Wang Z. Y., Takamizo T., Iglesias V. A., Osusky M., Nagel J., Potrykus I., Spangenberg G. (1992) Nature Biotechnology 10: 691-696.); and
for Bahia grass, the method of Smith *et al.* (Smith R. L., Grando M. F., Li Y. Y., Seib J. C., Shatters R. G (2002) Plant Cell Reports 20: 1017-1021.).

The plants into which the DNA in the context of the present invention is introduced may be explants, or the DNA may be introduced into the cultured cells prepared from these plants. "Plant cells" in the context of the present invention include, for example, plant cells of a leaf, root, stem, flower, scutellum in a seed, and immature embryo; calluses; suspension-cultured cells; and germinated seed, but are not limited thereto.

In order to efficiently select the cells transformed by introducing the DNA in the context of the present invention, the recombinant vector is introduced into the plant cells, preferably together with a suitable selection marker gene or a plasmid vector comprising a selection marker gene. The selection marker genes used for this purpose include, for example, the hygromycin phosphotransferase gene resistant to the antibiotic hygromycin, the neomycin phosphotransferase gene resistant to kanamycin or gentamycin, and the acetyltransferase gene resistant to the herbicide phosphinothricin.

The cells into which the recombinant vector has been introduced are placed on a known selection medium containing a suitable selection agent depending on the type of introduced selection marker gene, and then cultured. In this way, the transformed plant cultured cells can be obtained.

Next, plant bodies regenerated from the transformed cells are cultured in an acclimation medium. The acclimated, regenerated plants are then grown under usual culture conditions to obtain plants having deep rooting trait, from which seeds can be obtained once they mature and bear fruit. Specifically, the present invention provides methods for producing transformed plants, which comprise steps (a) and (b) below. The present invention also provides methods for conferring a deep rooting trait to plants, which comprise steps (a) and (b) below:
(a) introducing a plant cell with a DNA in the context of the present invention (Dro1 gene) or a vector carrying the DNA (Dro1 gene); and
(b) regenerating a plant from the plant cell introduced with the DNA or vector in step (a).

The above-described methods for producing transformed plants may additionally comprise the step of:
(c) selecting a plant to which the deep rooting trait is conferred.

The presence of the introduced foreign DNAs in the transformed plants that are regenerated and grown in this manner can be confirmed by the known PCR method or Southern hybridization method, or by analyzing the nucleotide sequences of the DNAs in plant bodies. In this case, extraction of the DNAs from the transformed plants can be carried out according to the known method by J. Sambrook et al. (Molecular Cloning, the 2nd edition, Cold Spring Harbor Laboratory Press, 1989). When analyzing the foreign genes which are present in the regenerated plant bodies and include the DNAs in the context of the present invention, using the PCR method, an amplification reaction is carried out using as a template the DNAs extracted from the regenerated plant bodies as mentioned above. An amplification reaction can also be performed in a reaction mixture containing as primers synthesized oligonucleotides which comprise nucleotide sequences suitably selected according to the nucleotide sequences of the DNAs in the context of the present invention. In the amplification reaction, denaturation, annealing, and extension reactions of DNAs can be repeated several tens of times to obtain amplified products of DNA fragments comprising the DNA sequences in the context of the present invention. By subjecting the reaction mixture comprising the amplified products, for example, to agarose electrophoresis, the various kinds of amplified DNA fragments are fractionated, thereby enabling confirmation of whether a certain DNA fragment corresponds to a DNA in the context of the present invention.

The present invention also relates to transformed plants, which are produced by introducing the Dro1 gene or a vector carrying the Dro1 gene into plant cells, and which have a deep rooting trait. The present invention also relates to methods for producing transformed plants, which comprise the step of introducing the Dro1 gene or a vector carrying the Dro1 gene into a plant cell.

Furthermore, in a preferred embodiment of the present invention, the transformed plants include those which are produced by steps (a) to (d) below, and which have a deep rooting trait. In another preferred embodiment of the present invention, the methods for producing transformed plants include those comprising the steps of:
(a) introducing the Dro1 gene or a vector carrying the Dro1 gene into a plant cell;
(b) determining the copy number of the artificially introduced Dro1 gene or vector carrying the Dro1 gene in the plant cell of step (a);
(c) selecting a transformed plant cell whose copy number of the artificially introduced Dro1 gene or vector carrying the Dro1 gene is one (that contains in a single copy of the gene or vector); and
(d) regenerating a plant from the transformed plant cell selected in step (c).

In the methods described above, the copy numbers of the artificially introduced Dro1 gene or vector carrying the Dro1 gene in the plants may be determined to select plants in which the copy number is 1 after a plant is regenerated from the transformed plant cell comprising the Dro1 gene. Thus, the present invention relates to transformed plants which are produced by steps (a) to (c) below, and which have a deep rooting trait. The present invention also relates to methods for producing transformed plants, which comprise the steps of:
(a) introducing the Dro1 gene or a vector carrying the Dro1 gene into a plant cell and regenerating a plant from the plant cell;
(b) determining the copy number of the artificially introduced Dro1 gene or vector carrying the Dro1 gene in the plant of step (a); and
(c) selecting a transformed plant whose copy number of the artificially introduced Dro1 gene or vector carrying the Dro1 gene is one.

Introduction of the Dro1 gene or a vector carrying the Dro1 gene into plant cells and plant regeneration from the transformed plant cells can be achieved by the methods described above. Meanwhile, the copy number of the Dro1 gene or vector carrying the Dro1 gene in transformed plant cells or transformed plants can be determined, for example, by Southern blot analysis, real-time PCR method, nucleotide sequence analysis, or the like. However, such methods are not limited to these examples.

Herein, "copy number" refers to the number of Dro1 genes or vectors carrying the gene introduced into plants by transformation. Specifically, herein, "copy number" does not include the number of endogenous Dro1 genes in the plants (endogenous genes).

As described in the Examples herein, the present inventors produced plants of T1 generation from transformed plants (T0 generation) in which the copy number of the artificially introduced Dro1 gene is 1, and checked the relation between the deep rooting ratio and the estimated copy number of the Dro1 gene in the T1 generation. The result demonstrated that in the T1 generation, the deep rooting ratio of the homozygous plants (copy number of the artificially introduced Dro1 gene: 2) was greater than that of the null type plants (copy number of the artificially introduced Dro1 gene: 0) and heterozygous plants (copy number of the artificially introduced Dro1 gene: 1). Thus, particularly preferred plants of the present invention include transformed plants of the T1 generation where the copy number of the artificially introduced Dro1 gene is 2 (homozygotes), which are produced from transformed plants of the T0 generation where the copy number of the artificially introduced Dro1 gene is 1.

Specifically, in a particularly preferred embodiment, the present invention include transformed plants produced by methods comprising the steps described below, in addition to the above-described steps (a) to (d) or (a) to (c). In another particularly preferred embodiment, the present invention includes methods for producing transformed plants, which comprise, in addition to the above-described steps (a) to (d) or (a) to (c), the steps of:
- producing a plant by crossing the plant transformants obtained in step (d) or (c); and
- selecting a plant homozygous for the Dro1 gene from plants obtained in the above step.

Methods for producing plants of the T1 generation from those of the T0 generation by crossing are known to those skilled in the art. The copy number of the Dro1 gene (null type, heterozygote, and homozygote) in a plant produced by crossing can be determined by methods known to those skilled in the art, such as Southern blot analysis and real-time PCR method.

Once a transformed plant having a DNA in the context of the present invention introduced into its chromosome is generated, its progeny can be obtained by sexual or asexual reproduction from the plant. Alternatively, the plant can be produced on a large scale from cells, organs, or propagation materials (for example, seeds, fruits, cut panicles, tubers, tuberous roots, stocks, calluses, and protoplasts) isolated from the plants, their progenies, or clones. The present invention or description, respectively, includes plant cells artificially introduced with a DNA in the context of the present invention; plants comprising the cells; organs (for example, flower, leaf, root, stem, etc.) of the plants; progenies and clones of the plants; and propagation materials of the plants and their progenies and clones. Such plant cells, plants comprising the cells, organs of the plants, progenies and clones of the plants, and propagation materials of the plants and their progenies and clones can be used to confer a deep rooting trait to plants.

Meanwhile, transformed plants of the present invention include, for example, monocotyledons. Monocotyledons include, but are not limited to, plants belonging to the *Gramineae* family, *Liliaceae* family, *Bromeliaceae* family, *Palmae* family, *Araceae* family, *Zingiberaceae* family, and *Orchidaceae* family. Plants belonging to the *Gramineae* family include, but are not limited to, rice and wheat varieties (wheat, barley, rye, oat, and Job's tears (hatomugi)), corn, millet, foxtail millet, Japanese millet, sorghum, finger millet, pearl millet, teff, sugarcane, timothy, Kentucky bluegrass, orchardgrass, Italian ryegrass, perennial ryegrass, tall fescue, and Bahia grass.

Furthermore, the present invention envisages processed foods obtained from at least any one of: cells, propagation materials, and organs in the context of the present invention. Herein, processed food refers to a product in an edible form for humans, which is produced by artificially processing plants such as rice, wheat varieties (wheat, barley, rye, oat, and Job's tears (hatomugi)), corn, millet, foxtail millet, Japanese millet, sorghum, finger millet, pearl millet, teff, sugarcane, and timothy, or portions thereof (cells, propagation materials, organs, etc.). In the present invention, processing includes treatments such as boiling, simmering, stir-frying, steaming, frying, and powdering, but is not limited thereto. "Powdering" includes threshing and rice polishing. The processed foods in connection with the present invention include those resulting from at least one of the treatments described above. An example of preferred processed foods in connection with the present invention is: processed foods resulting from threshing and polishing of rice seeds, followed by heating. Specifically, processed foods in connection with the present invention include, but are not limited to, cooked rice products obtained by rice boiling (including frozen cooked rice and sterilized cooked rice), rice powder, rice cake, rice noodle, cubic rice crackers, Japanese rice cracker, cookie, miso (fermented soybean paste), soy sauce, tofu (fermented bean curd), bread, soba (buckwheat noodle), wheat noodle, pasta, noodle such as Chinese noodle (raw noodle, rehydratable noodle, boiled noodle, etc.), cereal, and cornflakes.

The configuration of processed foods in connection with the present invention as a commercial product is not particularly limited. Examples include the configuration where the product is sold and distributed at an ambient or low temperature and at the time of eating/drinking heated to room temperature or high temperature using a heat cooker such as microwave oven. Specifically, such configuration as a commercial product include, but are not limited to, boxed lunches, rice balls, and cooked noodles, which are sold at convenience stores, supermarkets, delicatessens, and such.

Processed foods in connection with the present invention may be in a container-packaged form. For example, the foods can be in packaged in a molded plastic container, or packaged in retort pouch or the like and sterilized after sealing.

"Processed food obtained from at least one of cells, propagation materials, or organs" in the context of the present invention can also be referred to as "processed food produced from at least one of cells, propagation materials, or organs", "processed food comprising at least one of cells, propagation materials, or organs", or "processed food obtained by processing at least one of cells, propagation materials, or organs".

Furthermore, the present invention provides methods for assessing whether a plant has a deep rooting trait, which comprise steps (a) to (c) described below, wherein a test plant is judged to have a deep rooting trait or to potentially have a deep rooting trait when a molecular weight or nucleotide sequence is identical:
(a) preparing a DNA sample from the test plant;
(b) amplifying from the DNA sample an region comprising the whole or a portion of a DNA in the context of the present invention (Dro1 gene); and
(c) comparing the molecular weight or nucleotide sequence of the amplified DNA fragment with that of the DNA in the context of the present invention (Dro1 gene).

Such a preferred portion of the Dro1 gene include exon 4 of the Dro1 gene, more preferably regions that comprise a sequence comprising the nucleotide at bp position 116 from the 5' end of exon 4 of the Dro1 gene (the nucleotide at position 943 in the nucleotide sequence of SEQ ID NO: 2) (for example, regions consisting of at least 100, 50, 40, 30, 20, 10, 9, 8, 7, 6, 5, 4, 3, 2, or 1 nucleotide, which comprise the nucleotide at position 943 in the nucleotide sequence of SEQ ID NO: 2), but is not limited thereto. The present inventors revealed that in Kinandang Patong the nucleotide at bp position 116 from the 5' end of exon 4 of the Dro1 gene (the nucleotide at position 943 in the nucleotide sequence of SEQ ID NO: 2) is adenine while the nucleotide is deleted in IR64. Thus, whether a test plant has a deep rooting trait can be assessed by examining the presence or absence of this nucleotide. Specifically, the present invention provides methods for assessing whether a plant has a deep rooting trait, which comprise the step of detecting the presence or absence of the nucleotide at position 943 in the nucleotide sequence of SEQ ID NO: 2, wherein a test plant is judged not to have a deep rooting trait when the nucleotide deletion is detected. The single nucleotide deletion can be detected by comparing the nucleotide sequence or molecular weight of a region comprising the whole or a portion of the Dro1 gene.

Furthermore, the present invention relates to methods for assessing whether a plant has a deep rooting trait, which comprise the step of performing PCR with primers comprising the nucleotide sequences of SEQ ID NOs: 8 and 9, using as a template a genomic DNA prepared from a test plant. In these methods, a test plant is judged to have a deep rooting trait when the amplified product is obtained.

Furthermore, the present invention relates to methods for assessing whether a plant has a deep rooting trait, which comprise the step of performing PCR with primers comprising the nucleotide sequences of SEQ ID NOs: 10 and 11, using as a template a genomic DNA prepared from a test plant. In these methods, a test plant is judged not to have a deep rooting trait when the amplified product is obtained.

Furthermore, provided are primers for use in assessing whether a plant has a deep rooting trait. Such primers include, but are not limited to, DNAs comprising the nucleotide sequences of any one of SEQ ID NOs: 8 to 11.

Herein, "assessing whether a plant has a deep rooting trait" not only means assessing whether a cultivar that has been cultivated so far has a deep rooting trait but also means assessing whether a cultivar newly developed by crossing or using genetic engineering techniques has a deep rooting trait.

In the methods of the present invention for assessing whether a plant has a deep rooting trait, plants are assessed by testing whether they have a DNA encoding a functional Kinandang Patong-type Dro1 protein. Whether a plant has a DNA encoding a functional (Kinandang Patong-type) Dro1 protein can be assessed by examining genomic DNAs in terms of the difference in the molecular weight or nucleotide sequence of the region corresponding to Dro1.

In the assessment methods of the present invention, first, a DNA sample is prepared (extracted), and then a DNA region corresponding to the Dro1 gene is amplified from the DNA sample. Next, the molecular weight of the DNA fragment amplified from a DNA region for the Dro1 gene in a cultivar having a deep rooting trait is compared to that of the DNA fragment amplified from a DNA sample of a test plant. The test plant is judged to have a deep rooting trait when the molecular weights are the same. Alternatively, the nucleotide sequence of the DNA fragment amplified from a DNA region for the Dro1 gene in a cultivar having a deep rooting trait is compared to that of the DNA fragment amplified from a DNA sample of a test plant. The test plant is judged to have a deep rooting trait when the nucleotide sequences are identical.

DNA samples can be prepared (extracted) by methods known to those skilled in the art. Such preferred preparation methods include, for example, methods for extracting DNA by a CTAB method.

DNA samples to be assessed by the assessment methods of the present invention are not particularly limited. In general, genomic DNAs extracted from test plants are used as DNA samples. Furthermore, sources of genomic DNAs to be collected are not particularly limited, and they may be extracted from any plant tissues, for example, panicles, leaves, roots, stems, seeds, endosperm, bran, or germs. However, the sources are not limited to these examples.

In the assessment methods of the present invention, a DNA region of the Dro1 gene in connection with the present invention is then amplified by PCR or such. The "DNA region of the Dro1 gene" in the context of the present invention refers to a portion corresponding to the genomic DNA region for the Dro1 gene (for example, the DNA region of SEQ ID NO: 1). The region to be amplified may be the whole genomic DNA or a portion of the genomic DNA (for example, an ORF region encoding the protein or a portion thereof). Those skilled in the art can carry out PCR by appropriately selecting reaction conditions and such. The amplified DNA products can be labeled using primers labeled with isotopes such as ³²P, fluorescent dyes, biotin, or such when PCR is carried out. Alternatively, the amplified DNA fragments can be labeled by adding nucleotide substrates labeled with isotopes such as ³²P, fluorescent dyes, biotin, or such to PCR mixtures, and carrying out PCR. Furthermore, the amplified DNA fragments can also be labeled after PCR by attaching nucleotide substrates labeled with isotopes such as ³²P, fluorescent dyes, biotin, or such, using a Klenow enzyme or the like.

The labeled DNA fragments obtained in this way are denatured by heating or such, and electrophoresed in a polyacrylamide gel containing a denaturant such as urea or SDS. SDS-PAGE, which uses SDS as a denaturant, is an advantageous fractionation technique in the present invention. SDS-PAGE can be carried out according to the method of Laemmli (Laemmli (1970) Nature 227, 680-685). After electrophoresis, the mobility of the DNA fragments are detected and analyzed by autoradiography using X-ray films, fluorescence-detecting scanners, or such. When labeled DNAs are not used, the DNA fragments can be detected by staining the gel after electrophoresis with ethidium bromide, silver staining, or such. For example, DNA fragments are amplified from a cultivar having a deep rooting trait (for example, Kinandang Patong) and a test plant using primers comprising the nucleotide sequences of SEQ ID NOs: 8 and 9. Whether the test plant has a deep rooting trait can be assessed by comparing their molecular weights. The test plant is judged to have a deep rooting trait when the molecular weights are identical.

Alternatively, whether a plant has a deep rooting trait can be assessed by directly determining the nucleotide sequence of the DNA region of a test plant corresponding to the DNA in the context of the present invention and comparing the sequence with that of a cultivar having a deep rooting trait. The test plant is judged to have a deep rooting trait when the nucleotide sequences are identical.

Herein, "identical" means that for both alleles, the molecular weight of the gene or its nucleotide sequence or amino acid sequence is identical to that in a plant having a deep rooting trait. Accordingly, "identical" does not include the case where the molecular weight, nucleotide sequence, or amino acid sequence for one of the alleles is the same as that of a plant having a deep rooting trait but the other is different from that of the plant having a deep rooting trait.

The above-mentioned electrophoresis analysis may be conducted according to a conventional method. For example, electrophoresis is carried out by applying voltage in an agarose or polyacrylamide gel, and the separated DNA pattern is analyzed.

Meanwhile, nucleotide sequences can be determined, for example, using DNA sequencers available on the market.

Furthermore, plants to be identified to have a deep rooting trait can be selected at an early stage by using the assessment methods of the present invention.

Specifically, the present invention provides methods for selecting a plant having a deep rooting trait, which comprises the steps of (a) and (b) described below:
(a) producing a cultivar by crossing an arbitrary plant with a plant having a deep rooting trait; and
(b) assessing whether a plant produced in step (a) has a deep rooting trait by a method described herein for assessing whether a test plant has a deep rooting trait.

The selection methods of the present invention may additionally comprise the step of:
(c) selecting a plant that is judged to have a deep rooting trait in step (b).

A plant having a deep rooting trait can be crossed with an arbitrary plant by methods known to those skilled in the art.

Plants judged to have a deep rooting trait can be selected at an early stage by using the selection methods of the present invention. The present invention also provides such methods for selecting a plant judged to have a deep rooting trait at an early stage. Herein, "early stage" refers to, for example, the state before heading, preferably the state immediately after germination. By using the selection methods of the present invention, breeding of plant varieties having a deep rooting trait can be achieved in a shorter period of time than ever before.

Plants to be used in the assessment or selection methods of the present invention include, for example, monocotyledons, but are not limited thereto. Monocotyledons include, but are not limited to, plants belonging to the *Gramineae* family, *Liliaceae* family, *Bromeliaceae* family, *Palmae* family, *Araceae* family, *Zingiberaceae* family, and *Orchidaceae* family. Plants belonging to the *Gramineae* family include, but are not limited to, rice, wheat varieties (wheat, barley, rye, oat, and Job's tears (hatomugi)), corn, millet, foxtail millet, Japanese millet, sorghum, finger millet, pearl millet, teff, sugarcane, timothy, Kentucky bluegrass, orchardgrass, Italian ryegrass, perennial ryegrass, tall fescue, and Bahia grass.

Also provided are DNAs (oligonucleotides) comprising at least 15 (for example, 16, 17, 18, 19, 20, 21, 22, 23, 24, or 25) consecutive nucleotides complementary to the nucleotide sequence of the Dro1 gene in connection with the present invention or a complementary sequence thereof. Herein, "complementary sequence" refers to the sequence of an opposite strand with respect to the sequence of one strand of a double-stranded DNA consisting of base pairs [A:T] and [G:C]. Furthermore, "complementary" means not only a nucleotide sequence completely complementary to a continuous nucleotide sequence with at least 15 nucleotides but also an identity of at least 70%, preferably at least 80%, more preferably 90%, and still more preferably 95% or more (95%, 96%, 97%, 98%, or 99%) at the nucleotide sequence level. Such DNAs can be used as a probe for detecting or isolating a DNA in the context of the present invention, or as a primer for amplifying the DNA.

Such primers include, but are not limited to, the primer sets described below:
a primer set consisting of primers comprising the nucleotide sequences of SEQ ID NOs: 4 and 5, which are used to amplify Drol-INDEL09, an InDel marker for polymorphism between IR64 and Kinandang Patong;
a primer set consisting of primers comprising the nucleotide sequence of SEQ ID NOs: 6 and 7, which are used to amplify Dro1-CAPS05, a CAPS marker for polymorphism between IR64 and Kinandang Patong;
a primer set consisting of primers comprising the nucleotide sequences of SEQ ID NOs: 8 and 9, which are used to amplify SNP02-KP, a Kinandang Patong genomic DNA-specific marker; and a primer set consisting of primers comprising the nucleotide sequences of SEQ ID NOs: 10 and 11, which are used to amplify SNP02-IR64, an IR64-derived genomic DNA-specific marker.

Furthermore, the present invention envisages 20 to 100 consecutive nucleotides from the Dro1 gene, which comprise the whole or a portion of a DNA fragment amplified with an above-described primer set using as a template a genomic DNA derived from a plant (for example, rice plant). Such DNAs can be used to assess whether a test plant has the deep or shallow rooting.

When oligonucleotides in the context of the present invention are used as probes, they are preferably used after appropriate labelling. Labeling methods include, for example, those in which the 5' end of an oligonucleotide is phosphorylated with ³²P using a T4 polynucleotide kinase, and methods in which substrate nucleotides labeled with isotopes such as ³²P, fluorescent dyes, biotin, or the like are incorporated into the oligonucleotide by a DNA polymerase such as Klenow enzyme, using as primers random hexamer oligonucleotides or such (random priming methods and the like).

The oligonucleotides in the context of the present invention can be produced, for example, with a commercially available oligonucleotide synthesizer. The probes can also be produced as double-stranded DNA fragments obtained by restriction enzyme treatment or the like.

Furthermore, the present invention envisages pharmaceutical agents that confer a deep rooting trait to plants comprising the Dro1 gene or a vector carrying the gene in an expressible manner. The type of DNAs used in the pharmaceutical agents envisaged in the present invention is not particularly limited, and the DNAs may be cDNAs or genomic DNAs. In addition, it is possible to use not only DNAs encoding a rice plant-derived Dro1 protein but also DNAs encoding a protein structurally similar to the protein (for example, mutants, derivatives, alleles, variants, and homologs), as long as they can confer a deep rooting trait to a plant when introduced into the plant.

The DNAs included in the pharmaceutical agents envisaged in the present invention may be inserted into vectors. Vectors are not particularly limited, as long as they can allow introduced genes to be expressed in plant cells. For example, it is possible to use vectors containing promoters for homeostatic gene expressions in plant cells (e.g., the promoter of the potato SK2 chitinase gene, the cauliflower mosaic virus 35S promoter, etc.), or vectors containing promoters that are inducibly activated by external stimulation.

The pharmaceutical agents envisaged in the present invention may be DNAs described above or vectors inserted with a DNA described above, and they may be mixed with other ingredients for introduction into plant cells. For example, the DNAs described above, vectors inserted with an above-described DNA, Agrobacteria introduced with an above-described DNA, and biochemical reagents and solutions comprising them are also included in the pharmaceutical agents envisaged in the present invention.

Furthermore, the present invention relates to plants that are transformed with a DNA in the context of the present invention, and which are resistant to drought.

The present invention or description, respectively, also relates to cells, propagation materials, and organs isolated from the plants described above.

In addition, the present invention envisages processed foods obtained from at least one of cells, propagation materials, and organs described above.

Moreover, the present invention relates to methods for producing transformed plants resistant to drought, which comprise the steps of introducing a DNA or vector in the context of the present invention into plant cells and regenerating plants from the plant cells.

The present invention also relates to methods for assessing whether a plant is resistant to drought, which comprise steps (a) to (c) described below, where a test plant is judged to be resistant to drought when a molecular weight or nucleotide sequence is identical.
(a) preparing a DNA sample from a test plant;
(b) amplifying a region comprising a DNA in the context of the present invention from the DNA sample; and
(c) comparing the molecular weight or nucleotide sequence of the DNA in the context of the present invention with that of the amplified DNA fragment.

The present invention also relates to methods for assessing whether a plant is resistant to drought, which comprise the step of carrying out PCR with primers comprising the nucleotide sequences of SEQ ID NOs: 8 and 9 using as a template a genomic DNA prepared from a test plant, wherein the test plant is judged to be resistant to drought when PCR yields an amplification product.

Furthermore, the present invention relates to methods for assessing whether a plant is resistant to drought, which comprise the step of carrying out PCR with primers comprising the nucleotide sequences of SEQ ID NO: 10 and 11 using as a template a genomic DNA prepared from a test plant, wherein the test plant is judged not to be resistant to drought when PCR yields an amplification product.

In addition, the present invention relates to methods for selecting drought-resistant plants, which comprise the steps of:
(a) producing a cultivar by crossing an arbitrary plant with a drought-resistant plant; and
(b) assessing whether a plant produced in step (a) is resistant to drought by the above-described method for assessing whether a plant is resistant to drought.

The production of transformed plants that are resistant to drought, assessment of plants for drought resistance, and selection of drought-resistant plants can be achieved according to the description herein.

Whether a plant is resistant to drought can be assessed by measuring the leaf temperature.

Herein, as long as the leaf temperature of a plant transformant is decreased under an artificial drought stress condition (under a dry environment) as compared to a control, even if the temperature drop is very small, the plant is judged to "be resistant to drought".

Herein, leaf temperature refers to the leaf surface temperature of a plant. Plants absorb carbon dioxide through stomata for photosynthesis. At the same time, the intracellular water evaporates through stomata into the atmosphere. This results in a loss of water. The phenomenon is called transpiration. With regard to these connections, Takai *et al.,* have reported that the leaf temperature has a negative correlation to the photosynthesis rate and degree of stoma dilation (stomatal conductance) (Takai et al., Field Crops Research doi:10.1016/j.fcr.2009.10.019. 2009). The more stomata open, the more active the transpiration becomes. Transpiration takes heat away from the leaf surface, resulting in a reduction of the leaf temperature. When a plant is actively photosynthesizing, it opens stomata, and transpiration becomes active. This results in a temperature decrease at the leaf surface. Meanwhile, under drought stress, the plant suppresses transpiration by closing stomata to maintain the cellular water potential. This impairs photosynthesis and raises the leaf surface temperature. Drought-resistant plants, which can open the stomata and photosynthesize even under drought conditions, have a lower leaf temperature as compared to drought-sensitive plants. Hirayama *et al.* have reported that selection of drought-resistant rice plant lines using the leaf temperature as an indicator is effective in developing drought-resistant rice cultivars (Hirayama et al., Breeding Science 56: 47-54. 2006). As described in the Examples herein, whether a plant has a low leaf temperature can be assessed simply by testing it under drought stress using a device such as infrared thermography. Meanwhile, the photosynthesis and degree of stoma dilation can be assessed by simple tests under drought stress using a device such as the photosynthesis/transpiration measurement system, as described in the Examples herein.

Furthermore, it is known that in general plants exhibit leaf curling when exposed to drought stress. In particular, rice plant leaves are known to roll into a needle-like shape. However, the transformed plants of the present invention hardly show leaf curling even under drought stress.

Specifically, the present invention relates to plants that are transformed with a DNA in the context of the present invention, and which are resistant to leaf curling under drought stress.

The present invention is also concerned with cells, propagation materials, and organs isolated from the plants described above.

The present invention is also concerned with processed foods obtained from at least one of the cells, propagation materials, and organs described above.

Furthermore, the present invention relates to methods for producing transformed plants resistant to leaf curling under drought stress, which comprise the steps of introducing a DNA or vector in the context of the present invention into plant cells and regenerating plants from the plant cells.

The present invention also relates to methods for assessing whether a plant is resistant to leaf curling under drought stress, which comprise steps (a) to (c) described below, where a test plant is judged to be resistant to leaf curling under drought stress when a molecular weight or nucleotide sequence is identical:
(a) preparing a DNA sample from a test plant;
(b) amplifying a region comprising a DNA in the context of the present invention from the DNA sample; and
(c) comparing the molecular weight or nucleotide sequence of the DNA in the context of the present invention with that of the amplified DNA fragment.

The present invention also relates to methods for assessing whether a plant is resistant to leaf curling under drought stress, which comprise the step of carrying out PCR with primers comprising the nucleotide sequences of SEQ ID NOs: 8 and 9 using as a template a genomic DNA prepared from a test plant, wherein the test plant is judged to be resistant to leaf curling under drought stress when PCR yields an amplification product.

Furthermore, the present invention relates to methods for assessing whether a plant is resistant to leaf curling under drought stress, which comprise the step of carrying out PCR with primers comprising the nucleotide sequences of SEQ ID NOs: 10 and 11 using as a template a genomic DNA prepared from a test plant, wherein the test plant is judged not to be resistant to leaf curling under drought stress when PCR yields an amplification product.

In addition, the present invention relates to methods for selecting plants that are resistant to leaf curling under drought stress, which comprise the steps of:
(a) producing a cultivar by crossing an arbitrary plant with a plant that is resistant to leaf curling under drought stress; and
(b) assessing whether a plant produced in step (a) is resistant to leaf curling under drought stress by the above-described method for assessing whether a plant is resistant to leaf curling under drought stress.

The production of transformed plants that are resistant to leaf curling under drought stress, assessment of whether a plant is resistant to leaf curling under drought stress, and selection of plants that are resistant to leaf curling under drought stress can be achieved according the description herein.

Herein, leaf curling resistance refers to resistance to the leaf curling caused by drought stress. When rice plant leaves become dehydrated, the turgor pressure of epicuticular motor cells is reduced inside the leaves and as a result they curl so that the epicuticular side becomes concave. Whether a plant is resistant to leaf curling can be assessed simply by testing whether its leaves curl under drought stress.

Herein, as long as the degree of leaf curling in a plant transformant is smaller when compared to a control, even if the difference in the degree is very slight, the plant is judged to "be resistant to leaf curling".

Furthermore, under drought stress conditions, crop plants are in general often severely infertile, resulting in a decrease in the number of ripened grains and ripened grain weight. This leads to a reduction in the ultimate crop yield. However, even under drought stress, the transformed plants of the present invention produce a greater number of ripened grains or heavier ripened grains (reduction in the number of ripened grains or ripened grain weight has been suppressed) as compared to plants without having a DNA in the context of the present invention (control).

Specifically, the present invention relates to plants transformed with a DNA in the context of the present invention, which produce a greater number of ripened grains or heavier ripened grains as compared to a control under drought stress. Such a plant can also be referred to as a plant that has been improved to reduce a loss in the number of ripened grains or ripened grain weight under drought stress. Alternatively, the plant can be referred to as a plant that has been improved to have an increased number of ripened grains or increased ripened grain weight under drought stress as compared to a control.

Furthermore, the present invention is concerned with cells, propagation materials, and organs isolated from the plants described above.

The preset invention also relates to processed foods produced from at least one of the cells, propagation materials, and organs described above.

Moreover, the present invention relates to methods for producing a transformed plant that produces a greater number of ripened grains or heavier ripened grains under drought stress as compared to a control, which comprise the steps of introducing a DNA or vector in the context of the present invention into plant cells and regenerating plants from the plant cells.

In addition, the present invention relates to methods for assessing whether a plant produces a greater number of ripened grains or heavier ripened grains under drought stress as compared to a control, which comprise steps (a) to (c) below, wherein a test plant is judged to produce a greater number of ripened grains or heavier ripened grains under drought stress as compared to a control when a molecular weight or nucleotide sequence is identical:
(a) preparing a DNA sample from a test plant;
(b) amplifying a region comprising a DNA in the context of the present invention from the DNA sample; and
(c) comparing the molecular weight or nucleotide sequence of the DNA in the context of the present invention with that of the amplified DNA fragment.

The present invention also relates to methods for assessing whether a plant produces a greater number of ripened grains or heavier ripened grains under drought stress as compared to a control, which comprise the step of carrying out PCR with primers comprising the nucleotide sequences of SEQ ID NOs: 8 and 9 using as a template a genomic DNA prepared from a test plant, wherein the test plant is judged to produce a greater number of ripened grains or heavier ripened grains under drought stress as compared to a control when PCR yields an amplification product.

Furthermore, the present invention relates to methods for assessing whether a plant produces a greater number of ripened grains or heavier ripened grains under drought stress as compared to a control, which comprise the step of carrying out PCR with primers comprising the nucleotide sequences of SEQ ID NOs: 10 and 11 using as a template a genomic DNA prepared from a test plant, wherein the test plant is judged not to produce a greater number of ripened grains or heavier ripened grains under drought stress as compared to a control when PCR yields an amplification product.

In addition, the present disclosure relates to methods for selecting plants that produce a greater number of ripened grains or heavier ripened grains under drought stress as compared to a control, which comprise the steps of:
(a) producing a cultivar by crossing an arbitrary plant with a plant that produces a greater number of ripened grains or heavier ripened grains under drought stress as compared to a control; and
(b) assessing whether a plant created in step (a) produces a greater number of ripened grains or heavier ripened grains under drought stress as compared to a control by the above-described method for assessing whether a plant produces a greater number of ripened grains or heavier ripened grains under drought stress as compared to a control.

The production, assessment, and selection of the plants described above can be achieved according to the description herein.

Herein, "produce a greater number of ripened grains or heavier ripened grains" means that the number of grains ripened under drought stress is greater or the weight of grain ripened under drought stress is heavier when compared to a control without having a DNA in the context of the present invention. Herein, as long as the number of ripened grains is greater or the ripened grain weight is heavier when compared to the control, even if the difference is very small, the plant is judged to "produce a greater number of ripened grains or heavier ripened grains".

The number of ripened grains of a plant can be readily determined, for example, by counting ripened grains in harvested panicles excluding infertile panicles. However, the determination method is not limited to this example. Meanwhile, the ripened grain weight of a plant can be readily determined, for example, by weighing ripened grains in harvested panicles excluding infertile panicles. However, the determination method is not limited to this example.

SEQ ID NOs corresponding to respective sequences are listed below:
SEQ ID NO: 1, the genomic DNA nucleotide sequence for the Dro1 gene of Kinandang Patong;
SEQ ID NO: 2, the cDNA nucleotide sequence for the Dro1 gene of Kinandang Patong;
SEQ ID NO: 3, the amino acid sequence of the Dro1 protein of Kinandang Patong;
SEQ ID NOs: 4 and 5, a primer set used for amplifying Dro1-INDEL09, an InDel marker for polymorphism between IR64 and Kinandang Patong;
SEQ ID NOs: 6 and 7, a primer set used for amplifying Dro1-CAPS05, a CAPS marker for polymorphism between IR64 and Kinandang Patong;
SEQ ID NOs: 8 and 9, a primer set used for amplifying SNP02-KP, a Kinandang Patong genomic DNA-specific marker;
SEQ ID NOs: 10 and 11, a primer set used for amplifying SNP02-IR64, an IR64 genomic DNA-specific marker
SEQ ID NO: 12, the CDS nucleotide sequence of the sorghum Dro1 gene;
SEQ ID NO: 13, the amino acid sequence of the sorghum Dro1 protein;
SEQ ID NO: 14, the CDS nucleotide sequence of the corn Dro1 gene;
SEQ ID NO: 15, the amino acid sequence of the corn Dro1 protein;
SEQ ID NO: 16, the cDNA nucleotide sequence of Nipponbare used in the FOX hunting system [This sequence has a 1-bp addition at each of the 5' and 3' ends when compared to the cDNA sequence (the nucleotide sequence of SEQ ID NO: 2) determined based on the genomic DNA nucleotide sequence. Furthermore, this cDNA had a nucleotide substitution of G for A at bp position 373 from the 5' end in its nucleotide sequence (at bp position 372 from the 5' end in SEQ ID NO: 2).];
SEQ ID NO: 17, the nucleotide sequence of the Dro1 gene of Kinandang Patong and its upstream sequence including the promoter region; and
SEQ ID NO: 18, the nucleotide sequence of a region in Nipponbare that corresponds to SEQ ID NO: 17.

### Examples

In the present invention, a basket method was improved to enable simple, reproducible assessment of deep rooting in a space-saving way. Furthermore, a callus-based Agrobacterium transformation method was improved to introduce candidate nucleotide sequences into IR64, and the gene was introduced into IR64. The nucleotide sequence of Dro1, which is the deep rooting gene, was isolated and identified by a map-based cloning method. Thus, the present inventors developed techniques for conferring drought avoidance ability to rice plants by easily modifying the deep rooting using the gene.

Hereinbelow, the present invention will be specifically described with reference to the Examples, but it is not to be construed as being limited thereto.

### [Example 1] Identification of Dro1 gene locus

Two rice cultivars: Kinandang Patong (a Philipino upland rice variety) and IR64 (a paddy-field rice cultivar developed by the International Rice Research Institute) were distributed by the International Rice Research Institute. The two cultivars were crossed with each other to obtain materials for gene isolation. Among the BC₂F₂ population which results from crossing of the two cultivars, a population segregated at chromosome 9 but fixed to IR64 homozygous as much as possible in the other chromosomal regions segregated into shallow-rooted plants and deep-rooted plants. IR64 and Kinandang Patong show the shallow rooting type and deep rooting type, respectively. Therefore, the gene responsible for deep rooting of Kinandang Patong was assumed to be involved in the segregation. From the perspective described above, the inventors thoroughly evaluated the population and divided the plants into shallow rooting and deep rooting types to investigate the genotypes. The result showed that the quantitative trait locus (QTL) related to the deep rooting was located on chromosome 9 (Uga et al., The 2nd International Conference on Plant Molecular Breeding. 2007). A detailed genetic analysis was carried out by using the basket method that enables quantitative assessment of the deep rooting. Specifically, plastic baskets with a diameter of 15 cm were filled with soil and buried in pots. After sowing, rice plants were cultivated until they reached about leaf age 8. The deep rooting was assessed based on the deep rooting ratio, which was defined as the percentage of roots penetrating the bottom of the basket with respect to the total number of roots penetrating each basket. The baskets have flat bottoms. When extending downward more than 53° with respect to ground surface, the root penetrates the basket bottom. The mean deep rooting ratio was 1.6% for IR64, whereas the mean ratio of Kinandang Patong was 72.6%. To map the QTL related to the deep rooting as a single locus, eight plants having recombinations in a region near the QTL were selected from BC₂F₂ population. Then, inbred fixed lines (BC₂F₄) were selected from the selfed progenies (BC₂F₃). From each fixed line, 20 to 23 plants were cultivated in a pot. The genotypes were deduced from the deep rooting ratio determined by the basket method. From the BC₂F₄ line, strains whose region around QTL were fixed in the IR64 type showed a mean deep rooting ratio of 2.6%, which was almost comparable to that of IR64. Meanwhile, stains whose regions around QTL were fixed in the Kinandang Patong type exhibited a mean deep rooting ratio of 40.4%. Their QTL genotypes were clearly determined from these results. The QTL was mapped as a single locus located between InDel markers: ID07_14 and ID07_17. Thus, the QTL was named deep rooting-related locus "Dro1" (Deeper Rooting 1) (Uga et al., Nihon Ikusyu Gakkai Dai 112 Kai Kouenkai Youshisyu (112nd Meeting of The Japanese Society of Breeding, Program and Abstracts) PP. 188, 2007; Uga et al., Dai 27 Kai Ne Kenkyu Syukai (27th Research Meeting of The Japanese Society for Root Research), 2007; Uga et al., The 5th International Crop Science Congress Abstracts 243p. 2008). Furthermore, all SSR markers located in the region between the two InDel markers were assessed by polymorphism analysis based on public information on simple sequence repeat (SSR) markers (International Rice Genome Sequencing Project 2005). The candidate region for Dro1 was narrowed down to 608 kbp located between SSR markers: RM24393 and RM7424.

### [Example 2] High-resolution linkage analysis

To isolate the Dro1 gene by a map-based cloning method, 359 plants having recombinations within the candidate region were selected from BC₃F₂ population consisting of 4,560 plants. A large number of plants had to be assessed at one time for their deep rooting ratio to narrow down the candidate region using progenies of the selected plants. Then, the present inventors developed an evaluation method that enables hydroponical cultivation without burying baskets in pots. In the improved basket method developed by the present inventors, custom-made stainless-steel baskets with a diameter of 7.5 cm filled with soil were placed in a hydroponic medium, instead of being buried in pots. Thus, the method enables one to assess rice plants for the deep rooting ratio in one fourth of the space required in the original method. In the improved basket method, the deep root was defined as extending downward more than 50° with respect to ground surface (Fig. 1). Using the improved basket method, the deep rooting ratio was determined by assessing about 40 plants per line. The genotype of Dro1 gene in each line was predicted based on the frequency distribution of the deep rooting ratio. To narrow down the gene region, DNA markers were selected by screening. The information about SNPs located near the Dro1 in Azucena, a cultivar closely related to IR64 and Kinandang Patong, was extracted from the homepage of OryzaSNP Consortium (http://irfgc.irri.org/index.php?option=com_content&task=view&id=14&Itemid=106) for the designing of CAPS markers. The CAPS markers were tested for polymorphisms. As a result, six markers detected polymorphisms between IR64 and Kinandang Patong, and the six polymorphism markers were used in mapping. Furthermore, BAC libraries of IR64 and Kinandang Patong were constructed, and screened for clones comprising the candidate region. The selected clones were analyzed by nucleotide sequencing. The resulting sequence information was used to prepare 11 types of InDel markers and CAPS markers for polymorphism between IR64 and Kinandang Patong. Recombinant lines were selected from 359 lines using these markers. By linkage analysis, the Dro1 gene region was narrowed down to a 6.0-kbp region between an In Del marker Dro1-INDEL09 (primers: 5'- GCAGACGCTCGTAACACGTA -3' ((SEQ ID NO: 4) and 5'- GTGGCAGCTCCATCAACTCT -3' (SEQ ID NO: 5)) and a CAPS marker Dro1-CAPS05 (primers: 5'- GCACAAGATGGGAGGAGAGT -3' (SEQ ID NO: 6) and 5'- CATGGGTGAGAATCGTGTTG -3' (SEQ ID NO: 7); the amplified DNA is digested with restriction enzyme *Hinf*I)*.* A RAP-DB analysis of the genomic nucleotide sequence comprising the candidate region revealed the presence of one predicted gene. The predicted gene was found to have 1-bp deletion in exon 4 of the IR64 sequence that causes a frame shift, resulting in a stop cordon.

### [Example 3] Complementation test for identifying the Dro1 gene and assessment of the deep rooting ratio in Dro1 gene-overexpressing plants

### 3.1 Complementation test for identifying the Dro1 gene

The gene predicted by RAP-DB was presumed to be Dro1. A Kinandang Patong-derived 8.7-kbp KpnI-NotI fragment covering the 6.0-kbp candidate region for Dro1 and their upstream and downstream regions was inserted into pPZP2H-lac (Fuse et al., Plant Biotechnology 18: 219-222, 2001), and introduced into calluses of IR64 through Agrobacterium EHA101. Specifically, transformation of IR64 was carried out as follows.

### (Induction of calluses for Agrobacterium infection)

Sterilized IR64 seeds were placed in a callus induction medium containing 2,4-D, and cultured at 30 to 33°C for one week under continuous light. Then, the calluses were divided and transferred into a fresh callus induction medium. This procedure was repeated three times for callus formation. The callus induction medium used was modified from NBPRCH40 (Hiei and Komari Nature Protocols 3: 824-834. 2008), which had been used in Hiei and Komari for preculturing calluses for redifferentiation of plant transformants after selection of calluses transformed by the immature embryo method. The callus induction medium has the following composition:
100 mL of 10x N6 major salts, 10 mL of 100x Fe-EDTA, 1 mL of 1,000x B5 minor salts, 1 mL of 1,000x B5 vitamins, 30 g/L maltose, 0.5 g/L casamino acids, 0.5 g/L proline, 2 mg/L 2,4-D, and 5 g/L Gelrite (pH 5.8).

### (Agrobacterium infection)

Prior to infection, calluses are transferred into a fresh medium. After three days of preculture, the calluses were soaked in an Agrobacterium suspension. Then, the calluses were transferred into 2N6-AS medium (Hiei and Komari, Nature Protocols 3: 824-834. 2008) and co-cultured at 23°C in the dark.

### (Removal of bacteria and selection of transformed calluses)

Agrobacterium was removed after co-culture. Then, in order to select transformed cells, the transformed calluses were placed in a callus induction (selection) medium containing drugs (25 mg/L Hygromycin and 400 mg/L carbenicillin). After one week of culture at 30 to 33°C under continuous light, the calluses were divided and transferred into a fresh selection medium. This procedure was repeated three times to select the transformed cells.

### (Redifferentiation from transformants)

The calluses grown in the selection medium were transferred into a redifferentiation medium. After one week to 10 days of culture at 28°C under continuous light, sprouting calluses were transferred into a fresh redifferentiation medium. This procedure was repeated twice to select transformed plants. The composition of the redifferentiation medium is as follows: 100 mL of 10x N6 major salts, 10 mL of 100x Fe-EDTA, 1 mL of 1,000x B5 minor salts, 1 mL of 1,000x B5 vitamins, 30 g/L maltose, 30 g/L sorbitol, 2g/L casamino acids, 0.5 g/L proline, 0.02 mg/L NAA, 5 g/L, 2 mg/L kinetin, and 5 g/L Gelrite (pH 5.8). Hygromycin and carbenicillin were added at 25 mg/L and 300 mg/L to the prepared medium, respectively.

### (Naturalization)

The redifferentiated transformants were transferred and cultured in a rooting medium (MS medium (4 g/L Gelrite (pH 5.8) supplemented with 25 mg/L Hygromycin and 200 mg/L carbenicillin)) at 28°C under continuous light. The transformed plants were naturalized after confirmation of root growth.

The 17 independent callus-derived transformant clones obtained through Hygromycin selection were tested for their deep rooting ratio in the first generation (T0) by the improved basket method. Among lines introduced with the Kinandang Patong-derived 8,7-kbp fragment a number of lines showed a high deep rooting ratio, whereas the deep rooting ratio in the vector control was the same as that of IR64 (Figs. 1 and 2). The copy number of the transformation vector introduced into each plant of the T0 generation was determined by the combined use of Southern analysis and a real-time PCR system that allows detection of the sequence of the Hygromycin-resistant gene in the transformation vector. Plants carrying the gene in a single copy were selected and T1 seeds were produced. The null type (0 copy), heterozygote (1 copy), and homozygote (2 copies) of T1 plants from four lines with single-copy T0 were inferred based on the signal intensities obtained by the real-time PCR system, and the correlation with the deep rooting ratio was evaluated. In all of the four lines, plants with zero signal intensity showed the same deep rooting ratio as plants with the control vector (Fig. 3). Meanwhile, lines with a strong intensity exhibited a high deep rooting ratio. For example, two lines of D27-c were speculated to be a null type, because their signal intensities were zero and their deep rooting ratios were low. Meanwhile, four lines with a signal intensity of about 10 to 20 showed a medium deep rooting ratio and thus were assumed to be the heterozygous type; and all four lines with a signal intensity of about 40 to 70 exhibited a high deep rooting ratio, and therefore were judged to be the homozygous type. As described above, in T1 lines with a single copy of Dro1, a positive correlation was found between the deep rooting ratio and the genotype segregation for the introduced Dro1 gene.

### 3.2 Assessment of Dro1 gene-overexpressing plants for their deep rooting ratio

A full-length cDNA (AK068870) for the putative gene has been registered in RAP-DB. Two Nipponbare lines for each of the T1 and T2 generations are available on the FOX hunting system (Full-length cDNA Over-eXpressing gene hunting system). The full-length cDNA sequence (SEQ ID NO: 16) used for the FOX lines has a 1-bp addition at each of the 5' and 3' ends when compared to the cDNA sequence (SEQ ID NO: 2) determined from the genomic nucleotide sequence. Furthermore, this cDNA had a nucleotide substitution of G for A at bp position 373 from the 5' end in its nucleotide sequence (at bp position 372 from the 5' end in SEQ ID NO: 2). The nucleotide substitution resulted in a non-synonymous amino acid substitution of glutamic acid for lysine at position 37 in SEQ ID NO: 3. Five plants from each of the four lines were assessed for their deep rooting ratio by the improved basket method. The result showed that in the FOX lines the deep rooting ratio ranged from 8.1 to 50.0% while the ratio was between 12.2 and 23.5% for ten wild type Nipponbare (control). Thus, plants of the FOX lines include those exhibiting a deep rooting ratio significantly higher than Nipponbare (Fig. 4).

The result demonstrated that the predicted gene in the 6.0-kbp candidate region was the true Dro1. Meanwhile, the complementation test result suggested that the Kinandang Patong-type Dro1 was the functional form responsible for deep rooting, while the IR64-type Dro1 had lost the function or its function was impaired, resulting in shallow rooting.

Two lines (T1) having Dro1 in multicopies were assessed for the relation between signal intensity and deep rooting ratio. In D130-c, a positive correlation was found between signal intensity and deep rooting ratio (Fig. 3). Meanwhile, with respect to D91-e, a positive correlation was observed between signal intensity and deep rooting ratio in four plants that gave a signal intensity comparable to that of the homozygote with a single copy of the gene (10 to 350); however, six lines with a high signal intensity (500 to 2,000) had the same shallow rooting as that of the vector control. Introduction of Dro1 in multicopies was assumed to cause gene silencing in the plants. This suggests that it is necessary to adjust the expression level of the Dro1 gene, for example, by selecting plants having a single copy of the gene at the time of introduction.

### [Example 4] Amino acid sequence homology among rice, sorghum, and corn Dro1

Using the amino acid sequence of Dro1 as a query, genes homologous to Dro1 were searched by blastn on the NCBI homepage (http://blast.ncbi.nlm.nih.gov/Blast.cgi). The search identified highly homologues genes; one was a sorghum gene, and the other was a corn gene. In both sorghum and corn, the ORF with the highest homology to Dro1 had no gene name. Thus, the present inventors named the sorghum and corn genes "SbDro1L1" and "ZmDro1L1", respectively. The amino acid sequence for the sorghum gene was available on the NCBI homepage. Meanwhile, the amino acid sequence for the corn gene was obtained from MaizeGDB (http://www.maizegdb.org/) via search based on the mRNA sequence extracted at NCBI. The nucleotide sequence of SbDro1L1 CDS is shown in SEQ ID NO: 12, and the amino acid sequence is shown in SEQ ID NO: 13. On the other hand, the nucleotide sequence of ZmDro1L1 CDS is shown in SEQ ID NO: 14, and the amino acid sequence is shown in SEQ ID NO:15. SbDro1L1 and ZmDro1L1 showed 64% and 62% homology to Dro1, respectively (Fig. 5).

### [Example 5] Effect of Dro1 on the deep rooting under field condition

The present inventors assessed whether Dro1 was responsible for deep rooting under field conditions. The near-isogenic lines (Dro1-NIL) used as an experimental material in the field test were developed by the following method. IR64/Kinandang Patong F₁ was backcrossed with IR64 four times, followed by a selfing. From the resulting BC₄F₂ lines, plants homozygous only for the 16.6- to 19.5-Mbp region on chromosome 9 were selected, and selfed seeds from the lines were used as the near-isogenic lines. Seeds of IR64, Kinandang Patong, and Dro1-NIL were planted in an upland field and grown under fertilization management commonly used for upland rice plants. The plants were grown for 105 days under rainfed conditions. The soil near the plant was removed to a depth of about 1 m with a loading shovel. Then, the exposed surface was washed thoroughly with water spray up to about 5 cm from the plant in order to observe maximum root depth. The result showed that the root depth of IR64, Kinandang Patong, and Dro1-NIL in soil was about 20, 80, and 40 cm, respectively (Fig. 6). The root length of Dro1-NIL was the same as that of IR64. However, the root growth depth of Dro1-NIL was about twice as that of IR64. Thus, the root growth angle was increased due to the effect of Dro1, and as a result the roots of Dro1-NIL extended deeper up to the same depth (about 40 cm) as the root length of IR64.

### [Example 6] Effect of Dro1 on drought resistance in the experimental field for testing drought resistance

The present inventors tested whether the deep rooting due to Dro1 improved drought resistance. IR64 and Dro1-NIL were used as experimental material in the drought resistance test. The same drought resistance test facility used in the Plant Biotechnology Institute, Ibaraki Agricultural Center (Hirayama and Suga, Nougyo Kenkyu Senta Kenkyu Shiryo Dai 30 Gou, Ine Ikusyu Manyuaru (Agricultural Research Center Research Data NO. 30; Rice plant breeding manual) 152-155. 1995) was arranged in a plastic greenhouse at the inventors' Institute to carry out the drought resistance test. The facility includes an irrigation area and a drought stress area. In the irrigation area, 30 cm of additional topsoil within a wooden frame was placed on the floor soil. In the irrigation area, rice plants were watered intermittently to avoid drought stress during the period between sowing and harvesting. During the cultivation period, the soil water potential was monitored by a tensiometer placed in soil at a depth of 25 cm, and irrigation was applied when the potential was below about -0.015 MPa (Fig. 7). In general, plants do not suffer from drought stress at this level. Meanwhile, in the drought stress area, 10-mm gravel (5 cm thick) was layered on the bed soil of the experimental field and 25 cm additional topsoil was placed on it to block capillary water from the soil. Under this arrangement, the soil water content in the additional topsoil layer is gradually reduced and the plants are exposed to drought stress when irrigation is terminated. In the drought stress area, irrigation was terminated about two months after sowing and the plants were not watered until the first panicle appearance. The soil water potential at a depth of 25 cm was reduced to -0.07 MPa 10 days after irrigation was terminated. As a result, the drought stress condition was achieved in the drought stress area (Fig. 7). On the other hand, at 40 cm soil depth, the water potential was stable at about -0.03 MPa throughout the period of stress treatment. Thus, the drought stress condition was not achieved at this depth. In the drought stress area, leaf rolling was observed in IR64 on day 35 after the termination of irrigation, suggesting the drought stress effect. In contrast, leaf rolling was not detected in IR64 having Kinandang Patong-type Dro1 (Dro1-NIL) (Fig. 8). Then, the degree of leaf rolling in IR64 was increased in the stress area, and on day 49 the growth of the plants was revealed to be severely suppressed. Meanwhile, in Dro1-NIL, the degree of leaf rolling remained low even on day 49, and the plant growth was vigorous when compared to IR64. The mean leaf temperature of Dro1-NIL was 0.7°C lower than that of IR64 on day 35 after the termination of irrigation in the drought stress area (Fig. 9). The leaf temperature of Dro1-NIL was revealed to be lower than that of IR64 on every measurement day. Furthermore, twice on days 34 and 41, IR64 and Dro1-NIL were also measured for the stomatal conductance and photosynthesis rate. The result showed that both values of Dro1-NIL were significantly greater than those of IR64 (Fig. 9). After the first panicle appearance in the drought stress area, the plants were watered again for rice grain ripening. Each plant was separately harvested, and assessed for their culm length, panicle length, panicle number, panicle weight, number of filled grains, and the dry matter weight of their aerial part. No significant difference was observed in the culm and panicle lengths between IR64 and Dro1-NIL. Meanwhile, the dry matter weight of their aerial part, panicle number, panicle weight, and number of filled grains were significantly different between IR64 and Dro1-NIL (Fig. 10). In particular, the number of filled grains in Dro1-NIL was about 3.8 times greater than that of IR64. To ascertain whether the deep rooting of Dro1-NIL was responsible for this result, the experimental field was dissected to observe whether the roots penetrated the gravel layer. The result revealed that the roots of Dro1-NIL penetrated through the gravel layer into the deeper soil layer whereas the roots of IR64 could not penetrate through the gravel layer (Fig. 11). The findings described above demonstrated that rice plants acquired drought resistance from the deep rooting conferred by Dro1, resulting in increases in the photosynthesis ability and yield.

Meanwhile, the leaf temperature was measured using a device for displaying temperature distribution images by detecting the energy of infrared emitted from the subject and converting it into an apparent temperature (infrared thermography). Specifically, the leaf surface temperature of rice plants under a drought stress condition was measured by infrared thermography from a fixed distance. Then, the images were exported with exclusive software to determine the mean leaf temperatures of plants on the images. The photosynthesis rate was estimated by placing leaves in a chamber aerated with air containing a constant concentration of carbon dioxide and measuring the decrease of carbon dioxide concentration in the chamber output air. Meanwhile, the stomatal conductance was estimated by measuring the increase of water vapor content in the output air from the chamber containing the leaf. Specifically, the values were determined as follows. Fully expanded leaves from rice plants under a drought stress condition were each held nipped for a fixed period of time in the chamber of the photosynthesis transpiration measurement system. The same expanded leaves were measured in triplicate to determine the mean value.

### [Example 7] Drought resistant effect of Dro1 in an upland field under severe drought

In Example 6, Dro1 was demonstrated to be responsible for drought resistance in the experimental field for testing drought resistance. Then, the present inventors assessed whether Dro1 results in drought resistance even under greater drought stress in the natural environment of a crop field. Experiment was carried out using IR64 and Dro1-NIL in an upland field in triplicate for areas with (N:P:K = 12:12:9 kg/10 a) and without fertilizer. In each area (3 m x 3 m), 200 plants were planted with a spacing of 30 cm between rows and 15 cm between plants. The 200 plants consisted of 100 plants each of IR64 and Dro1-NIL. The plants were not watered during cultivation. Thus, the drought stress condition was severe, and the mean soil water potential was -0.08 MPa below at 40 cm soil depth (Fig. 12), because there was no rainfall for one month from 90 days after sowing. Under the drought stress condition, leaf rolling was hardly detectable in Dro1-NIL while severe leaf rolling was observed for IR64 in both areas (Fig. 13). Particularly, in the area without fertilizer, leaf rolling was hardly detected in Dro1-NIL, whereas IR64 showed leaf rolling and the heading date was significantly delayed as compared to that of Dro1-NIL. The yields were estimated by the quadrate method in the harvest season. Of the five parameters investigated, four parameters namely, panicle number, dry matter weight of aerial part, total grain weight, and filled grain weight, had greater values in Dro1-NIL than in IR64 (Table 1).

**[Table 1] YIELD TRAITS OF RICE PLANTS GROWN IN AN UPLAND FIELD UNDER SEVERE DROUGHT**

| TREATMENT | LINE | PANICLE NUMBER | | DRY MATTER WEIGHT OF AERIAL PART (g) | | DRY MATTER WEIGHT OF STEM LEAF (g) | | TOTAL GRAIN WEIGHT (g) | | FILLED GRAIN WEIGHT (g) | |
|---|---|---|---|---|---|---|---|---|---|---|---|
| | | Mean | S.D. | Mean | S.D. | Mean | S.D. | Mean | S.D. | Mean | S.D. |
| NON-FERTILIZATION | IR64 | 347.3 ± | 5.0 a | 1391.5 ± | 124.1 a | 1288.6 ± | 112.6 a | 102.9 ± | 13.3 a | 21.9 ± | 21.8 a |
| | Dro1-NIL | 394.3 ± | 12.2 bc | 1511.7 ± | 36.4 b | 1256.1 ± | 79.5 b | 255.6 ± | 60.4 b | 107.3 ± | 50.6 ab |
| | Dro1-NIL/IR64(%) | 113.5 | | 108.6 | | 97.5 | | 248.4 | | 491.1 | |
| FERTILIZATION | IR64 | 419.3 ± | 39.4 ab | 1710.7 ± | 194.2 ab | 1612.3 ± | 198.3 a | 98.4 ± | 9.5 a | 8.9 ± | 7.9 a |
| | Dro1-NIL | 457.0 ± | 28.2 c | 1733.8 ± | 106.6 b | 1541.9 ± | 118.1 b | 192.0 ± | 54.5 b | 69.9 ± | 48.0 b |
| | Dro1-NIL/IR64(%) | 109.0 | | 101.4 | | 95.6 | | 195.0 | | 783.4 | |

| | | | | | | | | | | | |
|---|---|---|---|---|---|---|---|---|---|---|---|
| Mean: MEAN VALUE FOR A TOTAL OF 24 PLANTS PER AREA IN TRIPLICATE, S.D.: STANDARD DEVIATION THE DIFFERENT ALPHABETICAL LETTERS INDICATE A SIGNIFICANT DIFFERENCE OF 5% IN A PAIRWISE COMPARISON BY Student's t-TEST. | | | | | | | | | | | |

In particular, the filled grain weight, which is the major factor for the yield, was greatly increased to 4.9 times in the area without fertilizer and to 7.8 times in the area with fertilizer. This finding demonstrates that Dro1 resulted in drought resistance in the natural cultivation environment of a crop field even under drought conditions.

### [Example 8] Markers for examining the presence of single nucleotide deletion in the Dro1 gene

Since the Dro1 gene sequence of IR64 has a 1 base deletion in its exon 4, PCR markers were designed to test the presence of the deletion. The two types of designed markers were: SNP02-KP (primers: 5'- GTCTAGATCACGCAGTGAAT -3' (SEQ ID NO: 8) and 5'-TCGCATGATGATGACCAAGT -3' (SEQ ID NO: 9)), which is amplified only in the presence of the Kinandang Patong-type DNA, and SNP02-IR64 (primers: 5'-ATCGTCTAGATCACGCAGTGAAC -3' (SEQ ID NO: 10) and 5'-AGGGTGGCTTTACCTCCGTA -3'(SEQ ID NO: 11)), which is amplified only in the presence of the IR64-type DNA.

The PCR mixture contained 0.2 µM primers, 0.6 U of Tag, 0.2 mM dNTPs, 2 mM MgCl₂, and 20 ng of DNA per reaction (15 µL). The PCR reaction conditions were as follows: (1) 95°C for 2 minutes; (2) 94°C for 30 seconds; (3) annealing temperature ranging from 51.8 to 62.2°C for 30 seconds; (4) 72°C for 1 minute; and (5) for 7 minutes; 25 cycles of reactions (2) to (4) for SNP02-KP; 30 cycles of reactions (2) to (4) for SNP02-IR64. The PCR products were electrophoresed. The result showed that SNP02-KP was amplified only when using Kinandang Patong DNA, while SNP02-IR64 was amplified only when using IR64 DNA (Fig. 9).

### Industrial Applicability

The present invention is concerned with the Dro1 gene, which controls the deep rooting of plants such as rice plants, and plants transformed with the gene. It is expected that plants with improved drought avoidance ability are produced by manipulating the Dro1 gene to convert a shallow-rooted plant into a deep-rooted plant. Alternatively, wet resistance can be conferred to plants by manipulating the Dro1 gene so that a deep-rooted plant is converted into a shallow-rooted plant.

Droughts have caused a serious reduction in the world crop yield. Meanwhile, in Japan, since paddy fields have poor drainage efficiency, wet damage has been problematic for soy and corn without wet resistance. The present invention is useful for solving such domestic and international problems.

## Claims

1. A method for conferring a deep rooting trait to a plant, which comprises the steps of (i) and (ii) below:
(i) introducing into a plant cell a DNA of any one of (a) to (e) below:
(a) a DNA comprising the nucleotide sequence of SEQ ID NO: 1;
(b) a DNA comprising a coding region of the nucleotide sequence of any one of SEQ ID NOs: 1, 2, 12, 14, 16, and 17;
(c) a DNA that encodes a protein comprising the amino acid sequence of any one of SEQ ID NOs: 3, 13, and 15;
(d) a DNA that hybridizes under a stringent condition to a DNA comprising the nucleotide sequence of any one of SEQ ID NOs: 1, 2, 12, 14, 16, and 17, and has an activity of conferring a deep rooting phenotype to a plant; or
(e) a DNA that encodes a protein comprising an amino acid sequence with one or more amino acid substitutions, deletions, additions, and/or insertions in the amino acid sequence of any one of SEQ ID NOs: 3, 13, and 15, and has an activity of conferring a deep rooting phenotype to a plant
or a vector carrying the said DNA of any one of (a) to (e); and
(ii) regenerating a plant from the plant cell introduced with the DNA or the vector in step (i).

2. A method for producing a transformed plant having a deep rooting phenotype, wherein said method comprises the steps of (i) and (ii) below:
(i) introducing into a plant cell a DNA of any one of (a) to (e) below:
(a) a DNA comprising the nucleotide sequence of SEQ ID NO: 1;
(b) a DNA comprising a coding region of the nucleotide sequence of any one of SEQ ID NOs: 1,2, 12, 14, 16, and 17;
(c) a DNA that encodes a protein comprising the amino acid sequence of any one of SEQ ID NOs: 3, 13, and 15;
(d) a DNA that hybridizes under a stringent condition to a DNA comprising the nucleotide sequence of any one of SEQ ID NOs: 1, 2, 12, 14, 16, and 17, and has an activity of conferring a deep rooting phenotype to a plant; or
(e) a DNA that encodes a protein comprising an amino acid sequence with one or more amino acid substitutions, deletions, additions, and/or insertions in the amino acid sequence of any one of SEQ ID NOs: 3, 13, and 15, and has an activity of conferring a deep rooting phenotype to a plant
or a vector comprising the said DNA of any one of (a) to (e); and
(ii) regenerating a plant from the plant cell;
particularly wherein the method further comprises the step of selecting a transformed plant cell or transformed plant, which has the DNA of (a) to (e) in a single copy.

3. The method of claim 2, which comprises the steps of (i) to (iv) below:
(i) introducing into a plant cell a DNA of any one of (a) to (e) below:
(a) a DNA comprising the nucleotide sequence of SEQ ID NO: 1;
(b) a DNA comprising a coding region of the nucleotide sequence of any one of SEQ ID NOs: 1, 2, 12, 14, 16, and 17;
(c) a DNA that encodes a protein comprising the amino acid sequence of any one of SEQ ID NOs: 3, 13, and 15;
(d) a DNA that hybridizes under a stringent condition to a DNA comprising the nucleotide sequence of any one of SEQ ID NOs: 1, 2, 12, 14, 16, and 17, and has an activity of conferring a deep rooting phenotype to a plant; or
(e) a DNA that encodes a protein comprising an amino acid sequence with one or more amino acid substitutions, deletions, additions, and/or insertions in the amino acid sequence of any one of SEQ ID NOs: 3, 13, and 15, and has an activity of conferring a deep rooting phenotype to a plant
or a vector comprising the said DNA of any one of (a) to (e);
(ii) determining the copy number of the artificially introduced Dro1 gene or vector carrying the Dro1 gene in the plant cell of step (i);
(iii) selecting a transformed plant cell containing the introduced DNA or vector in a single copy; and
(iv) regenerating a plant from the transformed plant cell selected in step (iii).

4. A transformed plant which has a deep rooting phenotype, wherein
I) said transformed plant is a plant transformed with a DNA of any one of (a) to (e) below:
(a) a DNA comprising the nucleotide sequence of SEQ ID NO: 1;
(b) a DNA comprising a coding region of the nucleotide sequence of any one of SEQ ID NOs: 1, 12, 14, and 17;
(c) a DNA that encodes a protein comprising the amino acid sequence of any one of SEQ ID NOs: 3, 13, and 15;
(d) a DNA that hybridizes under a stringent condition to a DNA comprising the nucleotide sequence of any one of SEQ ID NOs: 1, 12, 14, and 17, and has an activity of conferring a deep rooting phenotype to a plant; or
(e) a DNA that encodes a protein comprising an amino acid sequence with one or more amino acid substitutions, deletions, additions, and/or insertions in the amino acid sequence of any one of SEQ ID NOs: 3, 13, and 15, and has an activity of conferring a deep rooting phenotype to a plant; or
II) said transformed plant is produced by introducing into a plant cell a DNA of any one of (a) to (e) below:
(a) a DNA comprising the nucleotide sequence of SEQ ID NO: 1;
(b) a DNA comprising a coding region of the nucleotide sequence of any one of SEQ ID NOs: 1, 12, 14, and 17;
(c) a DNA that encodes a protein comprising the amino acid sequence of any one of SEQ ID NOs: 3, 13, and 15;
(d) a DNA that hybridizes under a stringent condition to a DNA comprising the nucleotide sequence of any one of SEQ ID NOs: 1, 12, 14, and 17, and has an activity of conferring a deep rooting phenotype to a plant; or
(e) a DNA that encodes a protein comprising an amino acid sequence with one or more amino acid substitutions, deletions, additions, and/or insertions in the amino acid sequence of any one of SEQ ID NOs: 3, 13, and 15, and has an activity of conferring a deep rooting phenotype to a plant
or a vector carrying the said DNA of any one of (a) to (e).

5. The plant or transformed plant of claim 4, wherein the plant is a monocotyledon,
especially wherein the monocotyledon is a gramineous plant,
in particular wherein
i) the gramineous plant is selected from the group consisting of rice, wheat variety (wheat, barley, rye, oat, Job's tears (hatomugi)), corn, millet, foxtail millet, Japanese millet, sorghum, finger millet, pearl millet, teff, sugarcane, timothy, Kentucky bluegrass, orchardgrass, Italian ryegrass, perennial ryegrass, tall fescue, and Bahia grass;
or
ii) the gramineous plant is selected from the group consisting of rice, sorghum, and corn.

6. A transformed plant which is a progeny or clone of the transformed plant of claim 4 or 5 and comprises a DNA of any one of (a) to (e) below:
(a) a DNA comprising the nucleotide sequence of SEQ ID NO: 1;
(b) a DNA comprising a coding region of the nucleotide sequence of any one of SEQ ID NOs: 1, 12, 14, and 17;
(c) a DNA that encodes a protein comprising the amino acid sequence of any one of SEQ ID NOs: 3, 13, and 15;
(d) a DNA that hybridizes under a stringent condition to a DNA comprising the nucleotide sequence of any one of SEQ ID NOs: 1, 12, 14, and 17, and has an activity of conferring a deep rooting phenotype to a plant; or
(e) a DNA that encodes a protein comprising an amino acid sequence with one or more amino acid substitutions, deletions, additions, and/or insertions in the amino acid sequence of any one of SEQ ID NOs: 3, 13, and 15, and has an activity of conferring a deep rooting phenotype to a plant.

7. A propagation material of the transformed plant of any one of claims 4 to 6, wherein the transformed plant is homozygous for the introduced DNA and the propagation material comprises a DNA of any one of (a) to (e) below:
(a) a DNA comprising the nucleotide sequence of SEQ ID NO: 1;
(b) a DNA comprising a coding region of the nucleotide sequence of any one of SEQ ID NOs: 1, 12, 14, and 17;
(c) a DNA that encodes a protein comprising the amino acid sequence of any one of SEQ ID NOs: 3, 13, and 15;
(d) a DNA that hybridizes under a stringent condition to a DNA comprising the nucleotide sequence of any one of SEQ ID NOs: 1, 12, 14, and 17, and has an activity of conferring a deep rooting phenotype to a plant; or
(e) a DNA that encodes a protein comprising an amino acid sequence with one or more amino acid substitutions, deletions, additions, and/or insertions in the amino acid sequence of any one of SEQ ID NOs: 3, 13, and 15, and has an activity of conferring a deep rooting phenotype to a plant.

8. A method for assessing whether a plant has a deep rooting phenotype, wherein the method is any one of i) to iii) below:
i) a method comprising the steps of (a) to (c) below:
(a) preparing a DNA sample from a test plant;
(b) amplifying from the DNA sample a region comprising a DNA of any one of (A) to (E) below:
(A) a DNA comprising the nucleotide sequence of SEQ ID NO: 1;
(B) a DNA comprising a coding region of the nucleotide sequence of any one of SEQ ID NOs: 1,2, 12, 14, 16, and 17;
(C) a DNA that encodes a protein comprising the amino acid sequence of any one of SEQ ID NOs: 3, 13, and 15;
(D) a DNA that hybridizes under a stringent condition to a DNA comprising the nucleotide sequence of any one of SEQ ID NOs: 1, 2, 12, 14, 16, and 17, and has an activity of conferring a deep rooting phenotype to a plant; or
(E) a DNA that encodes a protein comprising an amino acid sequence with one or more amino acid substitutions, deletions, additions, and/or insertions in the amino acid sequence of any one of SEQ ID NOs: 3, 13, and 15, and has an activity of conferring a deep rooting phenotype to a plant; and
(c) comparing the molecular weight or nucleotide sequence of the amplified DNA fragment with that of the DNA of any one of (A) to (E) described above,
wherein a test plant is judged to have a deep rooting phenotype when the molecular weight or nucleotide sequence of the amplified DNA fragment is identical to that of the DNA of any one of (A) to (E) described above;
ii) a method comprising the steps of carrying out PCR with a primer comprising the nucleotide sequence of SEQ ID NO: 8 and a primer comprising the nucleotide sequence of SEQ ID NO: 9 using a genomic DNA prepared from a test plant as a template, wherein a test plant is judged to have a deep rooting phenotype when an amplified product is obtained;
or
iii) a method comprising the steps of carrying out PCR with a primer comprising the nucleotide sequence of SEQ ID NO: 10 and a primer comprising the nucleotide sequence of SEQ ID NO: 11 using a genomic DNA prepared from a test plant as a template,
wherein a test plant is judged not to have a deep rooting phenotype when an amplified product is obtained.

9. A method for selecting a plant having a deep rooting phenotype, which comprises the step of assessing by the method of claim 8 whether a plant that has been obtained in producing a cultivar by crossing an arbitrary plant with a plant having a deep rooting phenotype has a deep rooting phenotype.

## Patentansprüche

1. Verfahren zum Übertragen eines Merkmals von tiefer Verwurzelung ("deep rooting") auf eine Pflanze, welches die Schritte von (i) und (ii) nachstehend umfasst:
(i) Einführen in eine Pflanzenzelle einer DNA von einem beliebigen von (a) bis (e) nachstehend:
(a) eine DNA, umfassend die Nukleotidsequenz von SEQ ID Nr: 1;
(b) eine DNA, umfassend eine kodierende Region der Nukleotidsequenz von einer beliebigen von SEQ ID Nr: 1, 2, 12, 14, 16 und 17;
(c) eine DNA, die ein Protein kodiert, umfassend die Aminosäuresequenz von einer beliebigen von SEQ ID Nr: 3, 13 und 15;
(d) eine DNA, die unter einer stringenten Bedingung an eine DNA hybridisiert, umfassend die Nukleotidsequenz von einer beliebigen von SEQ ID Nr: 1, 2, 12, 14, 16 und 17, und eine Aktivität des Übertragens eines Phänotyps von tiefer Verwurzelung auf eine Pflanze hat; oder
(e) eine DNA, die ein Protein kodiert, umfassend eine Aminosäuresequenz mit einer oder mehreren Aminosäuresubstitutionen, -deletionen, -additionen und/oder - insertionen in der Aminosäuresequenz von einer beliebigen von SEQ ID Nr: 3, 13 und 15, und eine Aktivität des Übertragens eines Phänotyps von tiefer Verwurzelung auf eine Pflanze hat,
oder eines Vektors, der diese DNA von einem beliebigen von (a) bis (e) trägt, und
(ii) Regenerieren einer Pflanze von der Pflanzenzelle, in die die DNA oder der Vektor in Schritt (i) eingeführt ist.

2. Verfahren zum Produzieren einer transformierten Pflanze, die einen Phänotyp von tiefer Verwurzelung hat, wobei dieses Verfahren die Schritte (i) und (ii) nachstehend umfasst:
(i) Einführen in eine Pflanzenzelle einer DNA von einem beliebigen von (a) bis (e) nachstehend:
(a) eine DNA, umfassend die Nukleotidsequenz von SEQ ID Nr: 1;
(b) eine DNA, umfassend eine kodierende Region der Nukleotidsequenz von einer beliebigen von SEQ ID Nr: 1, 2, 12, 14, 16 und 17;
(c) eine DNA, die ein Protein kodiert, umfassend die Aminosäuresequenz von einer beliebigen von SEQ ID Nr: 3, 13 und 15;
(d) eine DNA, die unter einer stringenten Bedingung an eine DNA hybridisiert, umfassend die Nukleotidsequenz von einer beliebigen von SEQ ID Nr: 1, 2, 12, 14, 16 und 17, und eine Aktivität des Übertragens eines Phänotyps von tiefer Verwurzelung auf eine Pflanze hat; oder
(e) eine DNA, die ein Protein kodiert, umfassend eine Aminosäuresequenz mit einer oder mehreren Aminosäuresubstitutionen, -deletionen, -additionen und/oder - insertionen in der Aminosäuresequenz von einer beliebigen von SEQ ID Nr: 3, 13 und 15, und eine Aktivität des Übertragens eines Phänotyps von tiefer Verwurzelung auf eine Pflanze hat,
oder eines Vektors, der diese DNA von einem beliebigen von (a) bis (e) umfasst; und
(ii) Regenerieren einer Pflanze von der Pflanzenzelle;
insbesondere wobei das Verfahren weiterhin den Schritt des Selektierens einer transformierten Pflanzenzelle oder transformierten Pflanze umfasst, die die DNA von (a) bis (e) in einer einzelnen Kopie hat.

3. Verfahren von Anspruch 2, welches die Schritte (i) bis (iv) nachstehend umfasst:
(i) Einführen in eine Pflanzenzelle einer DNA von einem beliebigen von (a) bis (e) nachstehend:
(a) eine DNA, umfassend die Nukleotidsequenz von SEQ ID Nr: 1;
(b) eine DNA, umfassend eine kodierende Region der Nukleotidsequenz von einer beliebigen von SEQ ID Nr: 1, 2, 12, 14, 16 und 17;
(c) eine DNA, die ein Protein kodiert, umfassend die Aminosäuresequenz von einer beliebigen von SEQ ID Nr: 3, 13 und 15;
(d) eine DNA, die unter einer stringenten Bedingung an eine DNA hybridisiert, umfassend die Nukleotidsequenz von einer beliebigen von SEQ ID Nr: 1, 2, 12, 14, 16 und 17, und eine Aktivität des Übertragens eines Phänotyps von tiefer Verwurzelung auf eine Pflanze hat; oder
(e) eine DNA, die ein Protein kodiert, umfassend eine Aminosäuresequenz mit einer oder mehreren Aminosäuresubstitutionen, -deletionen, -additionen und/oder - insertionen in der Aminosäuresequenz von einer beliebigen von SEQ ID Nr: 3, 13 und 15, und eine Aktivität des Übertragens eines Phänotyps von tiefer Verwurzelung auf eine Pflanze hat,
oder eines Vektors, der diese DNA von einem beliebigen von (a) bis (e) umfasst;
(ii) Bestimmen der Kopienzahl des künstlich eingeführten Dro1-Gens oder des Vektors, der das Dro1-Gen trägt, in der Pflanzenzelle von Schritt (i);
(iii) Selektieren einer transformierten Pflanzenzelle, die die eingeführte DNA oder den eingeführten Vektor in einer einzelnen Kopie enthält; und
(iv) Regenerieren einer Pflanze von der transformierten Pflanzenzelle, die in Schritt (iii) selektiert wurde.

4. Transformierte Pflanze, die einen Phänotyp von tiefer Verwurzelung hat, wobei
I) diese transformierte Pflanze eine Pflanze ist, die mit einer DNA von einem beliebigen von (a) bis (e) nachstehend transformiert ist:
(a) eine DNA, umfassend die Nukleotidsequenz von SEQ ID Nr: 1;
(b) eine DNA, umfassend eine kodierende Region der Nukleotidsequenz von einer beliebigen von SEQ ID Nr: 1, 2, 12, 14 und 17;
(c) eine DNA, die ein Protein kodiert, umfassend die Aminosäuresequenz von einer beliebigen von SEQ ID Nr: 3, 13 und 15;
(d) eine DNA, die unter einer stringenten Bedingung an eine DNA hybridisiert, umfassend die Nukleotidsequenz von einer beliebigen von SEQ ID Nr: 1, 2, 12, 14 und 17, und eine Aktivität des Übertragens eines Phänotyps von tiefer Verwurzelung auf eine Pflanze hat; oder
(e) eine DNA, die ein Protein kodiert, umfassend eine Aminosäuresequenz mit einer oder mehreren Aminosäuresubstitutionen, -deletionen, -additionen und/oder - insertionen in der Aminosäuresequenz von einer beliebigen von SEQ ID Nr: 3, 13 und 15, und eine Aktivität des Übertragens eines Phänotyps von tiefer Verwurzelung auf eine Pflanze hat; oder
II) diese transformierte Pflanze durch Einführen in eine Pflanzenzelle einer DNA von einem beliebigen von (a) bis (e) nachstehend:
(a) eine DNA, umfassend die Nukleotidsequenz von SEQ ID Nr: 1;
(b) eine DNA, umfassend eine kodierende Region der Nukleotidsequenz von einer beliebigen von SEQ ID Nr: 1, 2, 12, 14 und 17;
(c) eine DNA, die ein Protein kodiert, umfassend die Aminosäuresequenz von einer beliebigen von SEQ ID Nr: 3, 13 und 15;
(d) eine DNA, die unter einer stringenten Bedingung an eine DNA hybridisiert, umfassend die Nukleotidsequenz von einer beliebigen von SEQ ID Nr: 1, 2, 12, 14 und 17, und eine Aktivität des Übertragens eines Phänotyps von tiefer Verwurzelung auf eine Pflanze hat; oder
(e) eine DNA, die ein Protein kodiert, umfassend eine Aminosäuresequenz mit einer oder mehreren Aminosäuresubstitutionen, -deletionen, -additionen und/oder - insertionen in der Aminosäuresequenz von einer beliebigen von SEQ ID Nr: 3, 13 und 15, und eine Aktivität des Übertragens eines Phänotyps von tiefer Verwurzelung auf eine Pflanze hat,
oder eines Vektors, der diese DNA von einem beliebigen von (a) bis (e) trägt, hergestellt ist.

5. Pflanze oder transformierte Pflanze von Anspruch 4, wobei die Pflanze eine Monokotyledone ist, insbesondere wobei die Monokotyledone eine Gamineenpflanze ("gramineous plant") ist, insbesondere wobei
(i) die Gramineenpflanze ausgewählt ist aus der Gruppe bestehend aus Reis, Weizenvarietät (Weizen, Gerste, Roggen, Hafer, Hiobstränengras ("Job's tears") (Hatomugi), Mais, Hirse, Kolbenhirse ("foxtail millet"), Hühnerhirse ("Japanese millet"), Sorghum, Fingerhirse ("finger millet"), Perlhirse ("pearl millet"), Teff ("teff"), Zuckerrohr, Wiesenlieschgras ("timothy"), Wiesen-Rispengras ("Kentucky bluegrass"), Gewöhnliches Knäuelgras ("orchardgrass"), Italienisches Weidelgras ("Italian ryegrass"), Deutsches Weidelgras ("perennial ryegrass"), Rohrschwingel ("tall fescue") und Bahiagras ("Bahia grass");
oder
(ii) die Gramineenpflanze ausgewählt ist aus der Gruppe bestehend aus Reis, Sorghum und Mais.

6. Transformierte Pflanze, die ein Nachfahre oder Klon der transformierten Pflanze von Anspruch 4 oder 5 ist und eine DNA von einem beliebigen von (a) bis (e) nachstehend umfasst:
(a) eine DNA, umfassend die Nukleotidsequenz von SEQ ID Nr: 1;
(b) eine DNA, umfassend eine kodierende Region der Nukleotidsequenz von einer beliebigen von SEQ ID Nr: 1, 2, 12, 14 und 17;
(c) eine DNA, die ein Protein kodiert, umfassend die Aminosäuresequenz von einer beliebigen von SEQ ID Nr: 3, 13 und 15;
(d) eine DNA, die unter einer stringenten Bedingung an eine DNA hybridisiert, umfassend die Nukleotidsequenz von einer beliebigen von SEQ ID Nr: 1, 2, 12, 14 und 17, und eine Aktivität des Übertragens eines Phänotyps von tiefer Verwurzelung auf eine Pflanze hat; oder
(e) eine DNA, die ein Protein kodiert, umfassend eine Aminosäuresequenz mit einer oder mehreren Aminosäuresubstitutionen, -deletionen, - additionen und/oder -insertionen in der Aminosäuresequenz von einer beliebigen von SEQ ID Nr: 3, 13 und 15, und eine Aktivität des Übertragens eines Phänotyps von tiefer Verwurzelung auf eine Pflanze hat.

7. Propagierungsmaterial der transformierten Pflanze von einem beliebigen der Ansprüche 4 bis 6, wobei die transformierte Pflanze für die eingeführte DNA homozygot ist und das Propagierungsmaterial eine DNA von einem beliebigen von (a) bis (e) nachstehend umfasst:
(a) eine DNA, umfassend die Nukleotidsequenz von SEQ ID Nr: 1;
(b) eine DNA, umfassend eine kodierende Region der Nukleotidsequenz von einer beliebigen von SEQ ID Nr: 1, 2, 12, 14 und 17;
(c) eine DNA, die ein Protein kodiert, umfassend die Aminosäuresequenz von einer beliebigen von SEQ ID Nr: 3, 13 und 15;
(d) eine DNA, die unter einer stringenten Bedingung an eine DNA hybridisiert, umfassend die Nukleotidsequenz von einer beliebigen von SEQ ID Nr: 1, 2, 12, 14 und 17, und eine Aktivität des Übertragens eines Phänotyps von tiefer Verwurzelung auf eine Pflanze hat; oder
(e) eine DNA, die ein Protein kodiert, umfassend eine Aminosäuresequenz mit einer oder mehreren Aminosäuresubstitutionen, -deletionen, - additionen und/oder -insertionen in der Aminosäuresequenz von einer beliebigen von SEQ ID Nr: 3, 13 und 15, und eine Aktivität des Übertragens eines Phänotyps von tiefer Verwurzelung auf eine Pflanze hat.

8. Verfahren zum Bestimmen, ob eine Pflanze einen Phänotyp von tiefer Verwurzelung hat, wobei das Verfahren ein beliegiges von (i) bis (iii) nachstehend ist:
(i) Verfahren, umfassend die Schritte (a) bis (c) nachstehend:
(a) Herstellen einer DNA-Probe von einer Testpflanze;
(b) Amplifizieren einer Region, umfassend eine DNA von einem beliebigen von (A) bis (E) nachstehend, aus der DNA-Probe:
(A) eine DNA, umfassend die Nukleotidsequenz von SEQ ID Nr: 1;
(B) eine DNA, umfassend eine kodierende Region der Nukleotidsequenz von einer beliebigen von SEQ ID Nr: 1, 2, 12, 14, 16 und 17;
(C) eine DNA, die ein Protein kodiert, umfassend die Aminosäuresequenz von einer beliebigen von SEQ ID Nr: 3, 13 und 15;
(D) eine DNA, die unter einer stringenten Bedingung an eine DNA hybridisiert, umfassend die Nukleotidsequenz von einer beliebigen von SEQ ID Nr: 1, 2, 12, 14, 16 und 17, und eine Aktivität des Übertragens eines Phänotyps von tiefer Verwurzelung auf eine Pflanze hat; oder
(E) eine DNA, die ein Protein kodiert, umfassend eine Aminosäuresequenz mit einer oder mehreren Aminosäuresubstitutionen, -deletionen, -additionen und/oder - insertionen in der Aminosäuresequenz von einer beliebigen von SEQ ID Nr: 3, 13 und 15, und eine Aktivität des Übertragens eines Phänotyps von tiefer Verwurzelung auf eine Pflanze hat; und
(c) Vergleichen des Molekulargewichts oder der Nukleotidsequenz des amplifizierten DNA-Fragments mit dem oder der der DNA von einem beliebigen von (A) bis (E), vorstehend beschrieben,
wobei eine Testpflanze bewertet wird, einen Phänotyp von tiefer Verwurzelung zu haben, wenn das Molekulargewicht oder die Nukleotidsequenz des amplifizierten DNA-Fragments identisch ist zu dem oder der der DNA von einem beliebigen von (A) bis (E), vorstehend beschrieben;
(ii) ein Verfahren, umfassend die Schritte des Ausführens von PCR mit einem Primer, umfassend die Nukleotidsequenz von SEQ ID Nr: 8, und einem Primer, umfassend die Nukleotidsequenz von SEQ ID Nr: 9, unter Verwendung einer genomischen DNA, hergestellt von einer Testpflanze, als eine Matrize, wobei eine Testpflanze beurteilt wird, einen Phänotyp von tiefer Verwurzelung zu haben, wenn ein amplifiziertes Produkt erhalten wird;
oder
(iii) ein Verfahren, umfassend die Schritte des Ausführens von PCR mit einem Primer, umfassend die Nukleotidsequenz von SEQ ID Nr: 10, und einem Primer, umfassend die Nukleotidsequenz von SEQ ID Nr: 11, unter Verwendung einer genomischen DNA, hergestellt von einer Testpflanze, als eine Matrize,
wobei eine Testpflanze beurteilt wird, keinen Phänotyp von tiefer Verwurzelung zu haben, wenn ein amplifiziertes Produkt erhalten wird.

9. Verfahren zum Selektieren einer Pflanze, die einen Phänotyp von tiefer Verwurzelung hat, welches den Schritt des Beurteilens durch das Verfahren von Anspruch 8 umfasst, ob eine Pflanze, die beim Herstellen einer Sorte durch Kreuzen einer beliebigen Pflanze mit einer Pflanze, die einen Phänotyp von tiefer Verwurzelung hat, erhalten wurde, einen Phänotyp von tiefer Verwurzelung hat.

## Revendications

1. Procédé pour conférer une caractéristique d'enracinement profond à une plante, le procédé comprenant les étapes (i) et (ii) ci-dessous :
(i) l'introduction, dans une cellule végétale, d'un ADN selon l'un quelconque des ADN (a) à (e) ci-dessous :
(a) un ADN comprenant la séquence de nucléotides de SEQ ID NO: 1 ;
(b) un ADN comprenant une région codante de la séquence de nucléotides de l'une quelconque de SEQ ID NO: 1, 2, 12, 14, 16, et 17 ;
(c) un ADN qui code pour une protéine comprenant la séquence d'acides aminés de l'une quelconque de SEQ ID NO: 3, 13, et 15 ;
(d) un ADN qui s'hybride dans une condition stricte à un ADN comprenant la séquence de nucléotides de l'une quelconque de SEQ ID NO: 1, 2, 12, 14, 16, et 17, et qui possède une activité conférant un phénotype d'enracinement profond à une plante ; ou
(e) un ADN qui code pour une protéine comprenant une séquence d'acides aminés comportant une ou plusieurs substitutions, délétions, additions, et/ou insertions d'acides aminés dans la séquence d'acides aminés de l'une quelconque de SEQ ID NO: 3, 13, et 15, et qui possède une activité conférant un phénotype d'enracinement profond à une plante,
ou d'un vecteur portant ledit ADN selon l'un quelconque des ADN (a) à (e) ; et
(ii) régénérer une plante à partir de la cellule végétale dans laquelle l'ADN ou le vecteur de l'étape (i) a été introduit.

2. Procédé pour produire une plante transformée possédant un phénotype d'enracinement profond, le procédé comprenant les étapes (i) et (ii) ci-dessous :
(i) l'introduction, dans une cellule végétale, d'un ADN selon l'un quelconque des ADN (a) à (e) ci-dessous :
(a) un ADN comprenant la séquence de nucléotides de SEQ ID NO: 1 ;
(b) un ADN comprenant une région codante de la séquence de nucléotides de l'une quelconque de SEQ ID NO: 1, 2, 12, 14, 16, et 17 ;
(c) un ADN qui code pour une protéine comprenant la séquence d'acides aminés de l'une quelconque de SEQ ID NO: 3, 13, et 15 ;
(d) un ADN qui s'hybride dans une condition stricte à un ADN comprenant la séquence de nucléotides de l'une quelconque de SEQ ID NO: 1, 2, 12, 14, 16, et 17, et qui possède une activité conférant un phénotype d'enracinement profond à une plante ; ou
(e) un ADN qui code pour une protéine comprenant une séquence d'acides aminés comportant une ou plusieurs substitutions, délétions, additions, et/ou insertions d'acides aminés dans la séquence d'acides aminés de l'une quelconque de SEQ ID NO: 3, 13, et 15, et qui possède une activité conférant un phénotype d'enracinement profond à une plante
ou d'un vecteur comprenant ledit ADN selon l'un quelconque des ADN (a) à (e) ; et
(ii) régénérer une plante à partir de la cellule végétale ;
en particulier le procédé comprenant en outre l'étape consistant à sélectionner une cellule végétale transformée ou une plante transformée, qui possède l'ADN de (a) à (e) en une seule copie.

3. Procédé selon la revendication 2, qui comprend les étapes (i) à (iv) ci-dessous :
(i) l'introduction, dans une cellule végétale, d'un ADN selon l'un quelconque des ADN (a) à (e) ci-dessous :
(a) un ADN comprenant la séquence de nucléotides de SEQ ID NO: 1 ;
(b) un ADN comprenant une région codante de la séquence de nucléotides de l'une quelconque de SEQ ID NO: 1, 2, 12, 14, 16, et 17 ;
(c) un ADN qui code pour une protéine comprenant la séquence d'acides aminés de l'une quelconque de SEQ ID NO: 3, 13, et 15 ;
(d) un ADN qui s'hybride dans une condition stricte à un ADN comprenant la séquence de nucléotides de l'une quelconque de SEQ ID NO: 1, 2, 12, 14, 16, et 17, et qui possède une activité conférant un phénotype d'enracinement profond à une plante ; ou
(e) un ADN qui code pour une protéine comprenant une séquence d'acides aminés comportant une ou plusieurs substitutions, délétions, additions, et/ou insertions d'acides aminés dans la séquence d'acides aminés de l'une quelconque de SEQ ID NO: 3, 13, et 15, et qui possède une activité conférant un phénotype d'enracinement profond à une plante
ou d'un vecteur comprenant ledit ADN selon l'un quelconque des ADN (a) à (e) ;
(ii) déterminer le nombre de copies du gène Dro1 introduit de manière artificielle ou du vecteur portant le gène Dro1 dans la cellule végétale de l'étape (i) ;
(iii) sélectionner une cellule végétale transformée contenant l'ADN ou le vecteur introduit en une seule copie ; et
(iv) régénérer une plante à partir de la cellule végétale transformée sélectionnée à l'étape (iii).

4. Plante transformée qui possède un phénotype d'enracinement profond, dans laquelle
I) ladite plante transformée est une plante transformée avec un ADN selon l'un quelconque des ADN (a) à (e) ci-dessous :
(a) un ADN comprenant la séquence de nucléotides de SEQ ID NO: 1 ;
(b) un ADN comprenant une région codante de la séquence de nucléotides de l'une quelconque de SEQ ID NO: 1, 12, 14, et 17 ;
(c) un ADN qui code pour une protéine comprenant la séquence d'acides aminés de l'une quelconque de SEQ ID NO: 3, 13, et 15 ;
(d) un ADN qui s'hybride dans une condition stricte à un ADN comprenant la séquence de nucléotides de l'une quelconque de SEQ ID NO: 1, 12, 14, et 17, et qui possède une activité conférant un phénotype d'enracinement profond à une plante ; ou
(e) un ADN qui code pour une protéine comprenant une séquence d'acides aminés comportant une ou plusieurs substitutions, délétions, additions, et/ou insertions d'acides aminés dans la séquence d'acides aminés de l'une quelconque de SEQ ID NO: 3, 13, et 15, et qui possède une activité conférant un phénotype d'enracinement profond à une plante ; ou
II) ladite plante transformée est produite en introduisant, dans une cellule végétale, un ADN selon l'un quelconque des ADN (a) à (e) ci-dessous :
(a) un ADN comprenant la séquence de nucléotides de SEQ ID NO: 1 ;
(b) un ADN comprenant une région codante de la séquence de nucléotides de l'une quelconque de SEQ ID NO: 1, 12, 14, et 17 ;
(c) un ADN qui code pour une protéine comprenant la séquence d'acides aminés de l'une quelconque de SEQ ID NO: 3, 13, et 15 ;
(d) un ADN qui s'hybride dans une condition stricte à un ADN comprenant la séquence de nucléotides de l'une quelconque de SEQ ID NO: 1, 12, 14, et 17, et qui possède une activité conférant un phénotype d'enracinement profond à une plante ; ou
(e) un ADN qui code pour une protéine comprenant une séquence d'acides aminés comportant une ou plusieurs substitutions, délétions, additions, et/ou insertions d'acides aminés dans la séquence d'acides aminés de l'une quelconque de SEQ ID NO: 3, 13, et 15, et qui possède une activité conférant un phénotype d'enracinement profond à une plante
ou d'un vecteur portant ledit ADN selon l'un quelconque des ADN (a) à (e).

5. Plante ou plante transformée selon la revendication 4, la plante étant une monocotylédone, en particulier la monocotylédone étant une plante graminée, en particulier :
i) la plante graminée étant sélectionnée dans le groupe consistant en le riz, une variété de blé, tel que le blé, l'orge, le seigle, l'avoine ou l'herbe à chapelets (hatomugi), le maïs, le millet, le millet des oiseaux, le millet japonais, le sorgho, l'éleusine, le millet à chandelle, le tef, la canne à sucre, la fléole des prés, le pâturin des prés, le dactyle aggloméré, l'ivraie vivace d'Italie, le ray-grass anglais, la fétuque élevée, et l'herbe de Bahia ;
ou
ii) la plante graminée étant sélectionnée dans le groupe consistant en le riz, le sorgho, et le maïs.

6. Plante transformée qui est une descendance ou un clone de la plante transformée selon la revendication 4 ou 5 et comprend un ADN selon l'un quelconque des ADN (a) à (e) ci-dessous :
(a) un ADN comprenant la séquence de nucléotides de SEQ ID NO: 1 ;
(b) un ADN comprenant une région codante de la séquence de nucléotides de l'une quelconque de SEQ ID NO: 1, 12, 14, et 17 ;
(c) un ADN qui code pour une protéine comprenant la séquence d'acides aminés de l'une quelconque de SEQ ID NO: 3, 13, et 15 ;
(d) un ADN qui s'hybride dans une condition stricte à un ADN comprenant la séquence de nucléotides de l'une quelconque de SEQ ID NO: 1, 12, 14, et 17, et qui possède une activité conférant un phénotype d'enracinement profond à une plante ; ou
(e) un ADN qui code pour une protéine comprenant une séquence d'acides aminés comportant une ou plusieurs substitutions, délétions, additions, et/ou insertions d'acides aminés dans la séquence d'acides aminés de l'une quelconque de SEQ ID NO: 3, 13, et 15, et qui possède une activité conférant un phénotype d'enracinement profond à une plante.

7. Matériel de propagation de la plante transformée selon l'une quelconque des revendications 4 à 6, dans lequel la plante transformée est homozygote pour l'ADN introduit et le matériel de propagation comprend un ADN selon l'un quelconque des ADN (a) à (e) ci-dessous :
(a) un ADN comprenant la séquence de nucléotides de SEQ ID NO: 1 ;
(b) un ADN comprenant une région codante de la séquence de nucléotides de l'une quelconque de SEQ ID NO: 1, 12, 14, et 17 ;
(c) un ADN qui code pour une protéine comprenant la séquence d'acides aminés de l'une quelconque de SEQ ID NO: 3, 13, et 15 ;
(d) un ADN qui s'hybride dans une condition stricte à un ADN comprenant la séquence de nucléotides de l'une quelconque de SEQ ID NO: 1, 12, 14, et 17, et qui possède une activité conférant un phénotype d'enracinement profond à une plante ; ou
(e) un ADN qui code pour une protéine comprenant une séquence d'acides aminés comportant une ou plusieurs substitutions, délétions, additions, et/ou insertions d'acides aminés dans la séquence d'acides aminés de l'une quelconque de SEQ ID NO: 3, 13, et 15, et qui possède une activité conférant un phénotype d'enracinement profond à une plante.

8. Procédé pour évaluer si une plante possède un phénotype d'enracinement profond, le procédé étant l'un quelconque des procédés i) à iii) ci-dessous :
i) un procédé comprenant les étapes de (a) à (c) ci-dessous :
(a) la préparation d'un échantillon d'ADN à partir d'une plante de test ;
(b) l'amplification, à partir de l'échantillon d'ADN, d'une région comprenant un ADN selon l'un quelconque des ADN (A) à (E) ci-dessous :
(A) un ADN comprenant la séquence de nucléotides de SEQ ID NO: 1 ;
(B) un ADN comprenant une région codante de la séquence de nucléotides de l'une quelconque de SEQ ID NO: 1, 2, 12, 14, 16, et 17 ;
(C) un ADN qui code pour une protéine comprenant la séquence d'acides aminés de l'une quelconque de SEQ ID NO: 3, 13, et 15 ;
(D) un ADN qui s'hybride dans une condition stricte à un ADN comprenant la séquence de nucléotides de l'une quelconque de SEQ ID NO: 1, 2, 12, 14, 16, et 17, et qui possède une activité conférant un phénotype d'enracinement profond à une plante ; ou
(E) un ADN qui code pour une protéine comprenant une séquence d'acides aminés comportant une ou plusieurs substitutions, délétions, additions, et/ou insertions d'acides aminés dans la séquence d'acides aminés de l'une quelconque de SEQ ID NO: 3, 13, et 15, et qui possède une activité conférant un phénotype d'enracinement profond à une plante ; et
(c) la comparaison du poids moléculaire ou de la séquence de nucléotides du fragment d'ADN amplifié avec celui/celle de l'ADN selon l'un quelconque des ADN (A) à (E) décrit ci-dessus,
dans lequel une plante de test est jugée de phénotype d'enracinement profond lorsque le poids moléculaire ou la séquence de nucléotides du fragment d'ADN amplifié est identique à celui/celle de l'ADN selon l'un quelconque des ADN (A) à (E) décrit ci-dessus ;
ii) un procédé comprenant les étapes consistant à réaliser une PCR avec une amorce comprenant la séquence de nucléotides de SEQ ID NO: 8 et une amorce comprenant la séquence de nucléotides de SEQ ID NO: 9 en utilisant un ADN génomique préparé à partir d'une plante de test en tant que matrice, dans lequel une plante de test est jugée de phénotype d'enracinement profond lorsqu'un produit amplifié est obtenu ;
ou
iii) un procédé comprenant les étapes consistant à réaliser une PCR avec une amorce comprenant la séquence de nucléotides de SEQ ID NO: 10 et une amorce comprenant la séquence de nucléotides de SEQ ID NO: 11 en utilisant un ADN génomique préparé à partir d'une plante de test en tant que matrice,
dans lequel une plante de test est jugée comme n'ayant pas de phénotype d'enracinement profond lorsqu'un produit amplifié est obtenu.

9. Procédé pour sélectionner une plante possédant un phénotype d'enracinement profond, comprenant l'étape consistant à évaluer, par le procédé de la revendication 8, si une plante qui a été obtenue lors de la production d'un cultivar par le croisement d'une plante arbitraire avec une plante possédant un phénotype d'enracinement profond, possède un phénotype d'enracinement profond.
